# EUROPEAN PATENT SPECIFICATION

(11) **EP 1 260 220 B1**
(45) Date of publication and mention of the grant of the patent: **02.12.2009**
(21) Application number: 01901372.1
(22) Date of filing: 17.01.2001
(51) Int. Cl.: C11D 3/10, C11D 3/20, C11D 17/00, C11D 3/12, A61K 8/02, A61Q 19/10, A61Q 11/00, A61K 9/00

(54) **BUBBLING TABLET, BUBBLING BATH ADDITIVE TABLET, BUBBLING WASHING DETERGENT TABLET, BUBBLING TABLET FOR ORAL ADMINISTRATION, AND PROCESSES FOR PRODUCING THESE**
BRAUSETABLETTE, BRAUSETABLETTE ALS BADEZUSATZ, WASCHMITTELBRAUSETABLETTE, BRAUSETABLETTE ZUR ORALEN ANWENDUNG UND VERFAHREN ZU DEREN HERSTELLUNG
PASTILLE A EFFET MOUSSANT, PASTILLE D'ADDITION POUR LE BAIN A EFFET MOUSSANT, PASTILLE DETERGENTE DE LAVAGE A EFFET MOUSSANT, PASTILLE A EFFET MOUSSANT POUR ADMINISTRATION ORALE ET PROCEDES DE PRODUCTION DE CES PASTILLES

(30) Priority: 17.01.2000 JP 2000008349
(43) Date of publication of application: 27.11.2002
(73) Proprietor: Eurand Pharmaceuticals Ltd, Southern Cross Road Bray, Co. Wicklow (IE)
(72) Inventor: WATANABE, Yasushi, Kyowa Hakko Kogyo co., Ltd., Sunto-gun, Shizuoka 411-8731 (JP); OHTA, Motohiro, Kyowa Hakko Kogyo co., Ltd., Sunto-gun, Shizuoka 411-8731 (JP); MORIMOTO, Kiyoshi, Kyowa Hakko Kogyo co., Ltd., Sunto-gun, Shizuoka 411-8731 (JP)
(74) Representative: Vossius & Partner
(86) International application number: PCT/JP2001/000246
(87) International publication number: WO 2001/052820

(56) References cited:
- EP-A- 0 572 138
- EP-A1- 0 914 818
- WO-A-00/19983
- AU-A- 5 265 499
- JP-A- 5 140 595
- JP-A- 54 044 013
- JP-A- 55 066 507
- JP-A- 56 164 110
- JP-A- 59 205 970
- JP-B1- 45 038 639
- US-A- 4 707 309

## Description

The present invention relates to an effevescent tablet, an effevescent tablet for a bath agent, an effevescent tablet for a washing detergent, an effevescent tablet for oral administration and production methods thereof. Specifically, the present invention relates to an effevescent tablet and an effevescent tablet for a bath agent which don't generate almost any oil film on an aqueous solution surface when the tablet is dissolved in water to be used, an effevescent tablet for a washing detergent which doesn't generate almost any oil film on an aqueous solution surface when the tablet is dissolved in water to be used and which is thrown in a washing machine as a detergent to wash clothes or the like, an effevescent tablet for oral administration which doesn't generate almost any oil film on an aqueous solution surface when the tablet is dissolved in water to be used and which dissolves rapidly, and production methods of these tablets.

An effevescent tablet for oral administration which is dissolved in water and taken as a solution such as a supplement of vitamin like vitamin C and iron, antacids, analgesics and cold remedies, and an effevescent tablet for a bath agent which is used in a bath tab have been already placed on the market.

Further, a powder or granular washing detergent including an fluorescent bleach, enzyme and so on which is used for washing clothes in a washing machine has been also placed on the market.

However, those effevescent tablets generate an oil film on an aqueous solution surface when they are dissolved in water.

Such an oil film is lubricants added in a molding material for preventing tabletting problems such as capping, laminating, sticking and binding when an effevescent tablet is produced by compressing with a punch and a die. Such an oil film doesn't have any problem for human health, however, there is a problem that a person taking a solution and who soaks in a bath tub with an oil film doesn't feel good.

The disintegration pattern and time varies depending on the conventional effevescent tablets for oral administration and it requires a certain time to obtain a solution after the tablet is put in water (namely, before the tablet is completely dissolved in water). Therefore, there is a request from a patient to develop an effevescent tablet which can rapidly dissolve in water.

Further, the powders or granules of washing detergent are contained in a case as a final product to be sold and a user takes them out of the case by means of a measuring scoop and put them in a washing machine.

However, there is a problem that powdered or granular washing detergent is apt to be spilled out of the scoop to be scattered around the washing machine or it may get the user's hand or fingers dirty when it is fed in a sink of the washing machine.

Furthermore, the washing detergent powders or granules contained in a case get easily wet during storage, a user loses a measuring scoop, or such a detergent is hardly taken along for a journey. Therefore, there is a desire from a user to develop a washing detergent which is superior in storage and usability.

US-A-4,707,309 describes effervescent tablets comprising ascorbic acid, citric acid and NaHCO₃. The compounds were individually screened, then mixed and pressed whereby the pressing tools are lubricated.

EP-A-572 138 describes effervescent tablets comprising mannitol, citric acid and KHCO₃. The press used is modified to provide external lubrication by first producing a placebo tablet of Ca phosphate and Mg stearate at the first punch station after which the actual tablet material is filled into the punch at a second filling station.

EP-A-914 818 describes tablets made of granules consisting of mannitol, a disintegrant and water. The granules were pressed under the condition that Mg stearate is put on a metal mould and dies of a hydraulic press machine. The tablets disintegrate rapidly and show adequate hardness. No effervescent is described in EP-A-914 818.

AU-A-5265499 describes a process for making tablets using powder or granulates. The lubricant is sprayed via air pulsation vibration onto the surface of the punch and die. The produced tablets do not adhere to the surfaces of the punch and die, have practical hardness, dissolve rapidly. The exemplified tablets are not effervescent tablets.

Tablets comprising effervescent are known from JP-A-55 066 507, JP-B1-45 038 639, JP-A-5 140 595, JP-A-54 044 013 and JP-A-56 164 110. Especially JP-A- 55 066 507 describes the use of granulated acid and granulated base for making tablets.

The present invention has been proposed to solve the above-mentioned problems. The object of the present invention is to provide an effevescent tablet and an effevescent tablet for a bath agent which don't generate an oil film on a solution surface when it is dissolved in water, to provide a newly shaped washing soap which is superior in storage and usability, different from a conventional powdered or granular washing soap, to provide an effevescent tablet for oral administration which doesn't generate an oil film on a solution surface when it is dissolved in water, has uniform disintegration speed and pattern between tablets and further has a rapid dissolution speed against water comparing with conventional effevescent tablets for oral administration, and to provide a production method of such an effevescent tablet, an effevescent tablet for a bath agent, an effevescent tablet for a washing detergent and an effevescent tablet for oral administration.

According to the present invention, an effevescent tablet is comprised of a tablet body which is produced by compressing a mixture including granules of a main active ingredient, carbonate granules and organic acid granules, wherein said granules of a main active ingredient, the carbonate granules and the organic acid granules are blended such that the particle size distribution of their granules mixture presents a normal distribution with one peak, said mixture does not comprise lubricants, and wherein lubricant powders are attached on an outer surface of the tablet body. The lubricant powders are applied on a punch and a die and transferred to the surface of the tablet body when a compressing step is executed using the punch and the die.

According to such an effevescent tablet, lubricants aren't contained in the mixture to be compressed with the die and the punch. Only a slight amount of lubricant powders applied on the die and the punch is transferred and lubricants aren't included in the effevescent tablet.

Therefore, when the tablet is dissolved in water for use, an oil film is hardly appeared on a solution surface.

Further, for producing effevescent tablets by compressing a molding material with a punch and a die in the conventional manner, lubricants are contained in a molding material in order to prevent tabletting problems such as sticking as mentioned above. Because the lubricants have a water repellency, if they are dispersed in an effevescent tablet, water has difficulty to be permeated in the tablet because of the water repellency when the effevescent tablet is put in water so that the dissolution speed of the tablet becomes slow.

On the other hand, according to the effevescent tablet of the present invention, the lubricants aren't contained in the mixture to be compressed with the punch and the die, the effevescent tablet is rapidly dissolved while producing carbon dioxide (CO₂) because water is easily permeated in the tablet which is put in water.

Therefore, this effevescent tablet has a high dissolution speed against water comparing with a conventional effevescent tablet.

In order to achieve a function of an effevescent tablet, it is preferable that the sum of carbonate granules and organic acid granules other than granules of a main active ingredient is equal to or more than 10 weight %, or more preferably equal to or more than 30 weight % when the total weight of the tablet is 100 weight %.

According to the effevescent tablet of the present invention, the particle diameters of the granules of a main active ingredient, the carbonate granules and the organic acid granules are almost the same.

Such an effevescent tablet uses granules of a main active ingredient, carbonate granules and organic acid granules which have almost the same diameter.

When the composition of the granules of a main active ingredient, the carbonate granules and the organic acid granules at a fixed rate is mixed with a generally used mixer, each granule shows the same behavior against the external forces given by the mixer so that each granule can be uniformly mixed by itself without being distributed unevenly.

When thus obtained mixture is mixed with a generally used tabletting machine, each granule shows the same behavior against the external forces given by the tabletting machine so that each granule isn't distributed unevenly.

Therefore, the disintegration time, the disintegration pattern and the dissolution time of the tablet become uniform when the tablet is put in water for use because the granules of a main active ingredient, the carbonate granules and the organic acid granules are evenly dispersed in the effevescent tablet.

According to the effevescent tablet of the present invention, the granules of a main active ingredient, the carbonate granules and the organic acid granules are blended in such a manner that the particle size distribution of their mixture presents a normal distribution with one peak.

As the composition in which the granules of a main active ingredient, the carbonate granules and the organic acid granules are blended at a fixed ratio is mixed with a general mixer, it shows the same behavior as the case when one kind of powder material having a normal distribution with one peak against the external force given by the mixer is mixed. Therefore, the composition is uniformly mixed by itself without being distributed unevenly per each granule.

As a result, the granules of a main active ingredient, the carbonate granules and the organic acid granules are uniformly dispersed in the effevescent tablet so that there is no difference between the disintegration time, the disintegration pattern and the dissolution time of the tablets when tablets are put in water.

According to the effevescent tablet of the present invention, the carbonate granules are preferably those comprising at least one kind or a mixture of two kinds selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

Such components have been already established as safe components of carbon dioxide of an effevescent tablet and have been generally used. Therefore, the resulting tablet has no problem in view of safety.

According to the effevescent tablet of the present invention, the organic acid granules are preferably those comprising at least one kind selected from the group consisting of citric acid, tartaric acid, fumaric acid, succinic acid, adipic acid, malic acid, and maleic acid.

The organic acid granules has safety which has been already established, are easily obtainable and can decompose carbonate to generate carbon dioxide (CO₂) when they get in touch with water, therefore, the produced tablet has no problem in view of safety.

According to the effevescent tablet of the present invention, each one of the granules of a main active ingredient, the carbonate granules and the organic acid granules are preferably those granulated with a water-soluble polymer as a binder, respectively.

The granules of a main active ingredient, the carbonate granules and the organic acid granules are granulated materials produced with water-soluble polymer as a binder. Therefore, if the effevescent tablet is put in water for use, a binder forming each granule is dissolved in water so that each granule is easily dissolved into a particle level.

As a result, when the effevescent tablet is put in water, the contacting area of the main active agent, the carbonate and the organic acid with water becomes large. Then a reaction takes place by the carbonate and the organic acid so that the tablet is rapidly dissolved in water while generating carbon dioxide.

According to the effevescent tablet of the present invention, each one of the granules of a main active ingredient, the carbonate granules and the organic acid granules are those granulated preferably with a binder including a surfactant.

The granules of a main active ingredient, the carbonate granules and the organic acid granules are preferably granulated using a binder including a surfactant.

As a result, the effevescent tablet is constructed in such a manner that between the particles comprising the granules of a main active ingredient, between the particles comprising the carbonate granules and between the particles comprising the organic acid granules are combined with the binder including a surfactant. Therefore, when the effevescent tablet is put in water for use, the binder bonding the particles comprising each granule easily gets wet because of a surfactant contained therein so that the granules of a main active ingredient, the carbonate granules and the organic acid granules are easily decomposed into a particle unit.

Because the effevescent tablet is constructed in a manner that the granule components are rapidly decomposed into a particle unit when they get in contact with water, the dissolution speed in water is increased comparing with a conventional effevescent tablet.

According to the effevescent tablet for a bath agent of the present invention, the granules of a main active ingredient of the effevescent tablet are sodium carbonate granules.

The sodium carbonate which has been already established as safety component, is easily obtainable and can generate carbon dioxide (CO₂) getting in touch with water is used for the granules of a main active ingredient, therefore, such an effevescent tablet for a bath agent has no problem in view of safety.

Disintegrater, disintegration supplements, stabilizers, perfume agents, coloring agents and hot spring components are added in the effevescent tablet for a bath agent if necessary.

According to the effevescent tablet for a washing detergent of the present invention, the granules of a main active ingredient of the effevescent tablet include surfactant granules and fatty acid alkali salt granules.

The " fatty acid alkali salt" used herein is a soap component, for example sodium fatty acid.

The effevescent tablet for a washing detergent is shaped as a tablet, therefore, if one tablet has the amount to be used at one time for putting in the tub of the washing machine, one tablet is merely put in the tub for washing clothes. Unlike conventional powdered or granular washing detergents, it can save the trouble of measuring with a scoop each time of putting the detergents in the washing tub, therefore, such a tabletted detergent is facilitated to be used comparing with the conventional powdered or granular washing detergent.

Further because of the tabletted shape of the effevescent tablet for a washing detergent, there isn't problem such that powdered or granular detergent is scattered around the washing machine or a person's hands or fingers get dirty with the detergent when the detergent is put in the washing tub.

Disintegrater, disintegration supplements, stabilizers, perfume agents, coloring agents, enzyme and dispersing agent are added in the effevescent tablet for a bath agent if necessary.

It is preferable that the final product shape is a package of one effevescent tablet for a washing detergent in view of its storage.

According to the effevescent tablet for a washing detergent of the present invention, the effevescent tablet further includes anhydrous sodium sulphate.

Because anhydrous sodium sulphate with a hygroscopic property is included in the tablet, the tablet is prevented from naturally foaming by the moisture contained in air while the tablet is stored. Namely, such an effevescent tablet is superior in storage stability.

According to the effevescent tablet for oral administration according to claim 10, the granules of a main active ingredient, the carbonate granules and the organic acid granules in the effevescent tablet are those granulated with a binder including a saccharide with high wettability for water, respectively.

The "saccharide with high wettability for water" means a saccharide which are superior in wettability for water and have a little viscosity increase when a fixed amount of saccharide is dissolved in a fixed amount of water.

More specifically the "saccharide with high wettability for water" means a saccharide which satisfies the kinetic viscosity of a sample solution with 1.0g/100ml concentration is equal to or less than 0.92 centistoke (cSt) or the solubility in water (25°C) is equal to or less than 18 weight % when the viscosity is measured by the Ubbelohde viscosimeter according to the viscosity measuring method defined by the General Test Procedures of Japanese Pharmacopoeia, 13th edition.

In more detail, preferable samples of "saccharide with high wettability for water" include trehalose (0.891cSt), mannitol (0.896cSt), maltose (0.896cSt), sorbitol (0.897cSt), lactose (0.897cSt), maltitol (0.904cSt), xylitol (0.904cSt), sucrose (0.912cSt) and glucose (0.895cSt).

The value shown after each component in parenthesis is the kinetic viscosity of the solution in which 0.5g of each component is dissolved in 50ml of water (25°C).

The effevescent tablet for oral administration is produced by granulating each granules of a main active ingredient, carbonate granules and organic acid granules using a binder including the saccharide with high wettability for water.

As a result, the effevescent tablet for oral administration is constructed in such a manner that between the particles comprising the granules of a main active ingredient, between the particles comprising the carbonate granules and between the particles comprising the organic acid granules are combined with the binder including a saccharide with high wettability for water. Therefore, when the effevescent tablet for oral administration is put in water for dosing, the binder bonding the particles comprising each granule easily gets wet because of the saccharide with high wettability for water contained therein so that the granules of a main active ingredient, the carbonate granules and the organic acid granules are easily decomposed into a particle unit.

Because the effevescent tablet is constructed in a manner that the granule components are rapidly decomposed into a particle unit when they get in contact with water, the dissolution speed in water is increased comparing with a conventional effevescent tablet.

When the amount of medicament containing active ingredient in the tablet is quite slight, powders of the medicament containing active ingredient are added in powders of saccharide with high wettability for water to be mixed uniformly each other and the granulated powders of the mixture may be used as main particles.

According to the production method of the effevescent tablet according to claim 14, the method is comprised of the steps of preparing a mixture including granules of a main active ingredient, carbonate granules and organic acid granules; applying lubricant powders onto a material contacting surface of each of a punch and a die both of which are used for compressing the mixture to produce the tablet; and compressing the mixture with the punch and the die on material contacting surfaces of which the lubricant powders are attached.

In the production method of the effevescent tablet, surfaces of a punch and die, which are used for producing a tablet by compressing the mixture, are applied by lubricant powders, and the effevescent tablet is produced by compressing the mixture with the punch and die on which the surfaces thereof are applied by the lubricant powder. It is not necessary to contain lubricant powder in mixture.

The effevescent tablet is produced in such a manner no lubricant powders are included in the mixture or almost no lubricant powders are included therein, an oil film hardly floats on the solution surface if the tablet is dissolved in water for use.

If lubricants are contained in the mixture (molding material), water has difficulty to be permeated in the tablet because of the water repellency of the lubricants when the effevescent tablet is put in water so that the dissolution speed of the tablet becomes slow.

On the other hand, the effevescent tablet is produced according to the present invention wherein lubricant powders are applied on the material contacting surface of the punch and the die which are used for compressing a mixture to produce a tablet and the mixture is compressed with the lubricated punch and die. Therefore, even if lubricant powders aren't added in the mixture, the effevescent tablet can be produced without causing tabletting problems such as sticking and so on.

Hence, if such a production method of the effevescent tablet is used, the effevescent tablet without including lubricant powders therein or the effevescent tablet scarcely including lubricant powders can be produced. Therefore, according to the effevescent tablet produced by the production method of the present invention, water is rapidly permeated in the tablet so that the tablet is dissolved in water in a short time while generating carbon dioxide (CO₂).

Comparing to the conventional effevescent tablet, the effevescent tablet obtained by the present production method has a higher dissolving speed in water.

In order to achieve a function of an effevescent tablet according to the present production method, it is preferable that the sum of carbonate granules and organic acid granules other than granules of a main active ingredient is equal to or more than 10 weight %, or more preferably equal to or more than 30 weight % when the total weight of the tablet is 100 weight %.

According to the production method of an effevescent tablet of the present invention, the step of applying lubricant powders onto a material contacting surface of each of a punch and a die which are used for compressing the mixture to produce the tablet is executed in a manner that lubricant powders mixed with and dispersed in a positive pulsating vibration air are sprayed onto the material contacting surface of each of the punch and the die which are used for compressing the mixture to produce the tablet.

In such a production method, the lubricant powders mixed with and dispersed in a positive pulsating vibration air is sprayed on the material contacting surfaces of the punch and the die. Therefore, a minimum amount of lubricant powders can be uniformly applied on the material contacting surfaces of the punch and the die by a function of the positive pulsating vibration air.

As a result of such a production method, an effevescent tablet can be continuously produced without causing tabletting problems such as sticking on the produced tablets and without causing grinding on the punch and the die during tabletting.

In other words, the production method can be preferably applied as a production method of an effevescent tablet which is industrially viable.

According to the production method of an effevescent tablet of the present invention, the step of applying lubricant powders onto a material contacting surface of each of a punch and a die, both being used for compressing the mixture to produce the tablet, comprises the steps of a first lubricant applying step in which lubricant powders are applied onto the material contacting surfaces of a lower punch and a die, and a second lubricant applying step in which lubricant powders are applied onto the material contacting surface of an upper punch. The lower punch, the upper punch and the die are used for compressing the mixture to produce a tablet. The first lubricant applying step is comprised of spraying lubricant powders mixed with and dispersed in a positive pulsating vibration air from a lubricant spray port for upper punch, which is provided in a lubricant application means, onto the material contacting surface of the lower punch, which is inserted in a predetermined position in the die; and further applying lubricant powders onto the material contacting surface of the die, in which the lubricant powders are those blown off from the material contacting surface of the lower punch by the positive pulsating vibration air among lubricant powders sprayed onto the material contacting surface of the lower punch. The second lubricant applying step is comprised of blowing lubricant powders into a direction of the material contacting surface of the upper punch from a slit-like lubricant spray port for upper punch provided in the lubricant application means, the lubricant powders being such lubricant powders as to be left as residual lubricant powders which have not been attached onto each of the material contacting surface of the lower punch and the die among those sprayed, while being mixed with and dispersed in the positive pulsating vibration air onto each of the material contacting surface of the lower punch and that of the die from a lubricant spray port for lower punch, which is provided in a lubricant application means; and moving the upper punch from an initial end of the slit-like lubricant spray port for upper punch to the terminal end thereof, thereby taking enough time and applying lubricant powders to the material contacting surface of the upper punch.

In such a production method of an effevescent tablet, lubricant powders mixed with and dispersed in a positive pulsating vibration air are sprayed from the lubricant powder spray port provided for the lubricant apply means on the material contacting surface (upper face) of the lower punch on which lubricant powders are apt to be easily accumulated by gravity, thereby extra lubricant powders on the material contacting surface (upper face) of the lower punch can be blown off by the positive pulsating vibration air.

Therefore, a minimum amount of lubricant powders can be uniformly applied on the material contacting surface (upper face) of the lower punch on which extra lubricant powders are easily applied by gravity.

The extra lubricant powders blown out of the material contacting surface (upper face) of the lower punch by a positive pulsating vibration air apply on the material contacting surface of the die. Then the extra lubricant powders on the material contacting surface of the die is fed to the slit-like lubricant powder spray port for upper punch provided for the lubricant apply means.

As the result, a minimum amount of lubricant powders can be uniformly applied on the material contacting surface (inner circumference) of the die.

Further according to the production method of an effevescent tablet, lubricant powders can be applied on the material contacting surface (lower face) of the upper punch on which lubricant powders are hardly applied by gravity in such a manner that lubricant powders are sprayed from the slit- like lubricant spray port for upper punch into the direction of the material contacting surface (lower face) of the upper punch of the lubricant apply means while the upper punch is moved from the initial end to the terminal end of the slit-like lubricant spray port for upper punch taking enough time.

Thereby, necessary amount of lubricant powders can be applied on the material contacting surface (lower face) of the upper punch on which lubricant powders are hardly applied by gravity.

In other words, according to this production method of an effevescent tablet, the application method of lubricant powders on the material contacting surface (upper face) of the lower punch and that on the material contacting surface (lower face) of the upper punch are differed, thereby necessary amount of lubricant powders can be uniformly applied on the material contacting surface (upper face) of the lower punch and the material contacting surface (lower face) of the upper punch and further necessary amount of lubricant powders can be also uniformly applied on the material contacting surface (inner circumference) of the die.

As a result of such a production method, an effevescent tablet can be continuously produced for a long time without causing tabletting problems such as sticking on the produced tablets and without causing grinding on the punch and the die during tabletting.

In other words, the production method can be preferably applied as a production method of an effevescent tablet which is industrially viable.

According to the production method of the effevescent tablet of the present invention, the diameters of the granules of a main active ingredient, the carbonate granules and the organic acid granules are almost the same.

The granules of a main active ingredient, the carbonate granules and the organic acid granules which have almost the same diameter are used in this production method.

Therefore, when the composition of the granules of a main active ingredient, the carbonate granules and the organic acid granules at a fixed ratio is mixed with a general mixer, each granule shows the same behavior against the external forces given by the mixer so that each granule can be uniformly mixed by itself without being distributed unevenly.

Further, when thus obtained mixture is mixed with a generally used tabletting machine, each granule shows the same behavior against the external forces given by the tabletting machine so that each granule isn't distributed unevenly.

Applying the production method, the effevescent tablet in which the granules of a main active ingredient, the carbonate granules and the organic acid granules are uniformly dispersed can be easily produced. Therefore, the required number of the effevescent tablet which has uniform disintegration time, disintegration pattern and dissolution time when the tablet is put in water for use can be easily produced depending on the user's needs.

According to the production method of an effevescent tablet of the present invention, the granules of a main active ingredient, the carbonate granules and the organic acid granules are blended in such a manner that the particle size distribution of their mixture presents a normal distribution with one peak.

In this production method, the blended ratio of the granules of a main active ingredient, the carbonate granules and the organic acid granules is designed such that the mixture has a normal particle size distribution with one peak after they are mixed.

As the composition in which the granules of a main active ingredient, the carbonate granules and the organic acid granules are blended at a fixed ratio is mixed with a general mixer, it shows the same behavior as the case when one kind of powder material having a normal distribution with one peak against the external force given by the mixer is mixed. Therefore, the composition is uniformly mixed by itself without being distributed unevenly per each granule.

Applying the production method, the effevescent tablet in which the granules of a main active ingredient, the carbonate granules and the organic acid granules are uniformly dispersed can be easily produced. Therefore, the required number of the effevescent tablet which has uniform disintegration time, disintegration pattern and dissolution time when the tablet is put in water for use can be easily produced depending on the user's needs.

According to the production method of an effevescent tablet of the present invention, the carbonate granules are those comprising at least one kind or a mixture of at least two kinds preferably selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

In this production method, such components have safety which has been already established as carbon dioxide components of an effevescent tablet and are been generally used. Therefore, the effevescent tablet produced by this production method also has high reliability.

According to the production method of an effevescent tablet of the present invention, the organic acid granules are those comprising at least one kind preferably selected from the group consisting of citric acid, tartaric acid, fumaric acid, succinic acid, adipic acid, malic acid, and maleic acid.

In this production method, the organic acid granules has safety which has been already established, are easily obtainable and can decompose carbonate to generate carbon dioxide (CO₂) when they get in touch with water, therefore, such granules has no problem in view of safety.

According to the production method of an effevescent tablet of the present invention, each one of the granules of a main active ingredient, the carbonate granules and the organic acid granules are those preferably granulated with a water-soluble polymer as a binder.

In the production method, the granulated material is produced by granulating the granules of a main active ingredient, the carbonate granules and the organic acid granules preferably using water-soluble polymer as a binder. Therefore, if the effevescent tablet is put in water for use, the binder forming each granule is dissolved in water so that each granule is easily dissolved into a particle level.

As a result, in the production method, when the effevescent tablet is put in water, the contacting area of the active agent, the carbonate and the organic acid with water becomes large. Then a reaction takes place between the carbonate and the organic acid so that the tablet is rapidly dissolved in water while generating carbon dioxide (CO₂).

According to the production method of an effevescent tablet of the present invention, each one of the granules of a main active ingredient, the granules of a main active ingredient, the carbonate granules and the organic acid granules are a granulated material produced by means of a binder including a surfactant.

In the production method, a granulated material produced by using a binder including a surfactant is used for each one of the granules of a main active ingredient, the carbonate granules and the organic acid granules are.

As a result, the effevescent tablet is constructed in such a manner that between the particles comprising the granules of a main active ingredient, between the particles comprising the carbonate granules and between the particles comprising the organic acid granules are combined with the binder including a surfactant. Therefore, when the effevescent tablet is put in water for use, the binder bonding the particles comprising each granule easily gets wet because of a surfactant contained therein so that the granules of a main active ingredient, the carbonate granules and the organic acid granules are easily decomposed into a particle unit.

Because the effevescent tablet produced by this production method is constructed in a manner that the granule components are rapidly decomposed into a particle unit when they get in contact with water, the dissolution speed in water is increased comparing with a conventional effevescent tablet.

According to the production method of an effevescent tablet for a bath agent of the present invention, the granules of a main active ingredient used in the production method of an effevescent tablet are sodium carbonate granules.

In the production method, the sodium carbonate which has been already established safe component, are easily obtainable and can generate carbon dioxide (CO₂) when they get in touch with water is used for the granules of a main active ingredient, therefore, the effevescent tablet has no problem in view of safety.

Disintegrater, disintegration supplements, stabilizers, perfume agents, coloring agents and hot spring components are added in the effevescent tablet for a bath agent if necessary. In order to blend such adjuvants, they are added in the mixture.

According to the production method of an effevescent tablet for a washing detergent of the present invention, the granules of a main active ingredient used in the production method of an effevescent tablet include surfactant granules and fatty acid alkali salt granules.

In the production method, the effevescent tablet for a washing detergent is shaped as a tablet, therefore, if one tablet has the amount to be used at one time for putting in the tub of the washing machine, one tablet is merely put in the tub for washing clothes. Unlike a conventional powdered or granular washing detergent, it can save the trouble of measuring with a scoop each time of putting the detergent in the washing tub, therefore, such a tabletted detergent is facilitated to be used comparing with the conventional powdered or granular washing detergent.

Further in the production method, because of the tabletted shape of the effevescent tablet for a washing detergent, there isn't problem such that powdered or granular detergent is scattered around the washing machine or a person's hands or fingers get dirty with the detergent when the detergent is put in the washing tub.

Disintegrater, disintegration supplements, stabilizers, perfume agents, coloring agents and hot spring components are added in the effevescent tablet for a washing detergent produced by this method if necessary. In order to blend such adjuvants, they are added in the mixture.

It is preferable that the final product shape produced by this method is a package of one effevescent tablet for washing detergent in view of its storage.

According to the production method of an effevescent tablet for a washing detergent of the present invention, anhydrous sodium sulphate is further added in the mixture.

In the production method, because anhydrous sodium sulphate with a hygroscopic property is included in the tablet, the tablet is prevented from naturally foaming by the moisture contained in air while the tablet is stored. Namely, such an effevescent tablet is superior in storage stability.

According to the production method of an effevescent tablet for oral administration according to claim 25, each of the granules of a main active ingredient, the carbonate granules and the organic acid granules used in the production methods of an effevescent tablet for oral administration is granulated with a binder including a saccharide with high wettability fort water, respectively.

The production method of the effevescent tablet for oral administration uses the granulated material of granules of a main active ingredient, carbonate granules and organic acid granules obtained by granulation using a binder including a saccharide with high wettability for water.

As a result, the effevescent tablet for oral administration produced by this method is constructed in such a manner that between the particles comprising the granules of a main active ingredient, between the particles comprising the carbonate granules and between the particles comprising the organic acid granules are combined with the binder including a saccharide with high wettability for water. Therefore, when the effevescent tablet for oral administration is put in water for use, the binder bonding the particles comprising each granule easily gets wet because of the saccharide with high wettability for water contained therein so that the granules of a main active ingredient, the carbonate granules and the organic acid granules are easily decomposed into a particle unit.

Because the effevescent tablet for oral administration obtained by this method is constructed in a manner that the granule components are rapidly decomposed into a particle unit when they get in contact with water, the dissolution speed in water is increased comparing with a conventional effevescent tablet.

When the amount of medicament containing active ingredient in the tablet is quite slight, powders of the medicament containing active ingredient are added in powders of a saccharide with high wettability for water to be mixed uniformly each other and the granulated powders of the mixture may be used as main particles.

### Brief Description of Drawings

Fig.1 is an explanatory view of an effevescent tablet of the present invention, Fig.1a is an external perspective view diagrammatically showing an effevescent tablet of the present invention, Fig.1b is a sectional view diagrammatically showing the effevescent tablet shown in Fig.1a.
Fig.2 is a diagrammatical explanatory view showing the effevescent tablet of the present invention in granular unit when the area shown with an imaginary line in Fig.1b is enlarged.
Fig.3 is a diagrammatical explanatory view showing other embodiment of the effevescent tablet of the present invention in granular unit.
Fig.4 is a diagrammatical explanatory view showing other embodiment of the effevescent tablet of the present invention in granular unit.
Fig.5 is a diagrammatical explanatory view showing still other embodiment of the effevescent tablet of the present invention in granular unit.
Fig.6 is an explanatory view diagrammatically showing an effevescent tablet for a washing detergent of the present invention.
Fig.7 is an explanatory view diagrammatically showing a production method of an effevescent tablet for a washing detergent of the present invention.
Fig.8 is an explanatory view diagrammatically showing a production method of an effevescent tablet for a washing detergent of the present invention.
Fig.9 shows other embodiment of an effevescent tablet for a washing detergent wherein granules of a main active ingredient, carbonate granules, organic acid granules and anhydrous sodium sulphate granules are uniformly dispersed.
Fig.10 is an entire construction diagrammatically showing an external lubrication type tabletting machine which can continuously and stably apply a minimum amount of lubricant on each surface (lower face) of an upper punch, surface (inner circumference) of a die, and surface (lower face) of a lower punch.
Fig.11 is an explanatory view exemplifying a positive pulsating vibration air.
Fig.12 is an explanatory view diagrammatically showing a quantitative feeder.
Fig.13 is an explanatory view showing a hopper for storing lubricants in more detail, Fig.13a is a perspective view diagrammatically showing the hopper for storing lubricant and Fig.13b is a plan view diagrammatically showing an essential part of the hopper shown in Fig.13a.
Fig.14 is a plan view diagrammatically showing an elastic membrane.
Fig.15 is a perspective view when the elastic membrane is attached on an elastic membrane installation means of the quantitative feeder.
Fig.16 is an exploded perspective view diagrammatically showing the construction of the elastic membrane installation means shown in Fig.15.
Fig.17 is a sectional view diagrammatically showing the construction of the elastic membrane installation means shown in Fig.15.
Fig.18 is a plan view diagrammatically showing a position of a pulsating vibration air supply port provided for a dispersion chamber when the chamber is seen from top, Fig.18a is an explanatory view showing a preferable position for providing the pulsating vibration air supply port against the dispersion chamber and Fig.18b is an explanatory view showing an actual position for providing the pulsating vibration air supply port against the dispersion chamber.
Fig.19 is an explanatory view diagrammatically showing a position of a pulsating vibration air supply port and its discharge port provided for a dispersion chamber when the chamber is seen from top, Fig.19a is an explanatory view showing a preferable position for providing the pulsating vibration air supply port and its discharge port against the dispersion chamber and Fig.19b is an explanatory view showing an actual position for providing the pulsating vibration air supply port and its discharge port against the dispersion chamber.
Fig.20 is an explanatory view diagrammatically showing operations of a gas injection means and a material feed valve provided for a hopper for storing lubricants of a quantitative feeder.
Fig.21 is a flow chart diagrammatically showing operation programs of a gas injection means and a material feed valve stored in a memory of a processing unit in advance.
Fig.22 is an explanatory view diagrammatically showing operations of an elastic membrane and a bypass pipe when a positive pulsating vibration air is supplied in a dispersion chamber.
Fig.23 is a diagrammatic plan view showing a rotary type tabletting machine used for an external lubrication type tabletting machine of the present invention.
Fig.24 is a plan view diagrammatically showing an enlarged lubricant spray chamber (lubricant apply means) 91 shown in Fig.23.
Fig.25 is a diagrammatic sectional view of the lubricant spraying chamber along the line XXV - XXV in Fig.24.
Fig.26 is a diagrammatic constructional view enlarging around the lubricant suction means shown in Fig.10.
Fig.27 is a diagrammatic sectional view showing a construction of a pulsating vibration air generation means.
Fig.28 is a diagrammatic sectional view showing other embodiment of a pulsating vibration air generation means.
Fig.29 is an exploded perspective view diagrammatically showing other embodiment of a pulsating vibration air generation means.
Fig.30 is a diagrammatic plan view showing other embodiment of an elastic membrane used for a quantitative feeder of an external lubrication type tabletting machine of the present invention.

### Best Mode for Carrying Out the Invention

Now, preferable embodiments of the present invention will be detailed.

### (Embodiment of the Invention 1)

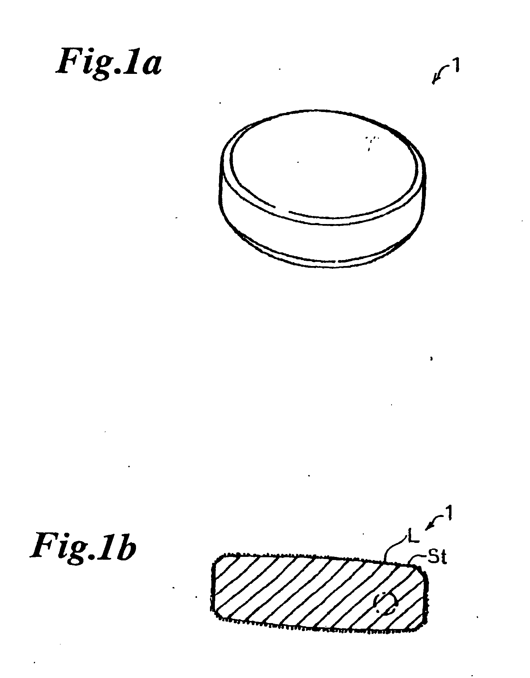

Fig.1 is an explanatory view of an effevescent tablet of the present invention, Fig.1a is an external perspective view diagrammatically showing an effevescent tablet of the present invention, Fig.1b is a sectional view diagrammatically showing the effevescent tablet shown in Fig.1a.

An effevescent tablet 1 is produced by compressing and tabletting a mixture including at least granules of a main active ingredient, carbonate granules and organic acid granules.

Further corrigent powders, coloring agent powders, disintegrater powders, disintegrater supplement powders, stabilizer powders and other adjuvant powders may be included in the effevescent tablet 1.

The granules of a main active ingredient are medicinal properties of the effevescent tablet 1 and several components are used depending on the purpose of the effevescent tablet 1. The granules of a main active ingredient may include one kind of component or plural kinds of component.

The carbonate granules are for example sodium hydrogen carbonate, sodium carbonate, sodium sesquicarbonate, calcium carbonate, potassium carbonate, magnesium carbonate, calcium hydrogen carbonate, and potassium hydrogen carbonate. They may be used solely or more than two of them may be combined to be used.

The organic acid granules are citric acid, tartaric acid, fumaric acid, succinic acid, adipic acid, malic acid, and maleic acid and so on. They may be used solely or more than two of them may be combined to be used.

Such organic acid granules are a component which reacts with carbonate and generates carbon dioxide (CO₂) when the effevescent tablet 1 is put in water for use and the granules are used as neutralizer.

The effevescent tablet 1 is **characterized in that** a slight amount of lubricant powders L is applied only on a surface St of the tablet and lubricant powders L aren't contained in the tablet.

The lubricant powders on the surface St of the effevescent tablet 1 are transferred after compression from the lubricant powders applied on the surface of the punch and the surface of the die. The lubricant powders are applied thereon in order to prevent grinding of the punch and the die and avoid tabletting problems such as capping, laminating, sticking and binding caused by the mixture (molding material) attached on the punch and the die when the mixture (molding material) substantially comprising granules of a main active ingredient, carbonate granules and organic acid granules are compressed with the punch and the die of a tabletting machine.

In order to prevent the mixture (molding material) from adhering on the punch and die of the tabletting machine, it is required to provide a powder layer of lubricants between the mixture (molding material) and the surface of the punch and die of the tabletting machine.

It is necessary to add a certain amount of lubricant powders in the mixture (molding material) in order to exist enough amount of lubricant powders between the mixture (molding material) and the surface of the punch and die of the tabletting machine.

On the other hand, if lubricant powders are applied on the surfaces of the punch and the die, adequate amount of lubricant powders can exist between the surfaces of the punch and the die and the surface of the mixture (molding material) with remarkably a little amount of lubricant powders comparing with the case when lubricant powders are added in the mixture (molding material).

Therefore, when the lubricant powders are applied on the surfaces of the punch and the die without adding them in the mixture (molding material) and the mixture (molding material) is compressed, the amount of lubricant used per one effevescent tablet 1 can be remarkably reduced comparing with the case when lubricant powders are added in the mixture (molding material).

Further, the lubricant powders of the produced effevescent tablet 1 are those transferred from the lubricant powders applied on the punch and die of the tabletting machine so that the amount of lubricant per one effevescent tablet 1 becomes significantly a little comparing with the case when lubricant powders are added in the mixture (molding material).

If a large amount of lubricant is contained in the effevescent tablet, the tablet itself has water repellency because of the water repellency of lubricants. Therefore, when the tablet is put in water for use, water is hardly permeated in the tablet so that disintegration time and the dissolution time of the effevescent tablet become long.

Contrary, the effevescent tablet 1 of the present invention has only a little amount of lubricants on the surface St thereof so that water is easily permeated in the tablet when the effevescent tablet 1 is put in water for use.

Accordingly, because water is easily permeated in the effevescent tablet 1 of the present invention, the tablet is immediately disintegrated and dissolved in water to be a water solution (solution) while generating carbon dioxide (CO₂) when the tablet is put in water for use.

The effevescent tablet 1 has characteristics in the following constructions.

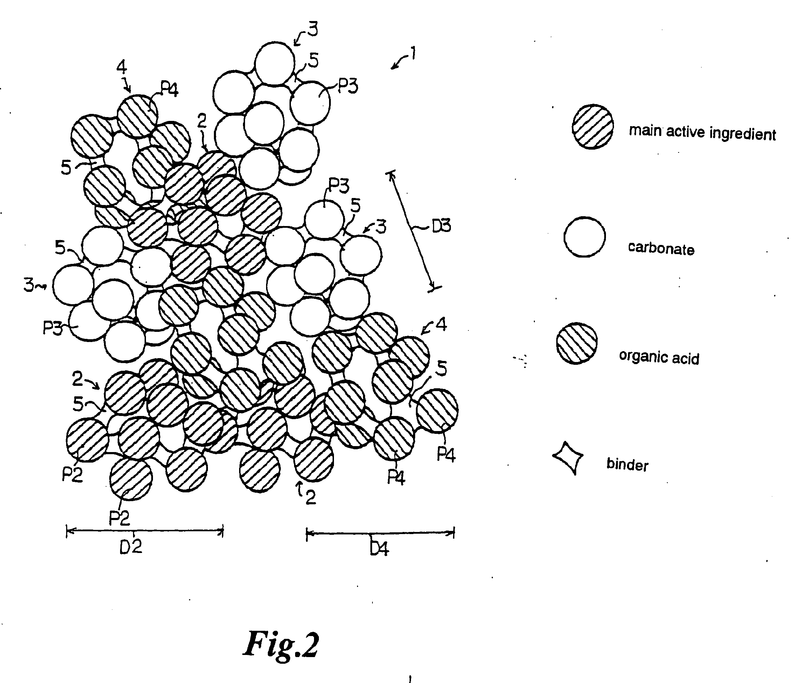

Fig.2 is a diagrammatical explanatory view showing the effevescent tablet in granular unit when the area shown with an imaginary line in Fig.1b is enlarged.

The mixture for the effevescent tablet 1 is substantially comprised of granules of a main active ingredient 2···, carbonate granules 3··· and organic acid granules 4··· as mentioned above. It is characterize in that granules of a main active ingredient 2···, carbonate granules 3··· and organic acid granules 4··· of which diameters are almost the same are used.

If the diameters of granules of a main active ingredient 2···, carbonate granules 3··· and organic acid granules 4··· are almost the same and the component thereof is mixed with a mixer, they can be uniformly mixed in spontaneously by the external force given by the mixer.

In the resulting mixture (molding material) in which granules of a main active ingredient 2···, carbonate granules 3··· and organic acid granules 4··· of which diameters are almost the same are uniformly mixed with the mixer, each particle shows the same behavior against the external force given by the tabletting machine. Therefore, demixing phenomenon of granules of a main active ingredient 2···, carbonate granules 3··· and organic acid granules 4··· isn't shown in the mixture (molding material) during a tabletting procedure.

Thus granules of a main active ingredient 2···, carbonate granules 3··· and organic acid granules 4··· are uniformly dispersed in the effevescent tablet 1. As the result, the disintegration time, the disintegration pattern and the dissolution time between tablets aren't varied when a tablet is put in water for use.

In the above-mentioned effevescent tablet 1, the granules of a main active ingredient 2··· are obtained by granulating main powders P2··· by a binder 5 and the granules 2··· are granulated with water-soluble polymer as a binder 5.

Further in the effevescent tablet 1, the carbonate granules 3··· are obtained by granulating carbonate powders P3··· using water-soluble polymer as a binder 5 and the organic acid granules 4··· are obtained by granulating organic acid powders P4···using water-soluble polymer as a binder 5.

Water-soluble polymers are for example hydroxypropylcellulose, polyvinylpyrrolidone, hydroxypropylmethylcellulose, partially saponified polyvinyl alcohol, methylcellulose (HPMC), pullulane, polyvinyl alcohol (PVA), and hydroxypropylcellulose (HPC).

In the effevescent tablet 1 granules of a main active ingredient 2···, carbonate granules 3··· and organic acid granules 4··· which are obtained by granulation with water-soluble polymer as a binder 5 are compressed to produce a tablet. When the tablet 1 is put in water for dosage, the binder 5 forming each particle 2···, 3···, 4··· is dissolved in water so that each particle 2···, 3···, 4··· is easily dissolved into a particle level (powder unit).

As the result, the contacting area of each main particles P2···, carbonate particles P3··· and organic acid particles P4··· with water becomes large, thereby a reaction of carbonate and organic acid is took place and the tablet is rapidly dissolved in water while producing carbon dioxide (CO₂).

In Fig.2 granules of a main active ingredient 2···, carbonate granules 3··· and organic acid granules 4··· are granulated by means of water-soluble polymer as a binder 5, however, the binder 5 isn't limited to a water-soluble polymer.

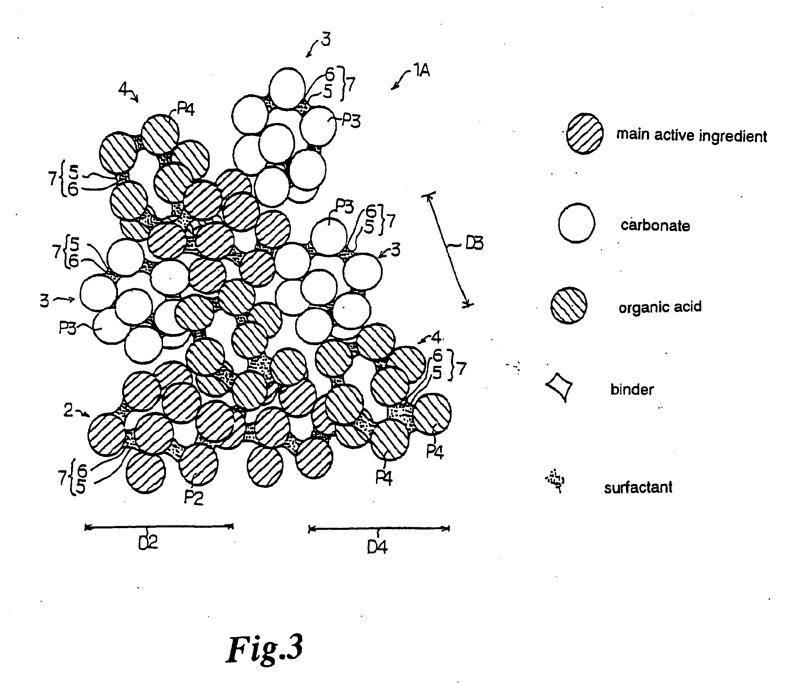

Fig.3 is a diagrammatical explanatory view showing other embodiment of the effevescent tablet of the present invention in granular unit.

In Fig.3 the same member as described in Fig.2 has the same reference numeral and its explanation is omitted.

In the effevescent tablet 1A each one of granules of a main active ingredient 2···, carbonate granules 3··· and organic acid granules 4··· are granulated by means of a binder 7 in which a surfactant 6 is dispersed in water-soluble polymer 5.

Surfactants 6 are for example anionic surfactants, cationic surfactants, nonionic surfactants, amphoteric surfactants, as well as high molecular surfactants such as Pluron or Poloxamer which aren't classified therein.

More concretely, preferable examples of anionic surfactants are sulfate S(R·O·SO₃-·M+) such as sodium lauryl sulfate.

Preferable examples of nonionic surfactants are sorbitan esters (Sorbitane SterS), and polysorbate. One of preferable example of polysorbate is polysorbate 80.

Surfactants 6··· having a HLB (hydrophile-lipophile balance) greater than or equal to 10 and less than or equal to 40 are preferable.

The binder 7 in which a surfactant 6··· is dispersed in water-soluble polymer 5 is used for the effevescent tablet 1A.

Therefore, when the effevescent tablet 1A is put in water for use, the binder 7 easily gets wet by the surfactant 6··· included in the binder 7 and the water-soluble polymer 5 are dissolved in water so that each particle 2···, 3···, 4··· is easily decomposed into a particle level (powder unit).

As the result, the contacting area of the powders of granules of a main active ingredient P2···, the carbonate powders P3··· and the organic acid powders P4··· with water becomes large, thereby a reaction of carbonate and organic acid is took place and the tablet 1A is rapidly dissolved in water while generating carbon dioxide (CO₂).

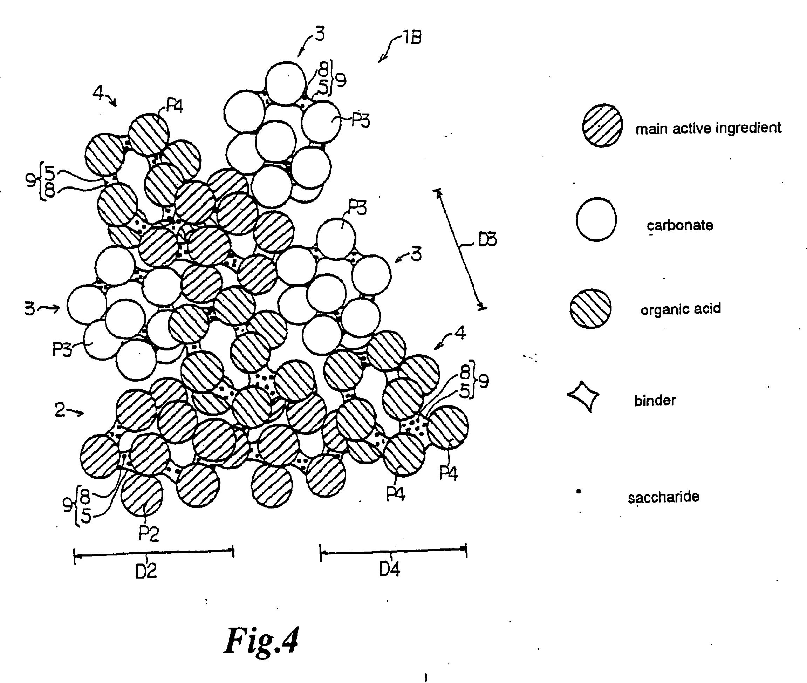

Fig.4 is a diagrammatical explanatory view showing other embodiment of the effevescent tablet of the present invention in granular unit.

In Fig.4 the same member as described in Fig.2 has the same reference numeral and its explanation is omitted.

In the effevescent tablet 1B each one of the granules of a main active ingredient 2···, the carbonate granules 3··· and the organic acid granules 4··· are granulated by means of a binder 9 in which a saccharide with high wettability for water 8··· is dispersed in water-soluble polymers.

The "saccharide with high wettability for water" is for example trehalose, mannitol, maltose, sorbitol, lactose, multitol, xylitol, sucrose, and glucose.

In the effevescent tablet 1B each one of the granules of a main active ingredient 2···, the carbonate granules 3··· and the organic acid granules 4··· are granulated by means of the binder 9 including a saccharide with high wettability for water.

As a result, between the particles comprising the granules of a main active ingredient 2···, between the particles comprising the carbonate granules 3··· and between the particles comprising the organic acid granules 4··· are combined with the binder 9 including a saccharide with high wettability for water. Therefore, when the effevescent tablet 1B is put in water for use, the binder 9 bonding the particles comprising each granule 2···, 3···, 4 ··· easily gets wet because of the saccharide with high wettability for water included in the binder 9. Therefore, the granules of a main active ingredient 2···, the carbonate granules 3··· and the organic acid granules 4··· are easily decomposed into particle level.

Because the components of the granules 2···, 3···, 4 ··· are rapidly decomposed into a particle level when the effevescent tablet 1B gets in touch with water, the dissolution speed in water is faster than that of the conventional effevescent tablets. Reaction of carbonate and organic acid is caused to generate carbon dioxide (CO₂), thereby the effevescent tablet 1B can be rapidly dissolved in water.

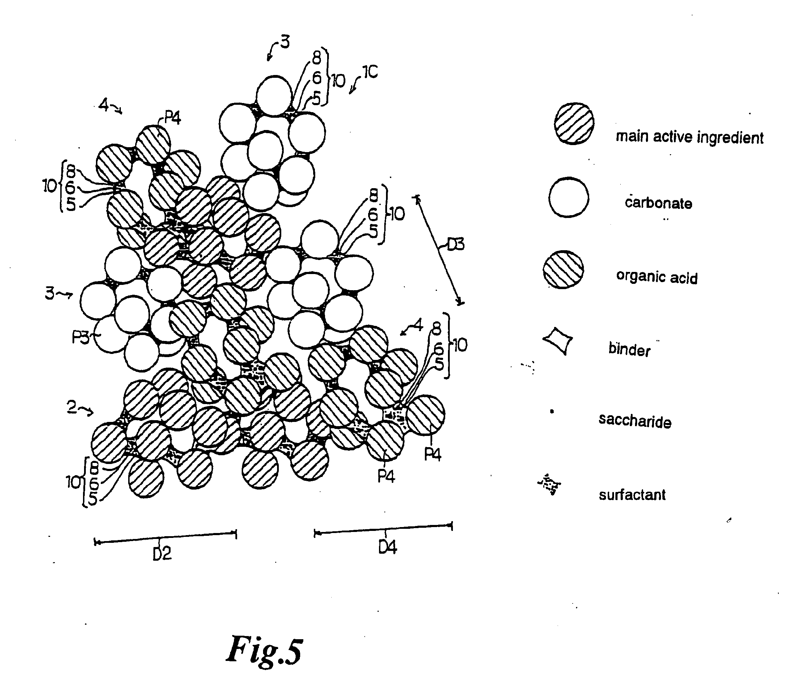

Fig.5 is a diagrammatical explanatory view showing other embodiment of the effevescent tablet of the present invention in granular unit.

In Fig.5 the same member as described in Fig.2 has the same reference numeral and its explanation is omitted.

In the effevescent tablet 1C each one of granules of a main active ingredient, carbonate granules and organic acid granules are granulated by means of a binder 10 including surfactants 6··· and saccharides with high wettability for water 8···.

As a result, between the particles comprising the granules of a main active ingredient 2···, between the particles comprising the carbonate granules 3··· and between the particles comprising the organic acid granules 4··· are combined with the binder 9 including surfactants 6··· and saccharides with high wettability for water 8···. Therefore, when the effevescent tablet 1C is put in water for use, the binder 9 bonding the particles comprising each granule 2···, 3···, 4 ··· easily gets wet because of the surfactants 6··· and the saccharide with high wettability for water 8··· included in the binder 9. Therefore, the granules of a main active ingredient 2···, the carbonate granules 3··· and the organic acid granules 4··· are easily decomposed into a particle level.

Because the components of the granules 2···, 3···, 4 ··· are rapidly decomposed into a particle level when the effevescent tablet 1C gets in touch with water, the dissolution speed in water is faster than that of the conventional effevescent tablets. Rreaction of carbonate and organic acid is took place to generate carbon dioxide (CO₂), thereby the effevescent tablet can be rapidly dissolved in water.

Next an embodiment when the effevescent tablet 1A is applied to washing detergents will be explained.

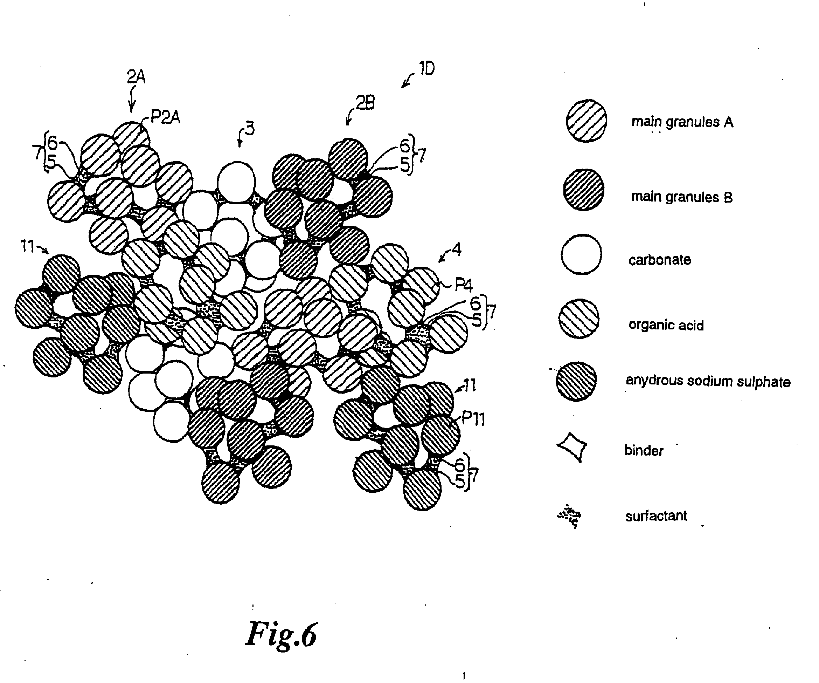

Fig.6 is an explanatory view diagrammatically showing an effevescent tablet for a washing detergent of the present invention.

In the effevescent tablet 1D each one of granules of a main active ingredient 2A···, granules of a main active ingredient 2B···, carbonate granules 3···, organic acid granules 4··· and anhydrous sodium sulphate granules 11 is granulated by means of a binder 7 including surfactants 6···.

Surfactant granules are used as the granules of a main active ingredient 2A.

Samples of a surfactant are alkylbenzene sulfonate or sodium lauryl sulfate.

Granules of soap component (fatty acid sodium salt) are used as granules of a main active ingredient 2B···.

Sodium hydrogen carbonate granules are used as carbonate granules 3···.

Fumaric acid granules are for example used as organic acid granules 4···.

Anhydrous sodium sulphate granules 11 is contained in the effevescent tablet for a washing detergent 1D in order to prevent the tablet 1D from naturally foaming because of the moisture in air during storage.

A binder solution in which a water-soluble polymer 5 and surfactants 6··· are dissolved in water is used for granulating each one of granules of a main active ingredient 2A···, granules of a main active ingredient 2B···, carbonate granules 3···, organic acid granules 4··· and anhydrous sodium sulphate granules 11 are granulated.

Water-soluble polymer 5 dissolved in the binder solution aren't limited if they are generally used water-soluble polymer. For example, polyvinyl alcohol (PVA), and hydroxypropylcellulose (HPC), and hydroxypropylmethylcellulose (HPMC) are used. One of them may be used or more than two of them are combined to be used.

The amount of water-soluble polymer 5 dissolved in the binder solution is preferably equal to or more than 3 weight % and equal to or less than 10 weight % for the water of 100 weight %.

Further, the amount of water-soluble polymer 5 to be used is preferably equal to or more than 1 weight % and equal to or less than 3 weight % for the tablet of 100 weight %.

It is because that if more than 3 weight % of water-soluble polymer 5 is used for the tablet of 100 weight %, the binder 10 stably binds between the particles comprising the granules 2A···, the granules 2B···, the carbonate granules 3··· and the organic acid granules 4··· which are to be granulated so that it takes a long time for each granules to be disintegrated when the effevescent tablet for a washing detergent 1D is put in water.

On the other hand, if less than 1 weight % of water-soluble polymer 5 is added in a 1tablet of 100 weight%, a mechanical strength binding between the particles comprising each granule 2A···, granule 2B···, carbonate granule 3··· and organic acid granule 4··· which are to be granulated so that the effevescent tablet for a washing detergent is easily cracked, thereby it is undesirable.

The amount of surfactants 6··· dissolved in the binder solution is decided by experiments considering the mechanical strength and wettability of the binder 10 against water.

Next, the production method of the effevescent tablet for washing detergent 1D is explained with an sample.

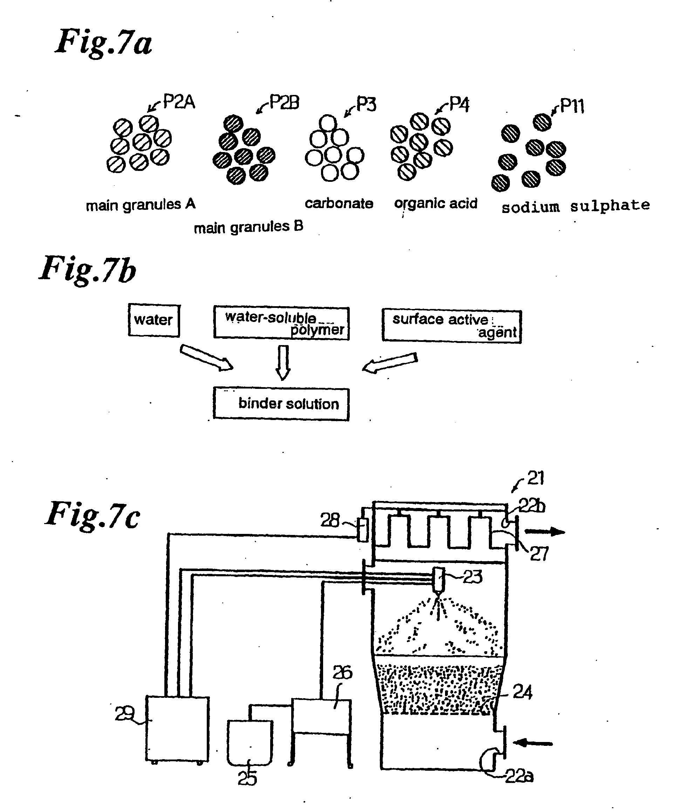

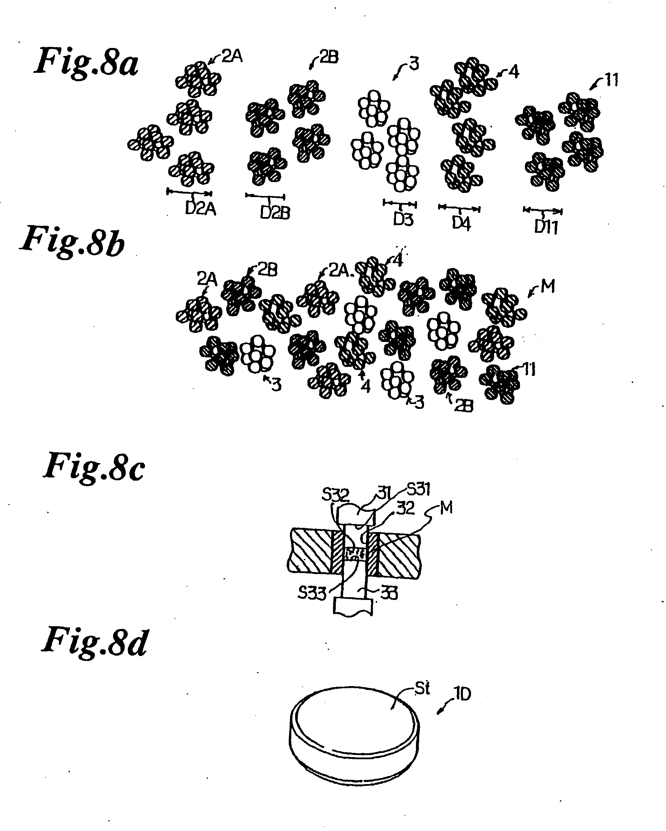

Fig.7 and Fig.8 are an explanatory views diagrammatically showing a production method of the effevescent tablet for a washing detergent 1D.

For producing the effevescent tablet for a washing detergent 1D, as shown in Fig.7 surfactant powders P2A comprising a material of granules of a main active ingredient 2A···, soap component (fatty acid sodium salt) powders P2B comprising a material of granules of a main active ingredient 2B···, carbonate powders P3 comprising a material of carbonate granules 3···, organic acid salt powders P4 comprising a material of organic acid granules 4, and sodium sulphate powders P11 comprising a material of sodium sulphate granules 11 are prepared.

A binder solution is prepared as shown in Fig.7b by dissolving water-soluble polymers and surfactants in water.

Each one of surfactant powders P2A, soap component (fatty acid sodium salt) powders P2B, carbonate powders P3, organic acid salt powders P4, anhydrous sodium sulphate powders P11 are granulated into granules of a main active ingredient 2A···, granules of a main active ingredient 2B···, carbonate granules 3···, organic acid salt granules 4 and sodium sulphate granules 11 by means of a fluid bed granulator 21 as shown in Fig.7c.

More specifically, surfactant powders P2a are contained in a granulation tank 22 of the granulator 21 for obtaining granules of a main active ingredient 2A··· and mixed with a heated air according to a normal method to become a fluid bed. The binder solution prepared in the process of Fig.7b is sprayed on thus fluidized surfactant powders P2A from a spray means 23 provided at a predetermined position in the granulation tank 22, thereby the powders P2A are dried to produce granules of a main active ingredient 2A···.

Further, for obtaining granules of a main active ingredient 2B··· by granulation, soap component (fatty acid sodium salt) powders P2 are contained in the granulation tank 22 of the granulator 21 and mixed with a heated air according to a normal method to become a fluid bed. The binder solution prepared in the process of Fig.7b is sprayed on thus fluidized soap component (fatty acid sodium salt) powders P2B from the spray means 23 provided at a predetermined position in the granulation tank 22, thereby the powders P2B are dried to produce granules of a main active ingredient 2B···.

Furthermore, for obtaining carbonate granules 3··· by granulation, carbonate powders P3 are contained in the granulation tank 22 of the granulator 21 and mixed with a heated air according to a normal method to become a fluid bed. The binder solution prepared in the process of Fig.7b is sprayed on thus fluidized carbonate powders P3 from the spray means 23 provided at a predetermined position in the granulation tank 22, thereby the powders P3 are dried to produce carbonate granules 3···.

Still further, for obtaining organic acid salt granules 4 by granulation, organic acid salt powders P4 are contained in the granulation tank 22 of the granulator 21 and mixed with a heated air according to a normal method to become a fluid bed. The binder solution prepared in the process of Fig.7b is sprayed on thus fluidized organic acid salt powders P4 from the spray means 23 provided at a predetermined position in the granulation tank 22, thereby the powders P4 are dried to produce organic acid salt granules 4.

Furthermore, for obtaining anhydrous sodium sulphate granules 11 by granulation, anhydrous sodium sulphate powders P11 are contained in the granulation tank 22 of the granulator 21 and mixed with a heated air according to a normal method to become a fluid bed. The binder solution prepared in the process of Fig.7b is sprayed on thus fluidized sodium sulphate powders P11 from the spray means 23 provided at a predetermined position in the granulation tank 22, thereby the powders P11 are dried to produce sodium sulphate granules 11. (See Fig.8a)

For obtaining the granules of a main active ingredient 2A···, the granules of a main active ingredient 2B···, the carbonate granules 3···, the organic acid granules 4··· and the anhydrous sodium sulphate granules 11, each particle size diameter thereof is designed to be almost the same during granulation.

Otherwise, the particle size diameters thereof are arranged to be almost the same by screening each granules of a main active ingredient 2A···, granules of a main active ingredient 2B···, carbonate granules 3···, organic acid granules 4··· and anhydrous sodium sulphate granules 11 after granulation.

The reference numeral 22a in Fig.7c shows a heated air supply port, 22b shows a discharge port for discharging the heated air supplied in the granulation tank 22 therefrom.

The member shown with the numeral 24 is a porous screen, 25 shows a binder solution storage tank for storing a binder solution, 26 shows a binder solution supply means for supplying the binder solution stored in the tank 25 to the spray means 23, 27 is a dust collection filter, 28 is a filter vibration means for vibrating the dust collection filter 27 in order to make the powders, granulated material or granulating material attached thereon drop in the granulation tank 22, and the numeral 29 shows an air source for supplying compressed air for spraying the binder solution from the spray means 23 and for supplying compressed air for driving the filter vibration means 28.

Then, as shown in Fig.8b, granules of a main active ingredient 2A···, granules of a main active ingredient 2B···, carbonate granules 3···, organic acid granules 4··· and anhydrous sodium sulphate granules 11 are blended at a fixed ratio and mixed with a well-known mixer (not shown) according to a normal method.

In the blended material of granules of a main active ingredient 2A···, granules of a main active ingredient 2B···, carbonate granules 3···, organic acid granules 4··· and anhydrous sodium sulphate granules 11, each particle size diameter of granules of a main active ingredient 2A···, granules of a main active ingredient 2B···, carbonate granules 3···, organic acid granules 4··· and anhydrous sodium sulphate granules 11 is almost the same. Therefore, when such a blended material is mixed with a general mixer, each granule shows the same behavior against the external force given the mixer, thereby each granule is uniformly mixed by itself without causing particle segregation.

Next, the uniformly mixed mixture M (molding material) obtained by the above-mentioned procedure is compressed with an upper punch 31, a die 32 and a lower punch 33 of a rotary type tabletting machine to be tabletted and an effevescent tablet for a washing detergent 1D is obtained.

Lubricants aren't added in the mixture M (molding material) and are sprayed on a surface S31 (lower face, material contacting surface) of the upper punch 31, a surface S32 (inner circumference, more specifically a material contacting surface above an upper face (material contacting surface) of the lower punch inserted into a fixed position in the die) of the die 32 and a surface S33 (upper face, material contacting surface) of the lower punch 33. Thereby a minimum amount of lubricant is applied on the surface S31 (lower face, material contacting surface) of the upper punch 31, the surface S32 (inner circumference, more specifically a material contacting surface above an upper face (material contacting surface) of the lower punch inserted into a fixed position in the die) of the die 32 and the surface S33 (upper face, material contacting surface) of the lower punch 33. The mixture (molding material) M without including lubricants is compressed with the upper punch 31 on which face S31 (lower face, material contacting surface) lubricants are applied, the die 32 on which surface S32 (inner circumference, more specifically a material contacting surface above an upper face (material contacting surface) of the lower punch inserted into a fixed position in the die) lubricants are applied and the lower punch 33 on which surface S33 (upper face, material contacting surface) lubricants are applied, thereby the effevescent tablet for a washing detergent 1D is produced. (See Fig.8c and Fig.8d)

In the mixture M (molding material), the particle size diameter D2A of granules of a main active ingredient 2A···, the particle size diameter D2B of granules of a main active ingredient 2B···, the particle size diameter D3 of carbonate granules 3···, the particle size diameter D4 of organic acid granules 4··· and the particle size diameter D11 of anhydrous sodium sulphate granules 11 are almost the same. Therefore, each granule 2A···, 2B···, 3···, 4···, 11 ··· shows the same behavior against the external force given by the tabletting machine, thereby each granule is uniformly mixed.

In the effevescent tablet for a washing detergent 1D, lubricants aren't contained in the mixture M (molding material) to be compressed with the punches 31, 33 and the die 32 and only a slight amount of lubricant powders applied on the punches 31, 33 and the die 32 is transferred to the surface St of the tablet 1D, thereby the tablet 1D doesn't hardly include lubricant.

Therefore, when the effevescent tablet for a washing detergent 1D is put in water to be used, the tablet 1D is rapidly permeated with water, thereby it is disintegrated and dissolved in a short time to produce washing detergents solution while generating carbon dioxide (CO₂).

Granules of a main active ingredient 2A···, granules of a main active ingredient 2B···, carbonate granules 3···, organic acid granules 4··· and anhydrous sodium sulphate granules 11 are uniformly dispersed in the effevescent tablet for a washing detergent 1D so that there are no difference of disintegration time, the disintegration pattern and the dissolving time between tablets when the tablet is put in water.

Further according to the effevescent tablet for a washing detergent 1D, each of the granules of a main active ingredient 2A···, the granules of a main active ingredient 2B···, the carbonate granules 3···, the organic acid granules 4··· and the anhydrous sodium sulphate granules 11 are bound by the binder 7 in which a surfactant 6 is included in a water-soluble polymer 5. Therefore, when the effevescent tablet for a washing detergent 1D is put in water, the binder binding the particles comprising each granules 2A···, 2B···, 3···, 4···, 11··· easily get wet because of the surfactant in the binder so that the granules of a main active ingredient 2A···, 2B···, the carbonate granules 3··· and the organic acid granules 4··· are easily decomposed into a particle unit level.

As the result, the contacting area of the main active agent, carbonate and organic acid in the effevescent tablet for a washing detergent 1D with water becomes large when the tablet 1D is put in water, thereby the carbonate reacts with the organic acid to cause the tablet 1D to be rapidly dissolved in water while generating carbon dioxide (CO₂).

Further, comparing with conventional powdered or granular washing detergent, if one effevescent tablet for a washing detergent 1D has the amount to be put in a washing tub of a washing machine at one time, only one tablet is required to be put in the washing tab for washing. Therefore, unlike conventional powdered or granular washing detergent, the tablet 1D can save the trouble of measuring with a scoop and putting in the tub, thereby facilitating its usage.

Furthermore, because of the tabletted shape of the effevescent tablet for a washing detergent 1D, there isn't a problem such that powdered or granular detergents are scattered around the washing machine or a person's hands or fingers get dirty with the detergents when the detergents are put in the washing tub.

Because anhydrous sodium sulphate granules 11··· with a hygroscopic property are included in the effevescent tablet for a washing detergent 1D, it is prevented from naturally foaming by the moisture contained in air while the tablet is stored. Namely, such an effevescent tablet is superior in storage stability.

In this embodiment, the granules of a main active ingredient 2A··· and 2B···, the carbonate granules 3···, the organic acid granules 4··· and the anhydrous sodium sulphate granules 11··· having almost the same particle diameter are used. However, the granules of a main active ingredient 2A··· and 2B···, the carbonate granules 3···, the organic acid granules 4··· and the anhydrous sodium sulphate granules 11··· may not have almost the same particle size diameter.

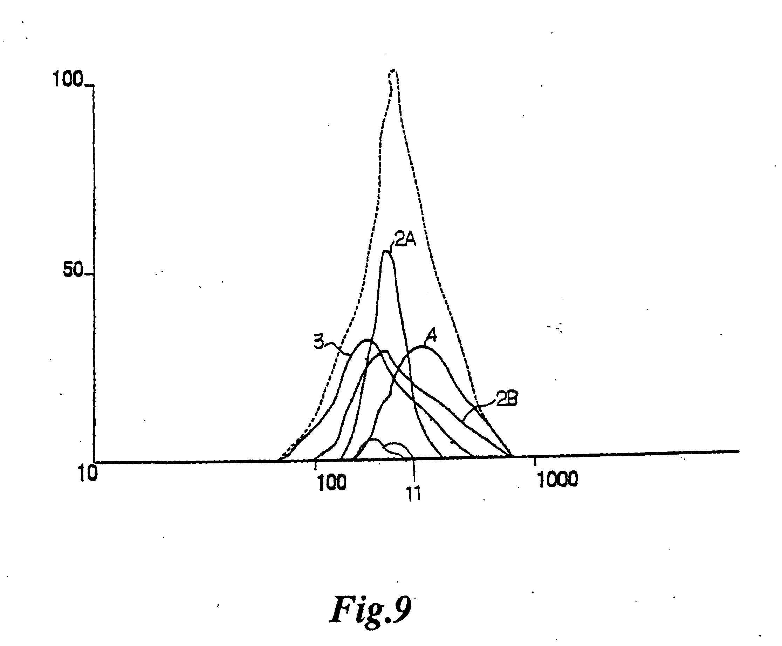

Fig.9 shows other embodiment of an effevescent tablet for a washing detergent 1D wherein granules of a main active ingredient 2A··· and 2B···, carbonate granules 3···, organic acid granules 4··· and anhydrous sodium sulphate granules 11··· are uniformly dispersed.

In Fig.9 the granules of a main active ingredient 2A··· and 2B···, the carbonate granules 3···, the organic acid granules 4··· and the anhydrous sodium sulphate granules 11··· are composed in such a manner that the particle size distribution of the mixture has a regular distribution having one peak after X mixing.

When the composed material of the granules of a main active ingredient 2A··· and 2H···, the carbonate granules 3···, the organic acid granules 4··· and the anhydrous sodium sulphate granules 11··· is mixed with a generally used mixer, it shows the same behavior against the external force given by the mixer when mixing proceeds as when one kind of powdered material having a normal particle size distribution with one peak is mixed. Therefore, the composed material can be uniformly mixed by itself without causing demixing phenomenon per each granule.

Such a method can produce an effevescent tablet for a washing detergent in which granules of a main active ingredient 2A··· and 2B···, carbonate granules 3···, organic acid granules 4··· and anhydrous sodium sulphate granules 11··· are uniformly dispersed.

Disintegraters, disintegration supplements, stabilizers, perfume agents, coloring agents, enzymes and dispersed agents are added in the effevescent tablet for a bath agent 1D if necessary.

Further, considering its storage stability, the final product of the effevescent tablet for a washing detergent 1D is preferably packaged in a press-through pack (PTP), a blister pack and a laminate pack per one tablet.

In this embodiment the effevescent tablet for a washing detergent 1D includes anhydrous sodium sulphate granules 11···, however, according to the effevescent tablet for a washing detergent of the present invention, anhydrous sodium sulphate granules 11··· aren't a indispensable material. The effevescent tablet for a washing detergent without including anhydrous sodium sulphate may be included in the present invention.

In the embodiment the granular anhydrous sodium sulphate is added in the effevescent tablet for a washing detergent 1D, however, powdered anhydrous sodium sulphate may be mixed in the granules of a main active ingredient 2A··· and 2H···, the carbonate granules 3··· and the organic acid granules 4···.

The effevescent tablet of the present invention is preferably used as an effevescent tablet for a bath agent.

For producing an effevescent tablet for bath agent, sodium carbonate granules are used as granules of a main active ingredient 2.

More specifically, sodium carbonate powders are granulated by means of a binder solution in which a water-soluble polymer is dissolved in water according to a fluid bed granulation method, thereby producing sodium carbonate granules.

Sodium hydrogen carbonate granules are for example used as carbonate granules 3.

More specifically, sodium carbonate hydrogen powders are granulated by means of a binder solution in which water-soluble polymers are dissolved in water according to a fluid bed granulation method, thereby producing sodium carbonate hydrogen granules.

Fumaric acid granules are for example used as organic salt granules 4.

More specifically, fumaric acid powders are granulated by means of a binder solution in which water-soluble polymers are dissolved in water according to a fluid bed granulation method, thereby producing fumaric acid granules.

In this case, each particle size diameter of the granules of a main active ingredient 2, the carbonate granules 3 and the organic acid granules 4 is arranged to be almost the same.

Or the granules of a main active ingredient 2, the carbonate granules 3 and the organic acid granules 4 may be blended so as to have a normal particle size distribution with one peak after they are mixed, like the production method shown in Fig.9.

Disintegraters, disintegration supplements, stabilizers, perfume agents, colorants and hot spring components are added in the effevescent tablet for bath agent 1D if necessary.

Then the granules of a main active ingredient 2, the carbonate granules 3 and the organic acid granules 4 after adding disintegraters, disintegration supplements, stabilizers, perfume agents, coloring agents and hot spring components if necessary are uniformly mixed to obtain a mixture M (molding material).

As shown in Fig.8c, lubricants aren't contained in the mixture M (molding material) and are sprayed on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Thereby a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. The mixture (molding material) M without including lubricants is compressed with the upper punch 31 on which face S31 (lower face, material contacting surface) lubricants are applied, the die 32 on which surface S32 (inner circumference, more specifically the material contacting surface above the upper face (material contacting surface) of the lower punch inserted into a fixed position in the die) lubricants are applied and the lower punch 33 on which surface S33 (upper face, material contacting surface) lubricants are applied, thereby the effevescent tablet for a bath agent (see the effevescent tablet 1A shown in Fig.3) is produced.

According to thus produced effevescent tablet for a bath agent, lubricants aren't contained in the mixture to be compressed with punches (see the upper punch 31 and the lower punch 33 in Fig.8c) and a die. Only a slight amount of lubricants is transferred on the surface of the tablet from the lubricant powders applied on the punches and the die (see the die 32 in Fig.8c), therefore, lubricants aren't contained in the tablet.

Herewith, if thus obtained effevescent tablet for a bath agent is dissolved in a hot water in a bath tub, an oil film doesn't appear on the water surface.

Further, the granules of a main active ingredient 2, the carbonate granules 3 and the organic acid granules 4 are uniformly dispersed in the effevescent tablet for a bath agent, therefore there are any variations of dissolving time in a hot water between the tablets. Namely, effevescent tablets for bath agent having the same quality can be supplied in a market.

The effevescent tablet 1A shown in Fig.3 may be produced as an effevescent tablet for a bath agent in the same manner mentioned above other than a binder solution in which water-soluble polymers and surfactants are dissolved in water is used for producing the granules of a main active ingredient 2, the carbonate granules 3 and the organic acid granules 4.

More specifically, the effevescent tablet for a bath agent like the effevescent tablet 1A shown in Fig.3 may be produced according to the following production method.

At first a granulated material in which a binder including a surfactant binds between the particles P2··· comprising the granules of a main active ingredient 2 is produced as the granules of a main active ingredient 2.

Further a granulated material in which a binder 7 including a surfactant 6 binds between the particles P3··· comprising carbonate granules 3 is produced as carbonate granules 2.

Furthermore a granulated material in which a binder 7 including a surfactant 6 binds between the particles P4··· comprising organic acid granules 4 is produced as organic acid granules 4.

The granulated material in which the particles P2··· comprising granules of a main active ingredient 2 are bound by the binder including a surfactant, the granulated material in which the particles P3··· comprising carbonate granules 3 are bound by the binder 7 including surfactants 6 and the granulated material in which the particles P4··· comprising organic acid granules 4 are bound by the binder 7 including surfactants 6 are blended, wherein other adjuncts excluding lubricant powders are added if necessary, and are uniformly mixed to obtain the mixture (molding material).

Lubricants aren't added in thus obtained mixture (molding material) M as shown in Fig.8c.

Lubricant powders are sprayed on the surface S31 (lower face, material contacting surface) of the upper punch 31, the surface S32 (inner circumference, more specifically the material contacting surface above the upper face (material contacting surface) of the lower punch inserted into a fixed position in a die) of the die 32 and the surface S33 (upper face, material contacting surface) of the lower punch 33. Thereby a minimum amount of lubricants is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

Then the mixture (molding material) M without including lubricants is compressed with the upper punch 31 on which face S31 (lower face, material contacting surface) lubricants are applied, the die 32 on which surface S32 (inner circumference, more specifically the material contacting surface above the upper face (material contacting surface) of the lower punch inserted into a fixed position in the die) lubricants are applied and the lower punch 33 on which surface S33 (upper face, material contacting surface) lubricants are applied, thereby the effevescent tablet for a bath agent like the effevescent tablet 1A shown in Fig.3 is produced.

According to the effevescent tablet for a bath agent, because particles comprising each granule 2···, 3···, 4··· are bound by the binder 7 including a surfactant 6, the binder 7 easily gets wet. Therefore, the tablet can achieve more rapid dissolving property when it is put in a hot water in the bath tub comparing with the effevescent tablet for a bath agent in which each granules 2···, 3···, 4··· isn't bound by the binder 5 without including surfactants 6.

Further, the effevescent tablet of the present invention can be preferably used as an effevescent tablet for oral administration.

For producing an effevescent tablet for oral administration, medicinal property granules are used for granules of a main active ingredient 2, for example vitamin granules such as vitamin C, antacid granules such as aldioxa, analgestic granules such as acetaminophen, non-narcotic antitussive granules like dextromethorphan hydrobromide, bronchodilator granules like dl-methylephedrine hydrochloride, antihistamic agent granules like chlorpheniramine maleate, caffeine granules like anhydrous caffeine. More than two of them may be combined and used as granules of a main active ingredient 2.

More specifically, medicinal property powders are granulated by means of a binder solution in which water-soluble polymers are dissolved in water according to a fluid bed granulation method, thereby obtaining granules of a main active ingredient 3.

Sodium hydrogen carbonate granules are for example used as carbonate granules 3.

More specifically, sodium hydrogen carbonate powders are granulated by means of a binder solution in which water-soluble polymers are dissolved in water according to a fluid bed granulation method, thereby obtaining sodium hydrogen carbonate granules.

Citric acid granules or tartaric acid granules are for example used as organic acid granules 4.

More specifically, citric acid powders are granulated by means of a binder solution in which water-soluble polymers are dissolved in water according to a fluid bed granulation method, thereby obtaining citric acid granules.

In this case the particle diameters of the granules of a main active agnet 2, the carbonate granules 3 and the organic acid granules 4 are arranged to be almost the same.

Or the granules of a main active ingredient 2, the carbonate granules 3 and the organic acid granules 4 may be blended so as to have a normal particle size distribution with one peak after they are mixed, like the production method shown in Fig.9.

Disintegrater, disintegration supplements, stabilizers, perfume agents, coloring agents and corrigent agents are added in the effevescent tablet for oral administration 1D if necessary.

Then the granules of a main active ingredient 2, the carbonate granules 3 and the organic acid granules 4 after adding disintegraters, disintegration supplements, stabilizers, perfume agents, coloring agents and corrigents if necessary are uniformly mixed to obtain a mixture M (molding material)

As shown in Fig.8c, lubricants aren't contained in the mixture M (molding material) and is sprayed on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Thereby a minimum amount of lubricants is applied on the surface S31 (lower face, material contacting surface) of the upper punch 31, the surface S32 (inner circumference, more specifically a material contacting surface above the upper face (material contacting surface) of the lower punch inserted into a fixed position in the die) of the die 32 and the surface S33 (upper face, material contacting surface) of the lower punch 33. The mixture (molding material) M without including lubricants is compressed with the upper punch 31 on which face S31 (lower face, material contacting surface) lubricants are applied, the die 32 on which surface S32 (inner circumference, more specifically the material contacting surface above the upper face (material contacting surface) of the lower punch inserted into a fixed position in the die) lubricants are applied and the lower punch 33 on which surface S33 (upper face, material contacting surface) lubricants are applied, thereby the effevescent tablet for oral administration (see the effevescent tablet 1 shown in Fig.2) is produced.

According to thus produced effevescent tablet for oral administration, lubricants aren't contained in the mixture to be compressed with punches (see the upper punch 31 and the lower punch 33 in Fig.8c) and the die (see the die 32 in Fig.8c). Only a slight amount of lubricants is transferred on the surface of the tablet from the lubricant powders applied on the punches (see the upper punch 31 and the lower punch 33 in Fig.8c) and the die (see the die 32 in Fig.8c), therefore, the lubricants aren't contained in the tablet.

Herewith, if thus obtained effevescent tablet for oral administration is dissolved in water or a hot water for dosing, an oil film doesn't appear on the resulting solution surface.

Further, granules of a main active ingredient 2, carbonate granules 3 and organic acid granules 4 are uniformly dispersed in the effevescent tablet for oral administration, therefore there are any variations of dissolving time in water or a hot water between the tablets. Namely, effevescent tablets for oral administration having the same quality can be supplied in a market.

The effevescent tablet 1A shown in Fig.3 may be produced as an effevescent tablet for oral administration in the same manner mentioned above other than a binder solution in which water-soluble polymers and surfactants are dissolved in water is used for producing granules of a main active ingredient 2, carbonate granules 3 and organic acid granules 4.

More specifically, the effevescent tablet for oral administration like the effevescent tablet 1A shown in Fig.3 may be produced according to the following production method.

At first a granulated material in which a binder including a surfactant binds between the particles P2··· comprising granules of a main active ingredient 2 is produced as granules of a main active ingredient 2.

Further a granulated material in which a binder 7 including a surfactant 6 binds between the particles P3··· comprising carbonate granules 3 is produced as carbonate granules 2.

Furthermore a granulated material in which a binder 7 including a surfactant 6 binds between the particles P4··· comprising organic acid granules 4 is produced as organic acid granules 4.

The granulated material in which the particles P2··· comprising granules of a main active ingredient 2 are bound by the binder including a surfactant, the granulated material in which the particles P3··· comprising carbonate granules 3 are bound by the binder 7 including surfactants 6 and the granulated material in which the particles P4··· comprising organic acid granules 4 are bound by the binder 7 including surfactants 6 are blended, wherein other adjuncts excluding lubricant powders are added if necessary, and are uniformly mixed to obtain the mixture (molding material).

Lubricant aren't added in thus obtained mixture (molding material) as shown in Fig.8c.

Lubricant powders are sprayed on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Thereby a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

Then the mixture (molding material) M without including lubricants is compressed with the upper punch 31 on which face S31 (lower face, material contacting surface) lubricants are applied, the die 32 on which surface S32 (inner circumference, more specifically a material contacting surface above the upper face (material contacting surface) of the lower punch inserted into a fixed position in the die) lubricants are applied and the lower punch 33 on which surface S33 (upper face, material contacting surface) lubricants are applied, thereby the effevescent tablet for oral administration is produced.

According to the effevescent tablet for oral administration, because particles comprising each granule 2···, 3···, 4··· are bound by the binder 7 including a surfactant 6, the binder 7 easily gets wet. Therefore, the tablet can achieve more rapid dissolving property when it is put in water or a hot water for use comparing with the effevescent tablet for a bath agent in which each granule 2···, 3···, 4··· is bound by the binder 5 without including surfactants 6.

The effevescent tablet 1B shown in Fig.4 may be produced as an effevescent tablet for oral administration in the same manner mentioned above other than a binder solution in which water-soluble polymers and a surfactant are dissolved in water is used for producing granules of a main active ingredient 2, the carbonate granules 3 and organic acid granules 4.

More specifically, the effevescent tablet for oral administration like the effevescent tablet 1B shown in Fig.4 may be produced according to the following production method.

At first a granulated material in which a binder 9 including a saccharide with high wettability for water 8 binds between the particles P2··· comprising the granules of a main active ingredient 2 is produced as granules of a main active ingredient 2.

Further a granulated material in which a binder 9 including a saccharide with high wettability for water 8 binds between the particles P3··· comprising carbonate granules 3 is produced as carbonate granules 3.

Furthermore a granulated material in which a binder 9 including a saccharide with high wettability for water 8 binds between the particles P4··· comprising organic acid granules 4 is produced as organic acid granules 4.

The granulated material in which the particles P2··· comprising granules of a main active ingredient 2 are bound by the binder 9 including a saccharide with high wettability for water 8, the granulated material in which the particles P3··· comprising carbonate granules 3 are bound by the binder 9 including a saccharide with high wettability for water 8 and the granulated material in which the particles P4··· comprising organic acid granules 4 are bound by the binder 9 including a saccharide with high wettability for water 8 are blended, wherein other adjuncts excluding lubricant powders are added if necessary, and are uniformly mixed to obtain the mixture (molding material).

Lubricants aren't added in thus obtained mixture (molding material).

As shown in Fig.8c lubricant powders are sprayed on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Thereby a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

Then the mixture (molding material) M without including lubricants is compressed with the upper punch 31 on which face S31 (lower face, material contacting surface) lubricants are applied, the die 32 on which surface S32 (inner circumference, more specifically the material contacting surface above the upper face (material contacting surface) of the lower punch inserted into a fixed position in the die) lubricants are applied and the lower punch 33 on which surface S33 (upper face, material contacting surface) lubricants are applied, thereby the effevescent tablet for oral administration is produced.

According to the effevescent tablet for oral administration, because particles comprising each granule 2···, 3···, 4··· are bound by the binder 9 including a saccharide with high wettability for water 8, the binder 9 easily gets wet. Therefore, the tablet can achieve more rapid dissolving property when it is put in water or a hot water for use comparing with the effevescent tablet for oral administration in which each granule 2···, 3···, 4··· isn't bound by the binder 5 without including a saccharide with high wettability for water 8.

The rapid dissolution property of the effevescent tablet for oral administration is realized by the saccharide with high wettability for water 8 included in the binder 9.

Thus, a surfactant isn't used for thus obtained effevescent tablet for administration, thereby having high safety comparing with the effevescent tablet for oral administration using surfactants.

The effevescent tablet 1C shown in Fig.5 may be produced as an effevescent tablet for oral administration in the same manner mentioned above other than a binder solution in which water-soluble high polymers and saccharides with high wettability for water and surfactants are dissolved in water is used for producing granules of a main active ingredient 2, carbonate granules 3 and organic acid granules 4.

More specifically, the effevescent tablet for oral administration like the effevescent tablet 1C shown in Fig.5 may be produced according to the following production method.

At first a granulated material in which a binder 10 including a surfactant 6 and a saccharide with high wettability for water 8 binds between the particles including granules of a main active ingredient P2··· comprising granules of a main active ingredient 2 is produced as the granules of a main active ingredient 2.

Further a granulated material in which a binder 10 including a surfactant 6 and a saccharide with high wettability for water 8 binds between the particles P3··· comprising the carbonate granules 3 is produced as carbonate granules 3.

Furthermore a granulated material in which a binder 10 including a surfactant 6 and a saccharide with high wettability for water 8 binds between the particles P4··· comprising organic acid granules 4 is produced as organic acid granules 4.

The granulated material in which the particles including granules of a main active ingredient P2··· comprising granules of a main active ingredient 2 are bound by the binder 10 including a surfactant 6 and a saccharide with high wettability for water 8, the granulated material in which the particles P3··· comprising carbonate granules 3 are bound by the binder 10 including a surfactant 6 and a saccharide with high wettability for water 8 and the granulated material in which the particles P4··· comprising organic acid granules 4 are bound by the binder 10 including a surfactant 6 and a saccharide with high wettability for water 8 are blended, wherein other adjuncts excluding lubricant powders are added if necessary, and are uniformly mixed to obtain the mixture (molding material).

Lubricants aren't added in thus obtained mixture M (molding material).

Then lubricant powders are sprayed on a surface S31 (lower face, material contacting surface) of an upper punch 31, a surface S32 (inner circumference, more specifically a material contacting surface above an upper face (material contacting surface) of a lower punch inserted into a fixed position in a die) of the die 32 and a surface S33 (upper face, material contacting surface) of the lower punch 33. Thereby a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

Then the mixture (molding material) M without including lubricants is compressed with the upper punch 31 on which face S31 (lower face, material contacting surface) lubricants are applied, the die 32 on which surface S32 (inner circumference, more specifically the material contacting surface above the upper face (material contacting surface) of the lower punch inserted into a fixed position in the die) lubricant are applied and the lower punch 33 on which surface S33 (upper face, material contacting surface) lubricants are applied, thereby the effevescent tablet for oral administration is produced.

According to the effevescent tablet for oral administration, because particles comprising each granule 2···, 3···, 4··· are bound by the binder 10 including a surfactant 6 and a saccharide with high wettability for water 8, the binder 10 easily gets wet. Therefore, the tablet can achieve more rapid dissolving property when it is put in water or a hot water for dosing comparing to the effevescent tablet for oral administration in which each granule 2···, 3···, 4··· isn't bound by the binder 5 without including a surfactant 6 and a saccharide with high wettability for water 8.

The rapid dissolution property of the effevescent tablet for oral administration is realized by the a surfactant 6 and the saccharide with high wettability for water 8 included in the binder 10.

Comparing to the effevescent tablet for oral administration which uses only a surfactant 6, the amount of surfactant contained in thus obtained effevescent tablet for oral administration can be reduced, therefore high safety is achieved.

By the way, according to the effevescent tablet, the effevescent tablet for a bath agent, the effevescent tablet for a washing detergent and the effevescent tablet for oral administration as mentioned above, lubricant powders are sprayed on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Thereby a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33. Then the mixture (molding material) is compressed with the upper punch 31 on which face S31 (lower face, material contacting surface) lubricants are applied, the die 32 on which surface S32 (inner circumference, more specifically a material contacting surface above an upper face (material contacting surface) of the lower punch inserted into a fixed position in the die) lubricants are applied and the lower punch 33 on which surface S33 (upper face, material contacting surface) lubricants are applied, thereby producing the effevescent tablet.

Namely, the effevescent tablet, the effevescent tablet for a bath agent, the effevescent tablet for a washing detergent and the effevescent tablet for oral administration of the present invention can be accomplished by the technology wherein a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

The inventors of the present invention have completed the technology wherein a minimum amount of lubricant is applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

Here the inventors of the present invention disclose an apply means and an apply method of lubricants for applying a minimum amount of lubricant on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

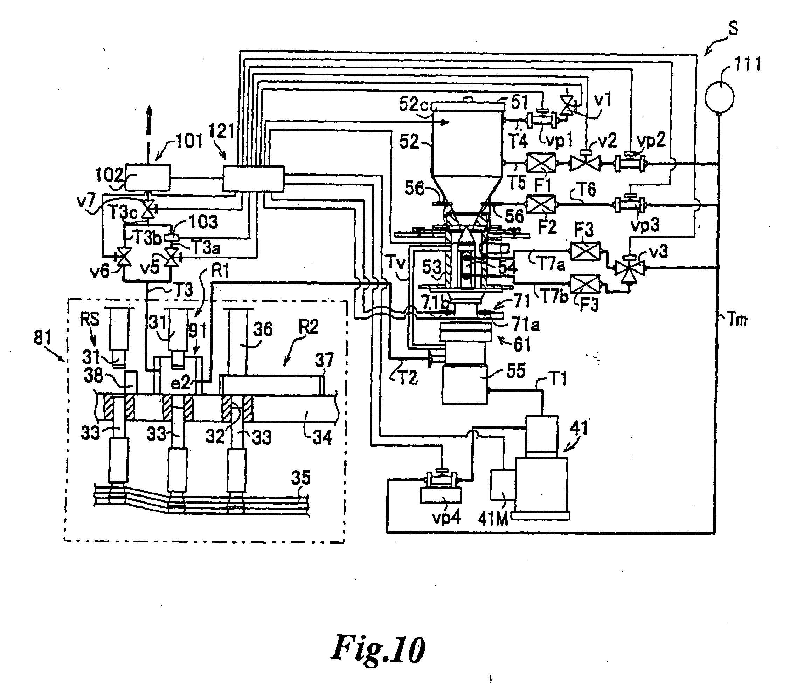

Fig.10 is an entire construction diagrammatically showing an external lubrication type tabletting machine which can continuously and stably apply a minimum amount of lubricant on each one of the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33.

The external lubrication type tabletting machine S is comprised of a pulsating vibration air generator 41, a quantitative feeder 51, a rotary type tabletting machine 81, a lubricant spray chamber (lubricant apply means) 91 provided at a fixed position of the rotary type tabletting machine 81, a lubricant suction means 101 for removing extra lubricants sprayed from a lubricant spray chamber (lubricant apply means) 91, an air source 111 such as a blower, and a processing unit 121 for controlling and supervising the entire external lubrication type tabletting machine S.

A conduit Tm connects between the air source 111 and the pulsating vibration air generator 41 of the external lubrication type tabletting machine S so that a compressed air generated by driving the air source 111 is supplied to the pulsating vibration air generator 41.

Between the pulsating vibration air generator 41 and the quantitative feeder 51 is connected with a conduit T1 so as to convert the compressed air fed through the conduit Tm into a positive pulsating vibration air and to supply into the conduit T1.

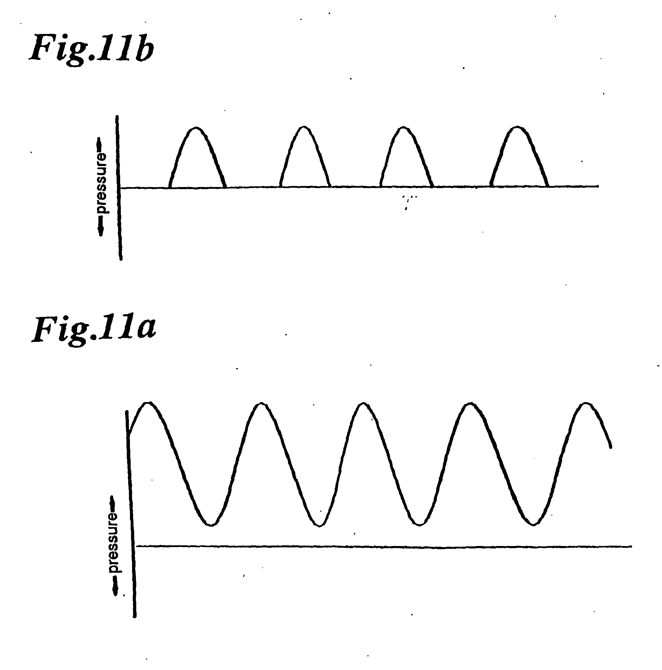

Fig.11 is an explanatory view exemplifying a positive pulsating vibration air.

The "pulsating vibration air" means an air wave of which pressure varies.

The "positive" means that the pressure is higher than the pressure (atmospheric pressure) out of the external lubrication type tabletting machine S.

The positive pulsating vibration air supplied in the conduit T1 may be a pulsating vibration air in which an amplitude peak is positive and an amplitude valley is atmospheric pressure as shown in Fig.11a or may be a pulsating vibration air in which both an amplitude peak and valley are positive as shown in Fig.11b.

The quantitative feeder 51 is connected to the lubricant spray chamber (lubricant apply means) 91 via a conduit T2.

When the positive pulsating vibration air is supplied in the quantitative feeder 51 via the conduit T1, lubricants (powder) are mixed with and dispersed in the pulsating vibration air. Thus obtained pulsating vibration air in which lubricant is mixed with and dispersed in is supplied in the conduit T2.

Then, the lubricants (powder) supplied with the positive pulsating vibration air are pneumatically transported in the conduit T2 together with the pulsating vibration air to be fed in the lubricant spray chamber (lubricant apply means) 91, and the lubricants are sequentially applied on the surface S31 (lower face, material contacting surface) of the upper punch 31, the surface S32 (inner circumference, more specifically the material contacting surface above the upper face (material contacting surface) of the lower punch inserted into a fixed position in the die) of the die 32 and the surface S33 (upper face, material contacting surface) of the lower punch 33, those punches 31, 33 and die 32 being accommodated in the lubricant spray chamber (lubricant apply means) 91.

The lubricant spray chamber (lubricant apply means) 91 and the lubricant suction means 101 are connected by a conduit T3.

When the lubricant suction means 101 is driven, the extra lubricants (powder) are suck to be removed via the conduit T3. The extra lubricants (powder) are those being applied on the surface S31 (lower face, material contacting surface) of the upper punch 31, the surface S32 (inner circumference, more specifically a material contacting surface above the upper face (material contacting surface) of the lower punch inserted into a fixed position in the die) of the die 32 and the surface S33 (upper face, material contacting surface) of the lower punch 33, while those punches 31, 33 and die 32 being accommodated in the lubricant spray chamber (lubricant apply means) 91.

Each member comprising the external lubrication type tabletting machine S will be more detailed hereinafter.

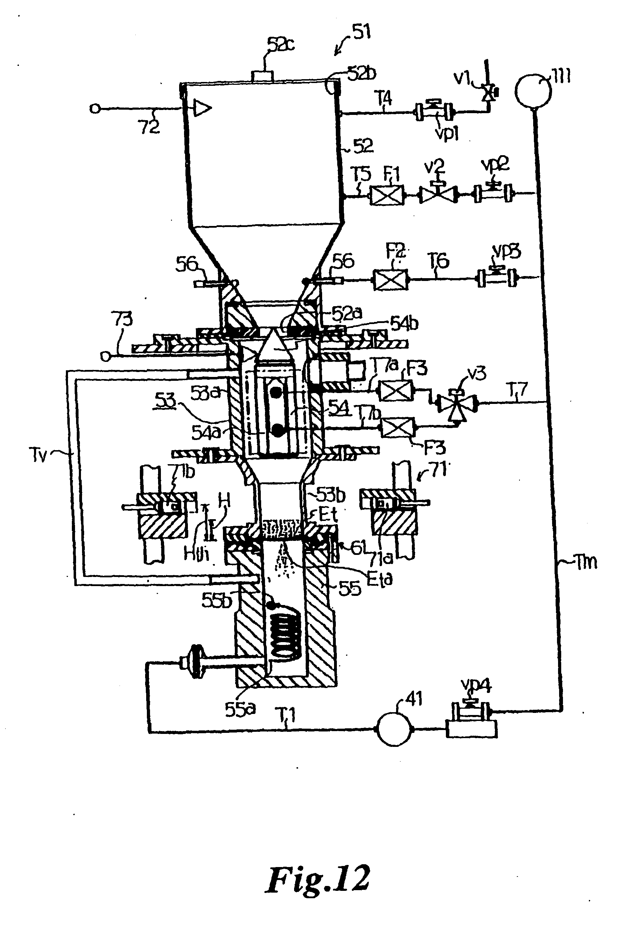

Fig.12 is an explanatory view diagrammatically showing a quantitative feeder.

The quantitative feeder 51 is comprised of a lubricant storage hopper 52, a tubular body 53 airtightly connected to a discharge port 52a of the lubricant storage hopper 52, a material feed valve 54 provided so as to be able to open and close the material discharge port 52a of the lubricant storage hopper 52, an elastic membrane Et provided so as to form a bottom of the tubular body 53 and a dispersion chamber 55 airtightly connected under the tubular body 53 via the elastic membrane Et.

In the lubricant storage hopper 52 gas injection means 56, 56 are provided around the material discharge port 52a.

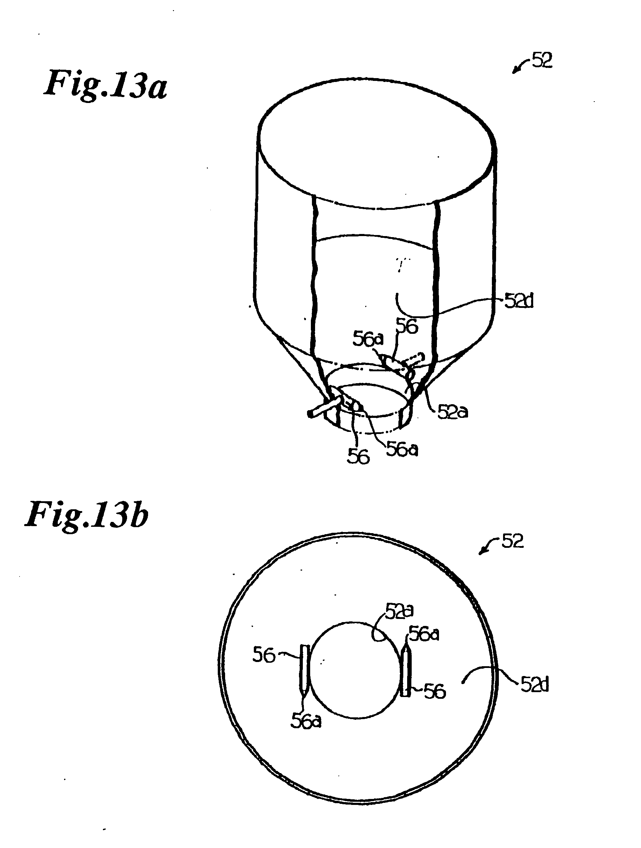

Fig.13 is an explanatory view showing the lubricant storage hopper 52 in more detail, Fig.13a is a perspective view diagrammatically showing the hopper 52 and Fig.13b is a plan view diagrammatically showing an essential part of the lubricant storage hopper 52 shown in Fig.13a.

The gas injection means 56, 56 are provided in a substantially tangential direction against the inner circumference of the lubricant storage hopper 52.

More specifically, each gas injection means 56, 56 is positioned at an outer circumference above the material discharge port 52a in a cone area 52d of the lubricant storage hopper 52 so as to be in a substantially tangential direction against the material discharge port 52a.

In Fig.12 and Fig.13, two gas injection means 56 are provided, however, the number of the gas injection means 56 isn't limited to two. One or more than three gas injection means may be provided. Further, if more than two gas injection means 56 are provided, they are arranged in such a manner that gas is injected in the same rotational direction from each gas injection port 56a··· of the gas injection means 56···.

The member indicated by the reference numeral 52c in Fig.12 is a cover detachably provided for the material feed port 52b of the lubricant storage hopper 52.

In this embodiment, the cover 52c is airtightly attached to the material feed port 52b of the lubricant storage hopper 52.

A conduit T4 is connected to the lubricant storage hopper 52 so as to be communicated with atmosphere.

The lubricant storage hopper 52 and the conduit Tm are connected with a conduit T5 and a switch valve v2 and a pressure control valve vp2 are provided in the midstream of the conduit T5.

The member indicated by the reference numeral F1 and provided in the midstream of the conduit T5 is a filter for removing dust in the air supplied in the conduit T5. The filter F1 may be provided if necessary.

Each gas injection means 56, 56 and the conduit Tm are connected with a conduit T6. In Fig.12 only the conduit T6 connected to one of the gas injection means 56 is shown and other conduit T6 is omitted.

A pressure control valve vp3 is provided for the conduit T6.

The member indicated by the reference numeral F2 provided in the midstream of the conduit T6 is a filter for removing dust in the air supplied in the conduit T6, however, the filter F2 is only provided if necessary.

In this embodiment the material feed valve 54 has a valve plug 54b and an open-close drive means (actuator) 54a for moving the valve plug 54b up and down.

Open and close of the material feed valve 54 is driven by air.

The material feed valve 54 and the conduit Tm are connected with a conduit T7.

The conduit T7 is branched into two pipes T7a and T7b to be connected with the open-close drive means (actuator) 54a of the material feed valve 54.

A switch valve v3 is provided in the midstream of the conduit T7. When the branch pipe T7a side of the control valve v3 is opened and the branch pipe side T7b side is closed, the valve plug 54b of the material feed valve 54 is moved down to open the material discharge port 52a of the lubricant storage hopper 52. When the branch pipe T7b side of the control valve v3 is opened and the branch pipe side T7a side is closed, the valve plug 54b of the material feed valve 54 is moved up to close the material discharge port 52a of the lubricant storage hopper 52.

The member indicated by the reference numeral F3 provided in the midstream of the branch pipe T7a and T7b is a filter for removing dust in the air supplied in the conduit T7, however, the filter F3 is only provided if necessary.

Next the construction of the elastic membrane Et will be explained.

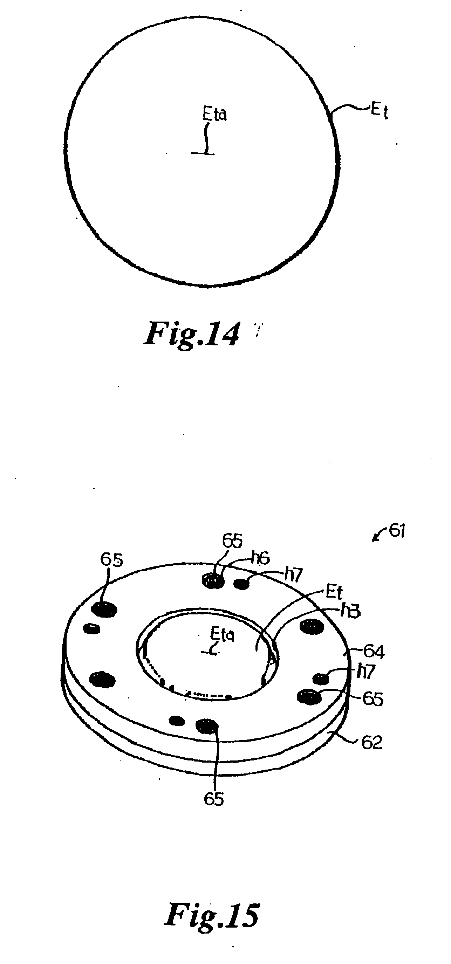

Fig.14 is a plan view diagrammatically showing the elastic membrane Et.

The elastic membrane Et is made of an elastic material such as a synthetic rubber like a silicone rubber and has a penetrating aperture Eta at the center. In this embodiment, the aperture Eta of the elastic membrane Et is formed like a slit.

The elastic membrane Et is installed between the tubular body 53 and the dispersion chamber 55 by means of an elastic membrane installation means 51.

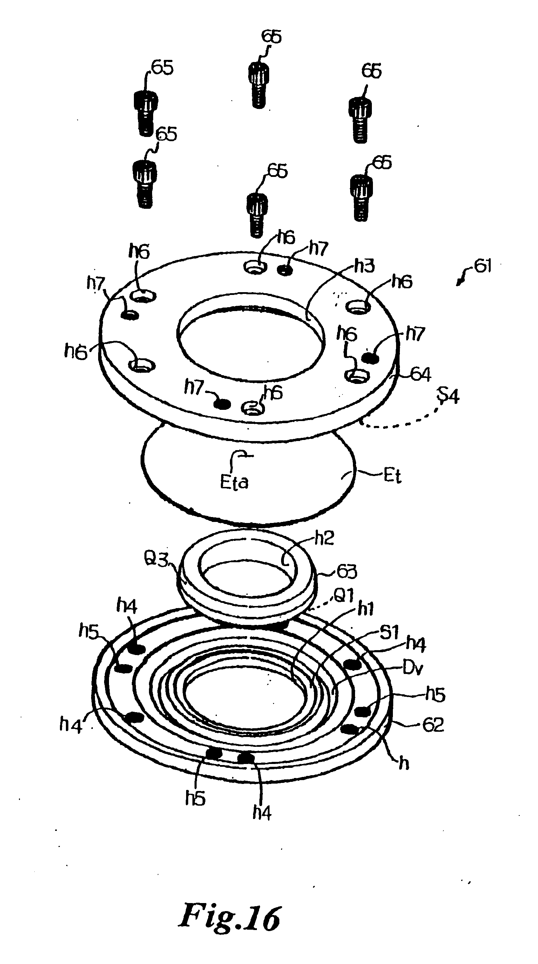

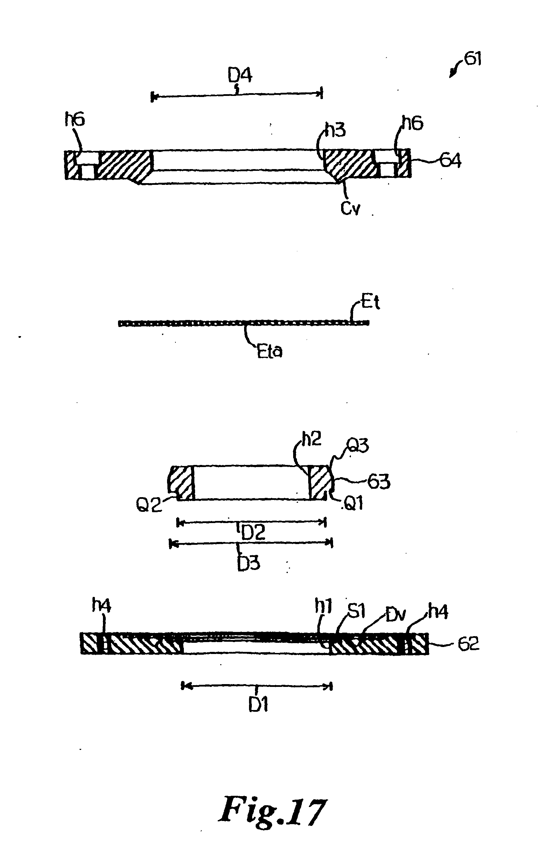

Fig.15 is a perspective view when the elastic membrane is attached on the elastic membrane installation means of the quantitative feeder 51. Fig.16 is an exploded view diagrammatically showing the construction of the elastic membrane installation means shown in Fig.15. Fig.17 is a sectional view diagrammatically showing the construction of the elastic membrane installation means shown in Fig.15.

The elastic membrane installation means 61 has a pedestal 62, a push-up member 63 and a presser member 64.

The pedestal 62 has a hollow part h1 and a ring-like platform S1 for placing the push-up member 63 is provided at the periphery of the hollow part h1. Further, a V-groove Dv is provided for the pedestal 62 so as to surround the hollow part h1 like a ring.

The push-up member 63 has a hollow part h2. In this embodiment, the push-up member 63 has a stepped part Q1 at its lower part as shown in Fig. 17 in such a manner that the part Q1 is positioned on the platform S1 of the pedestal 62 when the push-up member 63 is placed on the pedestal 62.

When the push-up member 63 is placed on the pedestal 62 in this embodiment, a lower extended part Q2 formed so as to be extended downward from the step Q1 of the push-up member 63 is designed to be incorporated in the hollow part h1 of the pedestal 62. Namely, the lower extended part Q2 of the push-up member 63 is precisely processed in such a manner that its outer diameter D2 is almost the same or a little smaller than the inside diameter

### D1 of the hollow part h1 of the pedestal 62.

Furthermore in this embodiment, an inclined plane extending from top to bottom in a sectional view is provided at the periphery of an upper part Q3 of the push-up member 63.

The presser member 64 has a hollow part h3. A ring-like V-shaped projection Cv is provided for a surface S4 of the presser member 64 facing the pedestal 62 so as to be incorporated in the V-groove Dv on the surface of the pedestal 62.

The member indicated by a numeral 65 in Fig.15 and Fig.16 shows fastening means such as a bolt.

The hole shown as h4 in Fig.16 is a fixing hole of the fastening means 65 formed on the pedestal 62, and the hole shown as h6 is a fixing hole of the fastening means 65 formed on the presser member 64 respectively. The hole shown as h5 in Fig.16 is a fixing hole of the pedestal 62 for attaching the elastic membrane installation means 61 to a desired device by means of fixing means such as a bolt (not shown). The hole h7 of the presser member 64 is for attaching the elastic membrane installation means 61 to a desired device by means of fixing means such as a bolt (not shown).

In this embodiment, the inside diameter D4 of the hollow part h3 of the presser member 64 is precisely processed so as to be the same as or a litter larger than the external diameter D3 of the push-up member 63.

Next, installation procedures of the elastic membrane Et on the elastic membrane installation means 61 will be explained hereinafter.

The push-up member 63 is placed on the surface of the pedestal 2 at first for installing the elastic membrane Et on the elastic membrane installation means 61.

Then, the elastic membrane Et is placed on the push-up member 63.

The presser member 64 is placed on the push-up member 63 so as to cover both the push-up member 63 and the elastic membrane Et in such a manner that each fixing hole h4 ··· on the pedestal 62 is aligned with each fixing hole h6 ··· on the presser member 64.

Next, the presser member 4 is fastened to the pedestal 62 by screwing each fastening means such as a bolt 65 ··· into each fastening hole h4 ··· and each corresponding fastening hole h6 ···.

Accordingly, the elastic membrane Et is placed on the push-up member 63 on the pedestal 62 of the elastic membrane installation means 61 and the presser member 64 is fastened to the pedestal 62 so that the elastic membrane Et is pushed upward to the presser member 64 by the push-up member 63. As a result, the elastic membrane Et is extended from its inside to its periphery by being pushed upward into the presser member 64.

At first, the elastic membrane Et extended by the push-up member 63 is gradually inserted between the V-groove Dv formed on the pedestal 62 and the V-shaped projection Cv formed on the surface of the presser member 64 facing the pedestal 62 via the space between the periphery Q3 of the push-up member 63 and the surface (inner surface) forming the hollow part h3 of the presser member 64.

Furthermore, as the presser member 64 is fastened to the pedestal 62 by means of the fastening means such as a bolt 65 ···, the elastic membrane Et comes to be held between the periphery Q3 of the push-up member 63 and the inner surface of the hollow part h3 of the presser member 64 while being pushed up into the presser member 64 by the push-up member 63. When the elastic membrane Et is further pushed up into the presser member 64 by the push-up member 63, the extended part of the elastic membrane Et from inside to outside is held between the V-groove Dv of the pedestal 62 and the V-shaped projection Cv on the surface of the presser member 64 facing the pedestal 62.

In other words, according to the elastic membrane installation means 61, the elastic membrane Et is placed on the push-up member 63 on the pedestal 62 and the presser member 64 is fastened to the pedestal 62, then the elastic membrane Et is pushed up to the presser member 64 by the push-up member 63, thereby the elastic membrane Et is kept being stretched from its inside to outside. Furthermore, the periphery of the elastic membrane Et extended by the push-up member 63 is held between the V-groove Dv of the pedestal 62 and the V-shaped projection Cv provided on the face opposing the pedestal 62 of the presser member 64. As a result, the elastic membrane installation means 61 can keep the elastic membrane Et stretched only by a simple operation such that the elastic membrane Et is placed on the push-up member 63 on the pedestal 62 and the presser member 64 is fastened to the pedestal 62.

In addition, the inclined plane Q3 enlarging from top to bottom in its section is provided at the periphery of the push-up member 63 of the elastic membrane installation means 61.

The inclined plane Q3 is an important element of the elastic membrane installation means 61 and is detailed hereinafter.

The inclined plane Q3 which is enlarged from top to bottom is provided for the periphery of the push-up member 63 of the elastic membrane installation means 61. Therefore, the extended part of the elastic membrane Et from inside to outside by being pushed up into the presser member 64 is easily moved between the V-groove Dv annularly formed on the pedestal 62 and the V-shaped projection Cv annularly formed on the surface of the presser member 64 facing the pedestal 62.

More specifically, when the external diameter of the inclined plane Q3 of the push-up member 63 is substantially smaller than the inner diameter D4 of the hollow part h3 of the presser member 64, there is an adequate gap (space) between the inclined plane Q3 of the push-up member 63 and the surface forming the hollow part h3 of the presser member 64, thereby the extended part of the elastic membrane Et from inside to outside by the push-up member 63 being easily guided to the V-groove Dv annularly provided on the surface of the pedestal 62 by the gap.

The inclined plane Q3 of the periphery of the push-up member 63 is designed so as to be enlarged from top to bottom in a sectional view. Therefore, the extended part of the elastic membrane Et from inside to outside by the push-up member 63 is guided to the V-groove Dv annularly provided on the pedestal 62 along the surface of the inclined plane Q3.

Then the presser member 64 is fastened to the pedestal 62 by screwing each fastening means such as a bolt 65 ··· into each fixing hole h4 ··· and each corresponding fixing hole h6 ···. Accordingly the external diameter of the inclined plane Q3 of the push-up member 63 gets closer to the inner diameter D4 of the hollow part h3 of the presser member 64. When the gap (space) between the inclined plane Q3 of the push-up member 63 and the surface consisting the hollow part h3 of the presser member 64 becomes about the thickness (wall thickness) of the elastic membrane Et, the elastic membrane Et comes to be held between the inclined plane Q3 of the push-up member 63 and the surface consisting the hollow part h3 of the presser member 64.

According to the above-mentioned operations, the elastic membrane Et is placed on the push-up member 63 on the pedestal 62 of the elastic membrane installation means 61, then the presser member 64 is fastened to the pedestal 62 by means of a simple operation of fixing means such as a bolt 65 ···, thereby keeping the elastic membrane Et strained.

When the presser member 64 is fastened to the pedestal 62 by means of the fixing means such as a bolt 65···, the distance between the inclined plane Q3 of the periphery of the push-up member 63 and the inner circumference of the hollow part h3 of the presser member 64 becomes small, and the elastic membrane Et is tightly held between the inclined plane Q3 of the push-up member 63 and the inner circumference of the hollow part h3 of the presser member 64, preventing the elastic membrane Et from being slack.

Further if the elastic membrane Et is attached on the elastic membrane installation means 61, it is doubly locked between the inclined plane Q3 of the push-up member 63 and the surface consisting the hollow part h3 of the presser member 64 and between the V-shaped projection Cv annularly provided on the surface of the presser member 64 facing the pedestal 62 and the V-groove Dv annularly provided on the pedestal 62. Thereby, the elastic membrane Et doesn't get slack after the presser member 64 is fastened to the pedestal 62.

According to the quantitative feeder 51, the presser member 64 of the elastic membrane installation means 61 on which the elastic membrane Et is attached is airtightly installed at the lower part of the tubular pipe 53 and the pedestal 62 is airtightly provided on the top of the dispersion chamber 55.

The quantitative feeder 51 has a bypass pipe Tv between the dispersion chamber 55 and tubular body 53 as shown in Fig.12. The bypass pipe Tv is provided so as to rapidly get the pressure in the dispersion chamber 55 and that in the tubular body 53 balanced.

A level sensor 71 for detecting the amount of lubricants (powder) stored on the elastic membrane Et in a lower tubular body 53b is provided for the lower tubular part 53b. The level sensor 71 has a light emitting element 71a for generating light such as infrared rays and visible rays and a light receiving element 71b for receiving the light generated from the light emitting element 71a. The light emitting element 71a and the light receiving element 71b are provided to be opposed each other so as to interpose the lower tubular part 53b.

The amount of lubricants (powder) stored on the elastic membrane Et in the lower tube 53b can be detected at a position Hth (at height where the level sensor 71 is provided above the elastic membrane Et).

Namely, when the amount of lubricants (powder) stored on the elastic membrane Et in the lower tube 53b exceeds the position Hth (height where the level sensor 71 is provided above the elastic membrane Et), the light radiated from the light emitting element 71a is blocked off by the lubricants (powder) and isn't received by the light receiving element 71b (being off). Then it can be detected that the height H of the lubricant stored on the elastic membrane Et in the lower tube 53b exceeds the height Hth (H>Hth).

On the other hand, when the amount of lubricants (powder) stored on the elastic membrane Et in the lower tube 53b becomes lower than the position Hth (height where the level sensor 71 is provided above the elastic membrane Et), the light emitted from the light emitting element 71a can be received by the light receiving element 71b (being on). Then it can be detected that the height H of the lubricants (powder) stored on the elastic membrane Et in the lower tube 53b is under the height Hth (H<Hth).

In this embodiment the material feed valve 54 moves up and down depending on the detected values of the level sensor 71 so as to open and close the discharge port 52a of the material storage hopper 52. More specifically according to the quantitative feeder 51, the light emitting element 71a of the level sensor 71 is turned on while the quantitative feeder 51 is driven. When the light from the light emitting element 71a doesn't come to be received in the light receiving element 71b (being off), the material feed valve 54 is moved up to close the discharge port 52a of the material storage hopper 52. When the light from the light emitting element 71a is received by the light receiving element 71b (being on), the material feed valve 54 is moved down to open the discharge port 52a of the lubricant storage hopper 52 until the light isn't received by the light receiving element 71b (being off), thereby approximately the same quantity of lubricants (powder) is always stored on the elastic membrane Et in the lower tube 53b while the quantitative feeder 51 is driven.

The inner shape of the dispersion chamber 55 is designed to be approximately tubular so as to make a positive pulsating vibration air swirl therein. In this embodiment, such a dispersion chamber 55 of which inner shape is tubular is used, however, its shape isn't limited as long as a positive pulsating vibration air easily swirls therein. Therefore, the inner shape isn't limited to be approximately tubular.

The lower tube 53b of the cylindrical body 53 is made of clear resin, specifically a light permeable material such as glass, acrylate resin, polycarbonate resin, and so on.

Further, it is preferable that the lower tube 53b is made of polycarbonate and its inner circumferential wall is mirror finished.

It is because that if the lower tubular body 53b is made of polycarbonate and its inner circumferential wall is mirror finished, a powdered material is hardly adhered on the inner circumference of the tubular body 53b comparing with the case when other material is used, thereby obtaining high detection accuracy of the level sensor 71.

The pulsating vibration air supply port 55a is provided at a lower part of the dispersion chamber 55 in approximately a tangential direction of the inside perimeter of the chamber 55. The discharge port 55b is provided at an upper part of the dispersion chamber 55 in approximately a tangential direction of the inside perimeter of the chamber 55. A conduit T5 is connected to the pulsating vibration air supply port 55a and a conduit (for example see the conduit T6 in Fig.12) is connected to the pulsating vibration air discharge port 55b.

Again, explained in reference to Fig.12, the pulsating vibration air supply port 55a is provided at a lower part of the dispersion chamber 55 in approximately a tangential direction of the inside perimeter of the dispersion chamber 55 and the discharge port 55b is provided at an upper part of the dispersion chamber 55 in approximately a tangential direction of the inside perimeter of the dispersion chamber 55.

The pulsating vibration air supply port 55a of the dispersion chamber 55 and the pulsating vibration air generation means 41 are connected with a conduit T1 in such a manner that when the pulsating vibration air generation means 41 is driven, a positive pulsating vibration air generated from the pulsating vibration air generation means 41 is supplied in the dispersion chamber 55 via the conduit T.

The discharge port 55b and the lubricant spray chamber (lubricant spray chamber (lubricant apply means) 91 shown in Fig.10) are connected with a conduit (the conduit T2 shown in Fig.10).

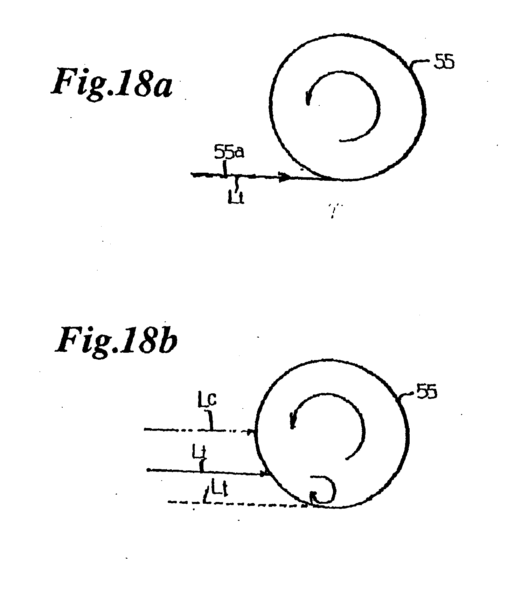

Here the position of the pulsating vibration air supply port 55a provided for the dispersion chamber 55 is detailed referring to Fig.18.

Fig.18 is a plan view diagrammatically showing a position of the pulsating vibration air supply port 55a provided for the dispersion chamber 55 when the chamber 55 is seen from top, Fig.18a is an explanatory view showing a preferable position for providing the pulsating vibration air supply port 55a against the dispersion chamber 55 and Fig.18b is an explanatory view showing an actual position for providing the pulsating vibration air supply port 55a against the dispersion chamber 55.

The curved arrows in Fig.18a and Fig.18b diagrammatically show the directions of the swirling positive pulsating vibration air generated in the dispersion chamber 55.

The pulsating vibration air supply port 55a is preferably provided in a substantially tangential direction (a direction shown with a dashed line Lt in Fig.18a) against the inside perimeter of the dispersion chamber 55 in order to generate a swirling positive pulsating vibration air in the dispersion chamber 55.

However, the supply port 55a isn't always provided in a tangential direction against the inside perimeter of the chamber 55 as shown in Fig.18a. It may be provided in an equivalent direction (namely, in a direction parallel to the tangential direction (a direction shown with a dashed line Lt in Fig.18b) of the inner circumference of the dispersion chamber 55, shown with a dashed line Lt in Fig.18b) to the tangential direction (a direction shown with a dashed line Lt in Fig.18b) as far as one dominant swirling flow is generated in the dispersion chamber 55.

If the pulsating vibration air supply port 55a is provided in a direction into a center line of the dispersion chamber 55 as shown with an imaginary line Lc in Fig.18b, two swirls, both of which don't seem a dominant flow, are generated when the inner shape of the dispersion chamber 55 is approximately cylindrical. Therefore, it isn't preferable to provide the supply port 55a in such a position considering generation of the swirling positive pulsating vibration air in the dispersion chamber 55.

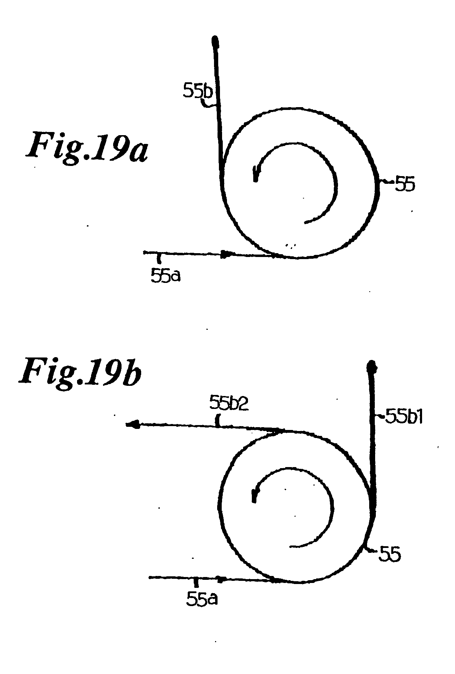

Next, the positional relation of the pulsating vibration air supply port 55a and the discharge port 55b in the dispersion chamber 55 is detailed referring to Fig.19.

Fig.19 is a plan view diagrammatically showing a position of the pulsating vibration air supply port 55a and its discharge port 55b provided for a dispersion chamber 55 when the chamber is seen from top, Fig.19a is an explanatory view showing a preferable position for providing the pulsating vibration air supply port 55a and its discharge port 55b against the dispersion chamber 55 and Fig.19b is an explanatory view showing an actual position for providing the pulsating vibration air supply port 55a and its discharge port 55b against the dispersion chamber 55.

The curved arrows in Fig.19a and Fig.19b diagrammatically show directions of the swirling positive pulsating vibration air generated in the dispersion chamber 55.

When the discharge port 55b is provided for the dispersion chamber 55 as shown in Fig.19a, the position of the port 55b becomes opposite to the direction of the swirling pulsating vibration air (movement of the air flow) generated in the chamber 55. In such a case, the discharge efficiency of the lubricants (powder) fluidized by being dispersed in air from the discharge port 55b can be set low.

Contrary if the discharge efficiency of the fluidized lubricant from the discharge port 55b is to be heightened, the port 55b is preferably provided in a forward direction of the swirling positive pulsating vibration air generated in the dispersion chamber 55 like the discharge port 55b1 or 55b2 illustrated in Fig.19b.

The inner shape of the dispersion chamber 55 is designed to be approximately tubular so as to make a positive pulsating vibration air swirl therein. In this embodiment, such a dispersion chamber 55 is used, however, its inner shape isn't limited to be tubular as long as a positive pulsating vibration air easily swirls therein. Therefore, the inner shape isn't limited to be approximately tubular.

The member 72 in Fig.12 is a pressure sensor for measuring the pressure in the lubricant storage hopper 52 and the member 73 is a pressure sensor for measuring the pressure in the tubular body 53.

In the external lubrication type tabletting machine S, as shown in Fig.10, the processing unit 121 and each member v1, v2, v3, v5, v6, v7, vp1, vp2, vp3, 41, 71, 72, 73, 102 and 111 are connected by signal lines so as to be able to drive, stop or control each member v1, v2, v3, v5, v6, v7, vp1, vp2, vp3, 41, 71, 72, 73, 102 and 111.

Then operations of the quantitative feeder 51 is explained.

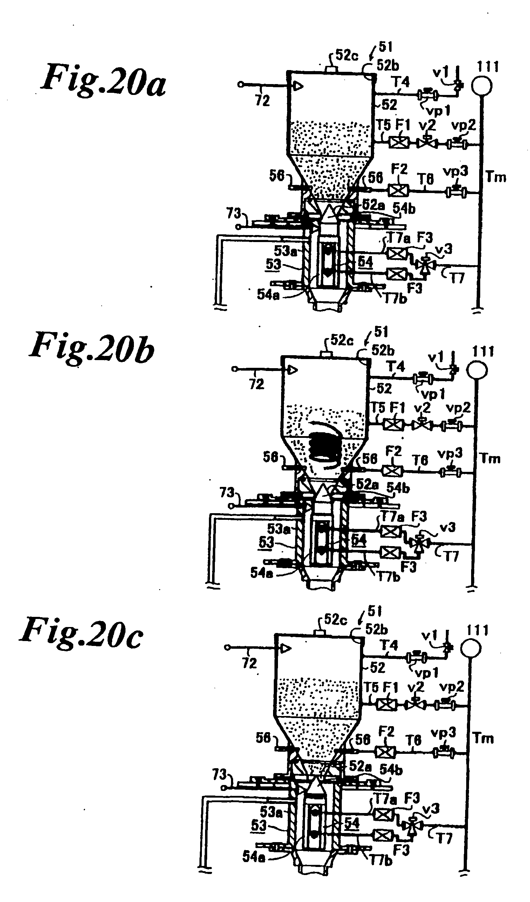

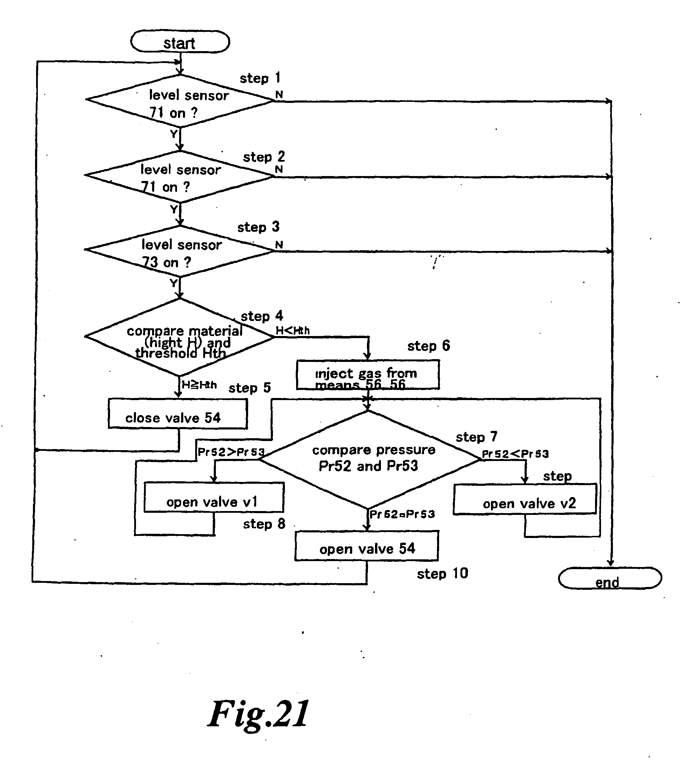

Fig.20 is an explanatory view diagrammatically showing operations of the gas injection means 56, 56 and the material feed valve 54 provided for the lubricant storage hopper 52 of the quantitative feeder 51. Fig.21 is a flow chart diagrammatically showing operation programs of the gas injection means 56, 56 and the material feed valve 54 stored in a memory of the processing unit 121 in advance.

The open and close operations of the material feed valve 54 are executed as follows in the quantitative feeder 51.

At an initial condition, the material feed valve 54 of the quantitative feeder 51 closes the material discharge port 52a of the lubricant storage hopper 52.

An operator stores lubricant powders in the lubricant storage hopper 52 and attaches a cover 52c on the material feed port 52b (See Fig.20a).

Next, an air source 111 is driven. Simultaneously the rotary cam 45 of the pulsating vibration air generation means 41 is rotated at a specified rotational speed so that a positive pulsating vibration air with a fixed flow amount, pressure and frequency and a desired wave shape is supplied in the conduit T1.

Each pressure control valve vp1, vp2, vp3 and vp4 is controlled.

At an initial condition, each switch valve v1, v2 and v3 is kept to be closed.

The level sensor 71 is actuated (see step 1) and each pressure sensor 72, 73 is also actuated (see step 2 and 3).

The light emitted from the light emitting element 71a of the level sensor 71 is received in the light receiving element 71b. The signal indicating the light receiving element 71b has received the light emitted from the light emitting element 71a is sent to the processing unit 121.

When the processing unit 121 receives the signal indicating the light receiving element 71b has received the light emitted from the light emitting element 71a, the processing unit 121 decides that the height H of the lubricant powders on the elastic membrane Et in the tubular body 54 is under a threshold Hth (see step 4).

In this case the processing unit 121 opens the switch valve v1 at a step 6 and keeps the pressure control valve vp3 opened for a predetermined time. Thereby, gas is injected from the gas injection means 56, 56 for a predetermined time so as to destroy the caked part even if such a part is generated in the lubricant powders stored in the lubricant storage hopper 52 (See Fig.20b).

The pressure (Pr52) in the lubricant storage hopper 52 measured by the pressure sensor 72 and the pressure (Pr53) in the tubular body 53 measured by the pressure sensor 73 are sent to the processing unit 121.

When the processing unit 121 receives a signal indicating gas has injected for a fixed time from the gas injection means 56, 56 (signal showing the pressure control valve vp3 is opened for a fixed time and closed thereafter), the pressure (Pr52) in the lubricant storage hopper 52 and the pressure (Pr53) in the tubular body 53 after gas is injected for a fixed time are compared (see step 7).

When the processing unit 121 detects that the pressure (Pr52) in the lubricant storage hopper 52 is the same as the pressure (Pr53) in the tubular body 53 (pressure Pr52 = pressure Pr53) in the step 7, the unit 121 keeps the material feed valve 54 opened. Namely, in this embodiment, a processing unit (not shown) keeps the branch pipe T7a side of the switch valve v3 opened, and the branch pipe T7b side closed.

Thereby, the material feed valve 54 is opened to discharge the lubricant powders stored in the lubricant storage hopper 52 into the tubular body 53 (see Fig.20c).

Then, the processing unit 121 receives the signal indicating that the light receiving element 71b doesn't receive the light emitted from the light emitting element 71a of the level sensor 71, the material feed valve 54 is closed. Namely in this embodiment, the processing unit 121 closes the branch pipe T7a side of the switch valve v3 and opens the branch pipe T7b side (See step 10).

Therefore, the material feed valve 54 is closed (See Fig.20a). The processing unit 121 detects that the pressure (Pr52) in the lubricant storage hopper 52 is higher than the pressure (Pr53) in the tubular body 53 (Pr52 > Pr53) in the step 7, the unit 121 keeps the switch valve v1 opened until the pressure (Pr52) in the lubricant storage hopper 52 becomes equal to the pressure (Pr53) in the tubular body 53. When the pressure (Pr52) in the lubricant storage hopper 52 becomes substantially equal to the pressure (Pr53) in the tubular body 53, the switch valve v1 is closed again (see step 7 and step 8). Thereafter, the processing unit 121 detects that the pressure (Pr52) in the lubricant storage hopper 52 is the same as the pressure (Pr53) in the tubular body 53 (Pr52 = Pr53) in the step 7, the unit 121 keeps the material feed valve 54 opened. Namely, in this embodiment, a processing unit 121 keeps the branch pipe T7a side of the switch valve v3 opened, and the branch pipe T7b side closed (see step 10).

Then, the processing unit 121 receives the signal indicating that the light receiving element 71b doesn't receive the light emitted from the light emitting element 71a of the level sensor 71, the material feed valve 54 is closed. Namely in this embodiment, the processing unit 121 closes the branch pipe T7a side of the switch valve v3 and opens the branch pipe T7b side (see step 5).

The processing unit 121 detects that the pressure (Pr52) in the lubricant storage hopper 52 is lower than the pressure (Pr53) in the tubular body 53 (Pr52 < Pr53) in the step 7, the unit 121 keeps the switch valve v2 opened until the pressure (Pr52) in the lubricant storage hopper 52 becomes equal to the pressure (Pr53) in the tubular body 53. When the pressure (Pr52) in the lubricant storage hopper 52 becomes substantially equal to the pressure (Pr53) in the tubular body 53, the switch valve v2 is closed again (see step 7 and step 8). Thereafter, the processing unit 121 detects that the pressure (Pr52) in the lubricant storage hopper 52 is the same as the pressure (Pr53) in the tubular body 53 (Pr52 = Pr53) in the step 7, the unit 121 keeps the material feed valve 54 opened. Namely, in this embodiment, a processing unit 121 keeps the branch pipe T7a side of the switch valve v3 opened, and the branch pipe T7b side closed (see step 10).

The processing unit 121 receives the signal indicating that the light receiving element 71b doesn't receive the light emitted from the light emitting element 71a of the level sensor 71, the material feed valve 54 is closed. Namely in this embodiment, the processing unit 121 closes the branch pipe T7a side of the switch valve v3 and opens the branch pipe T7b side (see step 5).

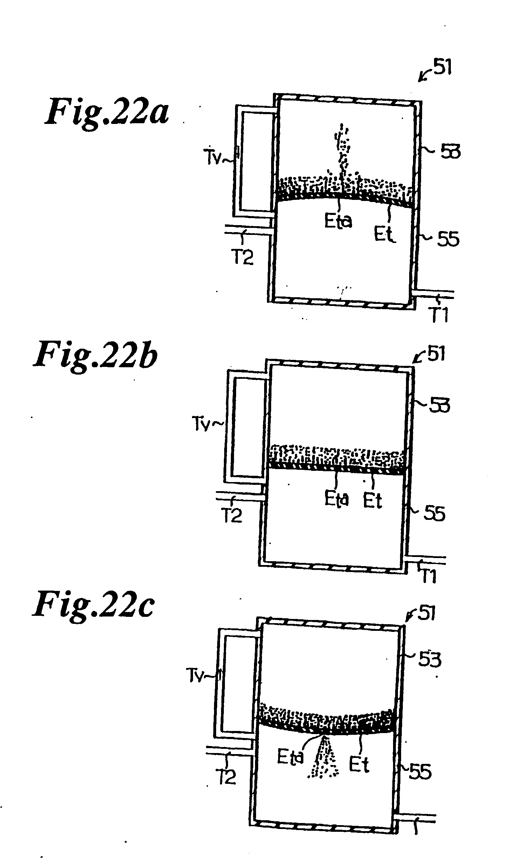

Fig.22 is an explanatory view diagrammatically showing operations of the elastic membrane Et and the bypass pipe Tv when a positive pulsating vibration air is supplied in the dispersion chamber 55.

When the pulsating vibration air generation means 41 is driven, a positive pulsating vibration air with a desired flow amount, pressure, wavelength, wave shape is supplied in the conduit T1.

The positive pulsating vibration air supplied in the conduit T1 is supplied from a pulsating vibration air supply port 55aa to the dispersion chamber 55.

The positive pulsating vibration air supplied in the dispersion chamber 55 becomes a positive pulsating vibration air swirling upwardly like a convolution such as a tornado therein, then is discharged from the discharge port 55b.

The swirling positive pulsating vibration air generated in the dispersion chamber 55 doesn't lose its nature as a pulsating vibration air so that the elastic membrane Et vibrates according to the frequency, amplitude, and wave shape of the positive pulsating vibration air.

At a peak of the positive pulsating vibration air supplied to the dispersion chamber 55 and when the pressure Pr55 in the dispersion chamber 55 becomes higher than the pressure Pr53 in the tubular body 53 (pressure Pr55 > pressure Pr53), the elastic membrane Et is elastically deformed so as to be curved upwardly as shown in Fig.22a.

A penetrating aperture Eta becomes V-shaped with its upper end opened in a sectional view and a part of the lubricant powders stored on the elastic membrane Et in the tubular body 53 falls in the V-shaped aperture Eta.

An air communication passage between the tubular body 53 and the dispersion chamber 55 is formed with two systems in this quantitative feeder 51: the penetrating aperture Eta of the elastic membrane Et and the bypass pipe Tv. Therefore, the air can pass between the tubular body 53 and the dispersion chamber 55 via an available system.

When the air flows from the dispersion chamber 55 to the tubular body 53 via the penetrating aperture Eta of the elastic membrane Et as shown in Fig.22a, the air flow from the tubular body 53 to the dispersion chamber 55 is generated in the bypass pipe Tv. Accordingly the air can smoothly flow from the dispersion chamber 55 to the tubular body 53 via the aperture Eta of the elastic membrane Et.

Then as the positive pulsating vibration air supplied in the dispersion chamber 55 moves to its valley, the elastic membrane Et returns to its original position from an upwardly curved position by its resilience. At the same time the penetrating aperture Eta returns to its original shape from the V shape and the lubricant powders dropped in the opened aperture Eta are kept therein (see Fig.22b).

As the air communication passage between the tubular body 53 and the dispersion chamber 55 of the quantitative feeder 51 is comprised of two lines: the penetrating aperture Eta of the elastic membrane Et and the bypass pipe Tv, the air can flow therebetween via an available line.

In other words, in case of the condition as shown in Fig.22b, even if the penetrating hole Eta is closed, the air can flow from the tubular body 53 to the dispersion chamber 55 via the bypass pipe Tv, therefore, the pressures in the chamber 55 and in the tubular body 53 are quickly balanced.

Then when the positive pulsating vibration air supplied in the dispersion chamber 55 becomes its amplitude valley and the pressure in the dispersion chamber 55 is reduced, the elastic membrane Et is elastically deformed with its center curved downwardly. The penetrating aperture Eta becomes reverse v-shaped with its lower end opened in its section. Then the powders kept in the aperture Eta fall in the dispersion chamber 55 (see Fig.22c).

As the air communication passage between the tubular body 53 and the dispersion chamber 55 of the quantitative feeder 51 is comprised of two lines: the penetrating aperture Eta of the elastic membrane Et and the bypass pipe Tv, therefore the air can flow therebetween via an available line.

In other words, the elastic membrane Et is curved downwardly and the volume of the tubular body 53 becomes larger, the air flows from the dispersion chamber 55 to the tubular body 53 via the bypass pipe Tv. Therefore, the air flow from the dispersion chamber 55 to the tubular body 53 via the penetrating aperture Eta isn't caused.

Accordingly, the lubricant powders can be smoothly discharged through the aperture Eta stably and quantitatively.

As the result of providing the bypass pipe Tv between the dispersion chamber 55 and the tubular body 53, the time required for balancing the pressure in the tubular body 53 and the pressure in the dispersion chamber 55 when the positive pulsating vibration air is supplied to the dispersion chamber 55 of the quantitative feeder 51 becomes short so that the responsibility of the vertical vibration of the elastic membrane Et to the vibration of the positive pulsating vibration air becomes superior. As a result, the lubricant powders can be smoothly discharged via the penetrating aperture Eta.

According to the quantitative feeder 51, the up and down vibrations wherein the center of the elastic membrane Et is operated as its antinode of the vibration and the periphery is operated as its node depend on the frequency, amplitude and wave shape of the positive pulsating vibration air supplied to the dispersion chamber 55.

Therefore, as far as the positive pulsating vibration air supplied to the dispersion chamber 55 is constant, a fixed amount of lubricant powder is always accurately discharged to the dispersion chamber 55 via the penetrating aperture Eta of the elastic membrane Et. According to this quantitative feeder 51, the lubricant powders can be stably supplied to the lubricant spray chamber (lubricant apply means) 91 at a fixed concentration.

The quantitative feeder 51 also has an advantage that if the frequency, amplitude and wave shape of the positive pulsating vibration air supplied to the dispersion chamber 55 are controlled, the amount of powder supplied to a desired place (instrument) can be easily changed.

Furthermore according to the quantitative feeder 51, the positive pulsating vibration air becomes a swirl directing upward in the dispersion chamber 55. Even if the aggregated particles with a large diameter are contained in the lubricant powders discharged to the dispersion chamber 55, most of all can be pulverized and dispersed to be small particles by being caught in the positive pulsating vibration air swirling in the dispersion chamber 55.

In addition, the positive pulsating vibration air in the dispersion chamber 55 becomes an upward swirling flow so that the dispersion chamber 55 has a size classification function like a cyclone.

Therefore, the lubricant powders with a predetermined particle size can be discharged to the conduit T2 from the discharge port 55b.

Namely, the aggregated particles with a large diameter keep swirling in the lower part of the dispersion chamber 55 and are pulverized into a predetermined particle size by being caught in the positive pulsating vibration air swirling in the chamber 55. Thereby, the aggregated material is controlled to be a predetermined particle size while being dispersed and is discharged to the conduit T2 from the discharge port 55b so that large sized lubricant powders aren't sprayed in the lubricant spray chamber (lubricant apply means) 91.

The lubricant powders supplied to the conduit T2 connected to the discharge port 55b are pneumatically transported to the other end e2 of the conduit T2 by means of the positive pulsating vibration air.

Thereby, according to the quantitative feeder 51, a deposit phenomenon and a pinhole phenomenon aren't caused in the conduit, which have been seen in transportation means wherein the powdered material supplied to the conduit is pneumatically transported by a steady pressure air with constant flow.

Therefore, according to the quantitative feeder 51, the lubricant powders can be discharged from the other end e2 of the conduit T2 while keeping the concentration of the original powders discharged in the conduit T2 from the discharge port 55b of the dispersion chamber 55, thereby enabling an accurate control of the quantitativeness of the lubricant powders sprayed from the other end e2 of the conduit T2.

Furthermore, according to the quantitative feeder 51, substantially a fixed amount of lubricant powders is placed on the elastic membrane Et (at the height Hth where the level sensor 62 is provided above the membrane Et) while operating the quantitative feeder 51. The amount of lubricant powders discharged from the penetrating aperture Eta of the elastic membrane Et doesn't vary depending on the change in the amount of lubricant powders placed on the elastic membrane Et. Accordingly, a fixed amount of lubricant powders can be stabley supplied to the lubricant spray chamber (lubricant apply means) 91.

Still further according to the quantitative feeder 51, even if the large size powders are discharged to the dispersion chamber 55, such powders are pulverized into a predetermined particle size by being caught in the positive pulsating vibration air swirling in the chamber 55 to be discharged to the conduit T2 from the discharge port 55b, so that the large size powders aren't deposited in the dispersion chamber 55.

Therefore, if the quantitative feeder 51 is operated for a long time, the lubricant powders don't deposit in the dispersion chamber 55 so that the number of cleaning in the dispersion chamber 55 can be reduced.

When such a quantitative feeder 51 is attached to the external lubrication type tabletting machine S, the cleaning in the dispersion chamber 55 isn't almost required while executing a continuous tabletting. Therefore, there is an effect that an externally lubricated tablet (tablet without including lubricant powders) can be effectively produced using such a tabletting machine S.

In addition, according to this quantitative feeder 51, the elastic membrane Et is stretched by means of the elastic membrane installation means 61 as shown in Fig.15, Fig.16 and Fig.17. The quantitativeness of the feeder 51 isn't damaged because of a loosed elastic membrane Et.

While a positive pulsating vibration air is supplied in the dispersion chamber 55 of the quantitative feeder 51, the lubricants (powder) are continuously discharged in the dispersion chamber 55 via the penetrating aperture Eta of the elastic membrane Et as mentioned above.

Next, the construction of the rotary type tabletting machine 81 will be explained.

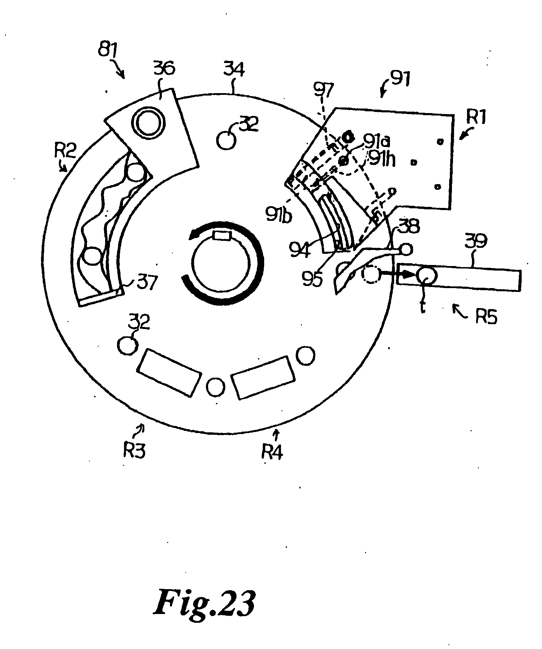

Fig.23 is a plan view diagrammatically showing the rotary type tabletting machine 81.

A normal rotary type tabletting machine is used as the rotary tabletting machine 81. The rotary type tabletting machine 81 has a turntable 34 rotatable for a rotary axis, plural upper punches (see the upper punches 31··· in Fig.10) and plural lower punches (see the lower punches 33··· shown in Fig.10).

Plural dies 32··· are provided for the turntable 34 and the upper punch 31··· and its corresponding lower punch 33··· are provided for the dies 32···. Those upper punches 31···, corresponding lower punch 33··· and corresponding die 32··· are synchronously rotated.

Further, the upper punches 31··· are constructed so as to move up and down in a rotary axis direction at a predetermined position by means of a cam mechanism (not shown). The lower punches 31··· are also constructed so as to move up and down in a rotary axis direction at a predetermined position by means of a cam mechanism (see the cam mechanism 35 in Fig.10).

The member shown as a reference numeral 36 in Fig.10 and Fig.23 indicates a feed shoe for charging a molding material in each die 32···, 37 shows a scraping plate for making the molding material charged in the dies 32··· at a fixed amount, 38 shows a scraper for discharging the produced effevescent tablet t into a discharge chute 39.

The position shown as R1 in Fig.23 is a lubricant spray position. According to this external lubrication type tabletting machine S, the lubricant spray chamber (lubricant apply means) 91 is provided at the lubricant spray point R1. More specifically, the lubricant spray chamber (lubricant apply means) 91 is fixedly provided on the turntable 34 in such a manner that the lubricants are applied on each surface of the dies 32···, the upper punches 31··· and the lower punches 33··· which are sequentially accommodated in the lubricant spray chamber (lubricant apply means) 91 when the turntable 34, the plural upper punches 31··· and the plural lower punches 33··· are rotated. The method of applying lubricants on each surface of the dies 32···, the upper punches 31··· and the lower punches 33··· in the lubricant spray chamber (lubricant apply means) 91 will be detailed later.

The position shown as R2 in Fig.23 is a molding material charge position where the molding material m is charged in the cavity made by the die 32 and the lower punch 33 inserted to a predetermined position in the die 32 by the feed shoe 36.

A position R3 in Fig.23 is a pre-tabletting point where a fixed amount of molding material which is filled in the cavity formed by the die 32 and the lower punch 33 and is scraped by the scraping plate 37 is preliminary tabletted by means of the upper punch 31 and the corresponding lower punch 33.

A position R4 in Fig.23 is a main tabletting point where the pre-tabletted molding material is fully compressed by the upper punch 31 and the corresponding lower punch 33 so as to produce an effevescent tablet t.

A position R5 in Fig.23 is a tablet discharge point where the effevescent tablet t discharged outside when the upper face of the lower punch 33 is inserted into the upper end of the die 32 is discharged to the discharge chute 39 by means of the tablet discharge scraper 38.

Next, the construction of the lubricant spray chamber (lubricant apply means) 91 will be detailed.

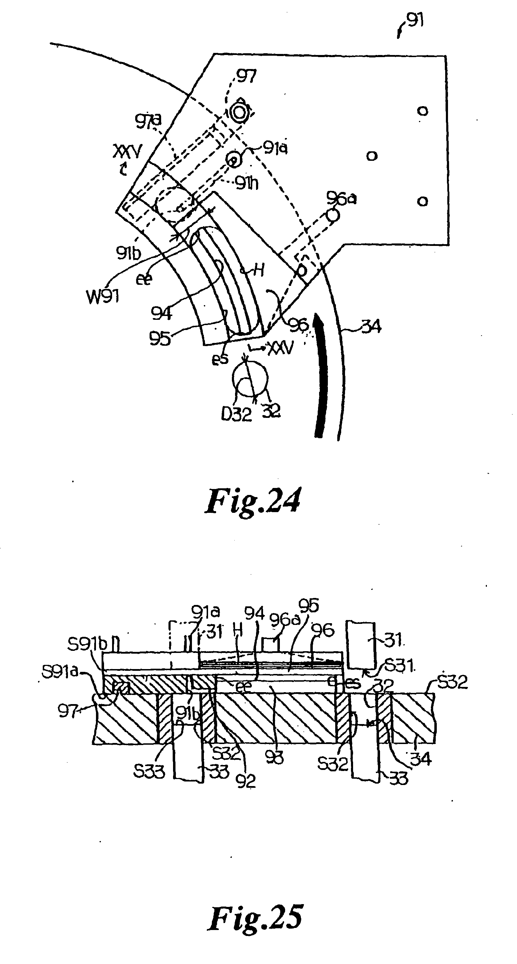

Fig.24 is an enlarged plan view of the lubricant spray chamber (lubricant apply means) 91 shown in Fig.23. Fig.25 shows a diagrammatical section of the lubricant spray chamber (lubricant apply means) 91 along the line XXV - XXV in Fig.24.

The lubricant spray chamber (lubricant apply means) 91 is fixedly provided at a predetermined position on the turntable 34 of the rotary type tabletting machine 81.

A surface (bottom) S91a of the lubricant spray chamber (lubricant apply means) 91 facing the turntable 34 is designed to get in touch with a surface S34 of the turntable 34 and the turntable 34 rubs on the bottom S91a.

The lubricant spray chamber (lubricant apply means) 91 has a lubricant introduction port 91a connecting the conduit T2 to its outer surface S91.

The lubricant powders which have been supplied from the lubricant introduction port 91a and dispersed in a positive pulsating vibration air is fed to the surface (bottom) facing the turntable 34 of the lubricant spray chamber (lubricant apply means) 91 via a penetrating hole (lubricant powder spray port for lower punch) 91h which penetrates the lubricant spray chamber (lubricant apply means) 91. Then the lubricant powders are sprayed on the surface (upper face, material contacting surface) S33 of the lower punch 33 inserted in a predetermined portion in the die 34 of the turntable 34 from the discharge port 91b of the penetrating hole (lubricant powder spray port for lower punch) 91h.

Further in this embodiment, the lubricant powders dispersed in air is designed to be perpendicularly sprayed on the surface (upper face, material contacting surface) S33 of the lower punch 33 from the penetrating hole (lubricant powder spray port for lower punch) 91h of the discharge port 91b.

A groove 92 is provided for the surface (bottom) S91a facing the turntable 34 of the lubricant spray chamber (lubricant apply means) 91 from the discharge port 91b of the penetrating hole (lubricant powder spray port for lower punch) 91h into the reverse direction of the rotation of the turntable 34.

The extra lubricant powders accumulated on the surface (upper face, material contacting surface) S33 of the lower punch 33 are blown off by the air supplied together with the lubricant powders. A part of blown-out powders is designed to be applied on the surface S32 (inner circumference, more specifically the material contacting surface above the upper face (material contacting surface) of the lower punch inserted into a fixed position in the die) of the die 32.

Further, the lubricant powders pass through a tubular portion formed by the groove 92 provided on the surface (bottom) of the lubricant spray chamber (lubricant apply means) 91 facing the turntable 34 and by the surface of the turntable 34 and are fed in reverse direction of the rotating direction of the turntable 34.

The end of the groove 92 provided on the surface (bottom) facing the turntable 34 of the lubricant spray chamber (lubricant apply means) 91 is communicated with a hollow chamber 93 provided at the surface (bottom) side of the lubricant spray chamber (lubricant apply means) 91 facing the turntable 34.

A slit 94 (slit-like lubricant powder spray port for upper punch) is formed above the hollow chamber 93 so as to penetrate the lubricant spray chamber (lubricant apply means) 91.

At the outer surface of the lubricant spray chamber (lubricant apply means) 91, an upper punch accommodation part 95 for sequentially accommodating the upper punches 31··· which rotates in synchronism with the turntable 34 along the slit 94 (slit-like lubricant powder spray port for upper punch) is formed along the rotary orbit of the upper punches 31···.

The width W95 of the upper punch accommodation part 95 is equal to or a little larger than the diameter of the upper punch 31.

A suction head 96 is provided above the slit 94 (slit-like lubricant powder spray port for upper punch).

The numeral 91a in Fig.25 is a connection port to be connected with the conduit T2.

The size of a suction port H of the suction head 96 is designed so as to cover the entire slit 94 (slit-like lubricant powder spray port for upper punch) and so as to be a similar shape to the slit 94 (slit-like lubricant powder spray port for upper punch).

As a result, when a suction means (the suction means 102 in Fig.10) is driven, an upward air flow is uniformly and evenly generated from one end eS to the other end ee of the slit 94 (slit-like lubricant powder spray port for upper punch).

Therefore, lubricant powders can be applied taking enough time on the surface (lower face, material contacting surface) S31 of the upper punch 31 on which lubricant powders have difficulty (because of gravity) to be applied while the upper punch 31 moves from the end eS to the other end ee of the slit 94 (slit-like lubricant powder spray port for upper punch) in the upper punch accommodation part 95.

Further in this embodiment, at the downstream of the lubricant spray point of the lubricant spray chamber (lubricant apply means) 91 (at the upstream of the material charge point), a lubricant suction part 97 is provided for removing the lubricant powders L flown out on the turntable 34 or the lubricant powders L additionally attached on the surface (upper face, material contacting surface) S33 of the lower punch 33 and on the circumferential wall S43 of the die.

A suction means such as a blower (not shown) is connected to the lubricant suction part 97. When the suction means (not shown) is driven, the lubricant powders flown out on the turntable 34 or the lubricant powders additionally attached on the surface (upper face, material contacting surface) S33 of the lower punch 33, on the surface (inner circumference, more specifically the material contacting surface above the upper face (material contacting surface) of the lower punch inserted into a fixed position in the die) S32 of the die 32 and on the surface (upper face, material contacting surface) S33 of the lower punch 33 can be suck and removed from the suction port 97a.

The suction port 97a is formed like a slit (long shape) on the surface (bottom) facing the turntable 34 in such a manner that the longitudinal direction becomes a substantially central direction from the periphery of the turntable 34 and the suction port 97a bridges the die 32.

The distance between the suction port 97a and the discharge port 91b is set to be a litter larger than the diameter D32 of the die 32.

Therefore, when the suction means such as a blower (not shown) connected to the lubricant suction part 97 is driven, the turntable 34 around the dies 32 can be always kept clean. As a result, the lubricant powders attached around the die 32 of the turntable 34 don't fall in the die 32 so that externally lubricated tablet which doesn't include any lubricant L in the tablet can be continuously tabletted.

Next, the construction of the lubricant suction means 101 will be detailed.

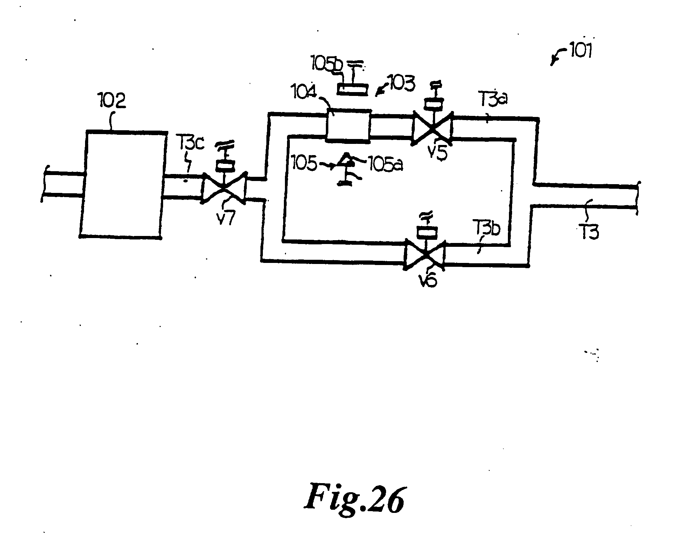

Fig.26 is a constructional view diagrammatically enlarging around the lubricant suction means 101 shown in Fig.10.

The lubricant suction means 101 has a suction means 102 such as a blower and a conduit T3 connected to a suction means 102.

The conduit T3 is connected to the suction head 96 of the lubricant spray chamber (lubricant apply means) 91.

Further, the conduit T3 is branched into two branch pipes T3a and T3b, integrated into one pipe T3c again and connected to the suction means 102.

A switch valve v5 and a light permeable type powder concentration measuring means 103 are sequentially provided from the lubricant spray chamber (lubricant apply means) 91 into a direction of the suction means 102.

The light permeable type powder concentration measuring means 103 has a measurement cell 104 and a light permeable type measuring means 105.

The measurement cell 104 is made of quartz and connected in midstream of the branch pipe T7a.

The light scattering type measuring means 105 is provided with a laser beam emitting system 105a for emitting laser beams and a scattering beam receiving system 105b for receiving the light scattered by an object and is designed to measure the flow rate, particle diameter, particle size distribution and concentration of the object according to the Mie theory. In this embodiment, the laser beam emitting system 105a and the scattering beam receiving system 105b are opposed so as to interpose the measurement cell 104 in such a manner that the flow rate, particle diameter, particle size distribution and concentration of the powdered material (lubricants (powder) in this embodiment) running in the branch pipe T3a can be measured in the measurement cell 104.

A switch valve v6 is provided for the branch pipe T3b.

Further, a switch valve v7 is provided for the conduit T7c.

For controlling the concentration of the lubricants (powder) in the lubricant spray chamber (lubricant apply means) 91 by means of the lubricant suction means 102, the switch valves v5 and v7 are opened while the switch valve v6 is closed, and then the suction means 102 is driven.

When the pulsating vibration air generation means 41 and the quantitative feeder 51 are driven, respectively, the lubricants (powder) mixed with and dispersed by a positive pulsating vibration air are supplied in the lubricant spray chamber (lubricant apply means) 91 together with the positive pulsating vibration air.

Then a part of the lubricants (powder) fed in the lubricant spray chamber (lubricant apply means) 91 is used for spraying on each surface (lower face, material contacting surface) S31 of the upper punches 31 ···, each surface S33 (upper face, material contacting surface) of the lower punch 33 ···, and each inner circumference S32 of the dies 32 ···. The extra lubricants are sucked to the suction means 102 from the suction head via the conduit T3, the branch pipe T3a and the conduit T3c.

This time the light permeable type measuring means 105 consisting of the light permeable type powder concentration measuring means 103 is driven to measure the flow rate, particle diameter, particle size distribution, and concentration of the lubricants (powder) running in the measurement cell 104, namely in the branch pipe T3a.

The concentration of the lubricants (powder) in the lubricant spray chamber (lubricant apply means) 91 is controlled by appropriately adjusting the drive amount of suction means 102 and the drive amount of pulsating vibration air generation means 71 depending on the measured value of the light permeable type measuring means 105.

Under such operations, a problem is caused such that the lubricants (powder) are adhered in the inner circumference of the measurement cell 104 and the permeable type measuring means 105 can't accurately measure the flow rate and so on of the lubricants (powder) running in the branch pipe T3a because of thus adhered lubricants (powder) in the measurement cell 104. In such a case a compensation is required for removing the affection (noise) caused by the lubricants (powder) adhered in the measurement cell 104 from the measured value of the measuring means 105. However, according to this suction means 102, the switch valve v5 is closed and the switch valve v6 is opened while keeping the suction means 102 driven for measuring the affection (noise) by the lubricants (powder) attached in the measurement cell 104. The lubricants (powder) sucked in the conduit T3 from the suction head is further sucked in the suction means 102 through the branch pipe T3b and the conduit T3c so that the lubricants (powder) doesn't run in the branch pipe T3a.

When the light permeable type measuring means 105 is driven at this time, the affection (noise) by the lubricants (powder) adhered in the measurement cell 104 can be measured.

The measured value of the affection (noise) by the lubricants (powder) adhered in the cell 104 is temporarily stored in a memory means of the processing unit 121.

Thereafter, the switch valve v5 is opened and the switch valve v6 is closed while keeping the suction means 102 driven so as to run the lubricants (powder) through the branch pipe T3a. Then the powder concentration measuring means 103 is driven to measure the flow rate and so on of the lubricants (powder) running in the branch pipe T3a. The compensation value obtained by removing the affection (noise) of the lubricants (powder) adhered in the cell 104 from the measured value of the light permeable type measurement means 105 based on the compensation program and the measured value of the affection (noise) of the lubricants (powder) adhered in the cell 104 stored in the memory means of the processing unit 121 in advance. Then the concentration of the lubricants (powder) in the lubricant spray chamber (lubricant apply means) 91 is controlled by adjusting the driving amount of suction means 102 and that of pulsating vibration air generation means 21 based on the compensation value.

Next, a production method of an effevescent tablet by means of the external lubricating tabletting machine S according to the present invention will be diagrammatically explained.

At first, predetermined operation conditions are input in the processing unit 121.

Lubricant powders are contained in the lubricant storage hopper 52.

A mixture (molding material) which is a raw material of an effevescent tablet is stored in the feed shoe 36 of the rotary type tabletting machine 81.

Then the rotary type tabletting machine 81 and the suction means 102 are driven.

Further a suction means (not shown) connected to the lubricant suction part 97 is driven if necessary.

Then an air source 111 is driven under the operational conditions input in the processing unit 121.

At the same time, the rotary cam 45 of the pulsating vibration air generation means 41 is driven at a fixed rotational speed, thereby supplying a positive pulsating vibration air with a fixed flow amount, pressure, frequency and wave shape in the conduit T1.

Further, the level sensor 71 is actuated.

Actuating the level sensor 71, the gas injection means 56 and 56 and the material feed valve 54 are operated like Fig.20 and Fig.21, thus a fixed amount of lubricant powders is stored on the elastic membrane Et.

The positive pulsating vibration air generated from the pulsating vibration air generation means 41 is supplied in the dispersion chamber 55. Therefore, the elastic membrane Et is vibrated up and down to discharge the lubricant powders into the dispersion chamber 55 through the penetrating aperture Eta provided on the elastic membrane Et.

Thus discharged lubricant powders in the dispersion chamber 55 are mixed with and dispersed in the positive pulsating vibration air swirling in the dispersion chamber 55 and are discharged to the conduit T2 from the discharge port 55b.

The lubricant powders mixed with and dispersed in the positive pulsating vibration air which have been discharged in the conduit T2 are pneumatically transported in the conduit T2 into the lubricant spray chamber 91 by means of the positive pulsating vibration air.

The lubricant powders fed in the lubricant spray chamber (lubricant apply means) 91 pass through the penetrating aperture (lubricant powder spray port for lower punch) 91h from the lubricant introduction port 91a together with the positive pulsating vibration air and sprayed on the surface (upper face, material contacting surface) S33 of the lower punch 33 inserted in a predetermined position in the die 32 which has come to the lubricant spray point R1 by the rotation of the turntable 34 from the discharge port 91b.

The extra lubricant powders accumulated on the surface (upper face, material contacting surface) S33 of the lower punch 33 are blown off by the air fed together with the lubricant powders L and a part of blown powders is applied on the surface S32 (inner circumference, more specifically the material contacting surface above the upper face (material contacting surface) of the lower punch inserted into a fixed position in the die) of the die 32.

Further the lubricant powders are fed through the tubular part formed by the groove 92 provided on the surface (bottom) of the lubricant spray chamber (lubricant apply means) 91 facing the turntable 34 and by the surface (bottom) of the turntable 34 into a reverse direction of rotation of the turntable 34 and are supplied in the hollow chamber 93.

The lubricant powders fed in the hollow chamber 93 ride on an upward flow uniformly generated above the slit 94 (slit-like lubricant powder spray port for upper punch) and moves in the suction port H of the suction head 96 when the suction means 102 is driven.

Lubricant powders are applied on the lower face S31 (material contacting surface) of the upper punch 31 passing in the upper punch accommodation part 95 while the upper punch 31 moves from the one end eS to the other end ee of the slit (slit-like lubricant powder spray port for upper punch).

Extra lubricant powders are removed from the suction head 96.

Next when the die 32 fed in the downstream of the lubricant spray point R1 by the rotation of the turntable 34 and the lower punch 33 fed in the downstream of the lubricant spray point in synchronism with the rotation of the turntable 34 pass under the suction port 97a of the lubricant suction part 97, the extra lubricant powders attached around the die 32 on the turntable 34 and the extra lubricant powders attached on the surface S32 (inner circumference, more specifically the material contacting surface above the upper face (material contacting surface) of the lower punch inserted into a fixed position in the die) of the die 32 and the surface S33 (upper face, material contacting surface) of the lower punch 33 are removed.

At the molding material charge point R2, a mixture (molding material) is charged in the die 32 in which lubricant powders are uniformly applied on the surface S32 (inner circumference, more specifically the material contacting surface above the upper face (material contacting surface) of the lower punch inserted into a fixed position in the die) and the lower punch 33 on which surface S33 lubricant powders are uniformly applied is inserted into a fixed position.

After extra mixture is removed by a scraping plate 37, a mixture (molding material) is preliminary tabletted at the pre-tabletting point R3 by means of the upper punch 31 of which surface S31 (lower face, material contacting surface) lubricant powders are uniformly applied, the lower punch 33 of which surface S33 (upper face, material contacting surface) lubricant powders are uniformly applied, and the die 32 on which surface S32 (inner circumference) lubricant powders are uniformly applied. Then the mixture is compressed at a main tabletting point R4 to be produced as an effevescent tablet t to be sequentially discharged into the discharge chute 39 at the tablet discharge point R5.

A part or all of the lubricant uniformly applied on the surfaces of the punches 31 and 33 and the surface of the die 32 is transferred on the surface of the effevescent tablet t.

Operators observe the effevescent tablets t··· discharged in the discharge chute 39.

If the effevescent tablets t··· causing tabletting problems such as sticking, capping and laminating are included, the concentration of the lubricants (powder) in the lubricant spray chamber (lubricant apply means) 91 is increased by appropriately controlling the drive amount of the air source 111 and the driving amount of the suction means 102 in order to reduce the frequency of the tabletting problems such as sticking, capping and laminating on the produced effevescent tablets t···. Further, the elastic membrane Et may be replaced with a one with a larger penetrating aperture Eta.

Because the external lubrication type tabletting machine S has the above-mentioned superior effects, externally lubricated tablets which have been difficult to produce in a good industrial productivity in prior arts can be stably produced in large scale under a high industrial productivity.

Even if tabletting problems such as sticking, capping and laminating aren't caused, the composition of the effevescent tablets t··· is analyzed. If the amount of lubricant in the tablet composition is formed to be increased comparing with a scheduled amount, the driving amounts of air source 111 and suction means 102 are appropriately controlled so as to control the concentration of the lubricants (powder) in the lubricant spray chamber (lubricant apply means) 91 in a steady condition. When the amount of lubricants (powder) applied on each surface of the upper punches 31···, each surface of the lower punches 33···, each surface of the dies 32··· is controlled to be a fixed amount, the amount of lubricants (powder) transferred on each surface of the effevescent tablet t··· from each surface of the upper punches 31···, each surface of the lower punches 33···, each surface of the dies 32··· is reduced. Further, the elastic membrane Et may be replaced with the one having a smaller penetrating aperture Eta.

Next, the construction of the pulsating vibration air generation means 41 will be explained in detail.

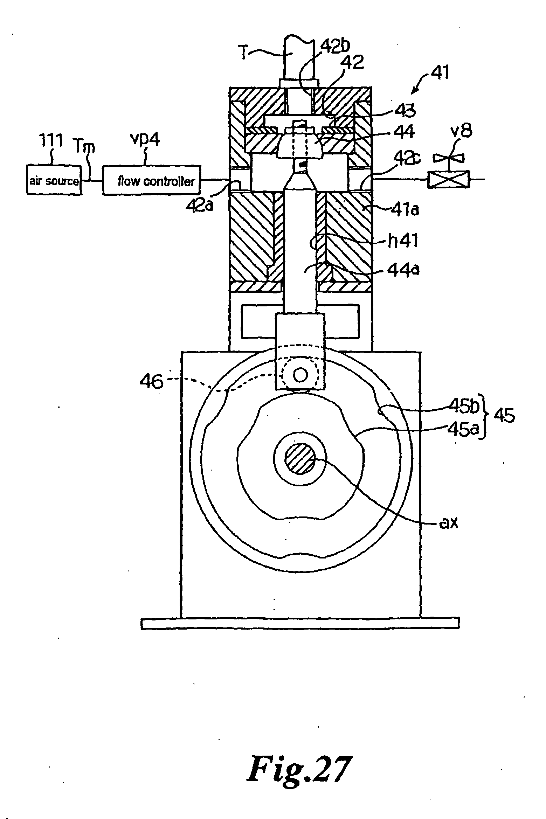

Fig.27 is a diagrammatic sectional view showing the construction of the pulsating vibration air generation means 41.

The pulsating vibration air generation means 41 has a hollow chamber 42 with an air supply port 42a and an air discharge port 42b, a valve seat 43 provided in the chamber 42, a valve plug 44 for opening and closing the valve seat 43, and a rotary cam 45 for opening and closing the valve plug 44 for the valve seat 43.

A conduit Tm is connected to the air supply port 42a and a conduit T1 is connected to the air discharge port 42b.

The member 42c in Fig.27 is a pressure control port provided for the hollow chamber 42 if required and a pressure control valve v8 is provided for the pressure control port 42c so as to communicate with and block off the atmosphere.

The valve plug 44 has a shaft 44a, under which a rotary roller 46 is rotatably connected.

A shaft hole h41 for containing the shaft 44a of the valve plug 44 airtightly and movably up and down is provided for a main body 41a of the pulsating vibration air generation means 41.

The rotary cam 45 has an inside rotary cam 45a and an outside rotary cam 45b.

A predetermined concavo-convex pattern is formed on each one of the inside rotary cam 45a and the outside rotary cam 45b so as to have a space about the distance of the diameter of the rotary roller 46.

The rotary cam 45 which has a concavo-convex pattern suitable for mixing and dispersing a lubricants (powder) depending on their physical property is used.

The rotary roller 46 is rotatably inserted between the inside rotary cam 45a and the outside rotary cam 45b of the rotary cam 45.

A member shown as ax in Fig.27 is a rotary axis of the rotary drive means such as a motor (rotary drive means 41M in Fig.10) and the rotary cam 45 is detachably provided for the rotary axis ax.

Next, a method for supplying a positive pulsating vibration air to the conduit T2 by means of the pulsating vibration air generation means 41 is explained.

At first, the rotary cam 45 with a concavo-convex pattern suitable for mixing and dispersing lubricants (powder) depending on their physical property is attached on the rotary axis ax of the rotary drive means 47.

Then the air source 111 is driven to supply a compressed air to the conduit Tm.

When the flow rate control valve vp4 is provided, the compressed air is supplied to the hollow chamber 42 from the air supply port 42a after being adjusted to a predetermined flow amount by the flow rate control valve vp4.

The air source 111 and the rotary drive means 47 are driven, so that the rotary cam 45 attached to the rotary axis ax of the rotary drive means 47 is rotated at a fixed rotational speed.

Accordingly, the rotary roller 46 is rotated between the inside rotary cam 45a and the outside rotary cam 45b of the rotary cam 45 which are rotated at a predetermined rotational speed in such a manner that the rotary roller 46 reproducibly moves up and down according to the concavo-convex pattern of the rotary cam 45. As a result, the valve plug 44 opens and closes the valve seat 43 according to the concavo-convex pattern formed on the rotary cam 45.

If a pressure-control port 42c and the pressure- regulating valve v8 are provided for the hollow chamber 42, the pressure of the positive pulsating vibration air supplied to the conduit T1 is regulated by appropriately controlling the pressure-regulating valve v8 provided for the pressure control port 42c.

Thus a positive pulsating vibration air is thus fed to the conduit T1.

The wavelength of the positive pulsating vibration air fed in the conduit T2 is properly controlled depending on the concavo-convex pattern of the rotary cam 45 and/or the rotational speed of the rotary cam 45. The wave shape of the positive pulsating vibration is also adjusted by the concavo-convex pattern of the rotary cam 45. The amplitude of the positive pulsating vibration air is controlled by adjusting the drive amount of the air source 111, by adjusting the pressure-regulating valve vp4 if they are provided or by adjusting the pressure-regulating valve v8 of the pressure regulating port 42c if it is provided, or by combining and adjusting them.

The pulsating vibration air generation means used for the external lubrication type tabletting machine S isn't limited to the pulsating vibration air generation means 41 and other pulsating vibration air generation means can be used.

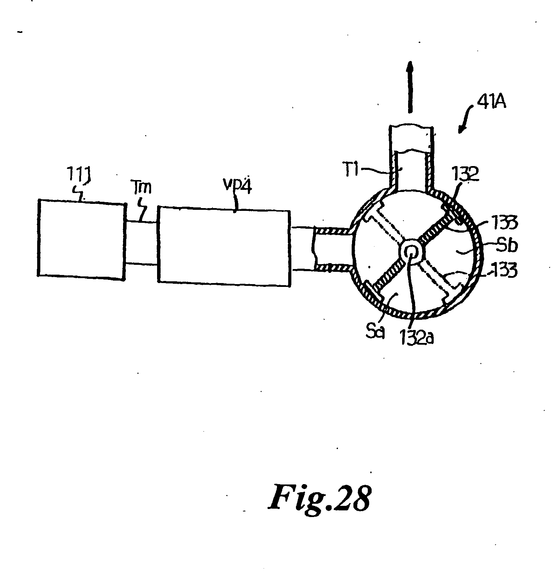

Fig.28 is a diagrammatic sectional view showing other embodiment of a pulsating vibration air generation means.

The pulsating vibration air generation means 41A has the same construction as the pulsating vibration air generation means 41 other than the following constructions. Corresponding members have the same reference numerals and their explanations are omitted here.

The pulsating vibration air generation means 41A has a cylindrical body 132 and a rotary valve 133 attached to a rotary axis 132a consisting a center axis of the cylindrical body 132 so as to divide a hollow chamber 133 into substantially two parts. The rotary axis 132a is designed to be rotated at a fixed rotational speed by a rotary drive means such as a motor (not shown).

Conduits Tm and T1 are connected to the external circumferential wall of the cylindrical body 132 with a fixed space.

A compressed air source 111 is driven to supply a fixed amount of compressed air to the conduit Tm for supplying a desired positive pulsating vibration air to the conduit T1 by means of the pulsating vibration air generation means 41A. If a flow rate control valve vp4 is provided, the flow rate of the compressed air to be fed in the conduit Tm is controlled by adjusting the flow rate control valve vp4.

The rotary axis 132a is rotated at a fixed rotational speed by the rotary driving means such as an electric motor (not shown) so that the rotary valve 133 attached to the axis 132a is rotated at a fixed speed.

Then the compressed air generated from the air source 111 is fed to the conduit T1 through the conduit Tm because the conduits Tm and T1 are communicated when the rotary valve 133 is at a position shown with solid lines in the figure.

When the rotary valve 133 is positioned as shown in imaginary lines, the conduits Tm and T1 are shut off by the rotary valve 133.

In such a case the compressed air is fed from the conduit Tm to one space Sa in the cylindrical body 132 divided by the rotary valve 133 and the air is compressed in the space Sa.

On the other hand, the compressed air stored in another space Sb in the cylindrical body 132 formed by the rotary valve 133 is fed to the conduit T1.

Repeating such operations by the rotation of the rotary valve 133, a positive pulsating vibration air is transmitted to the conduit T5b.

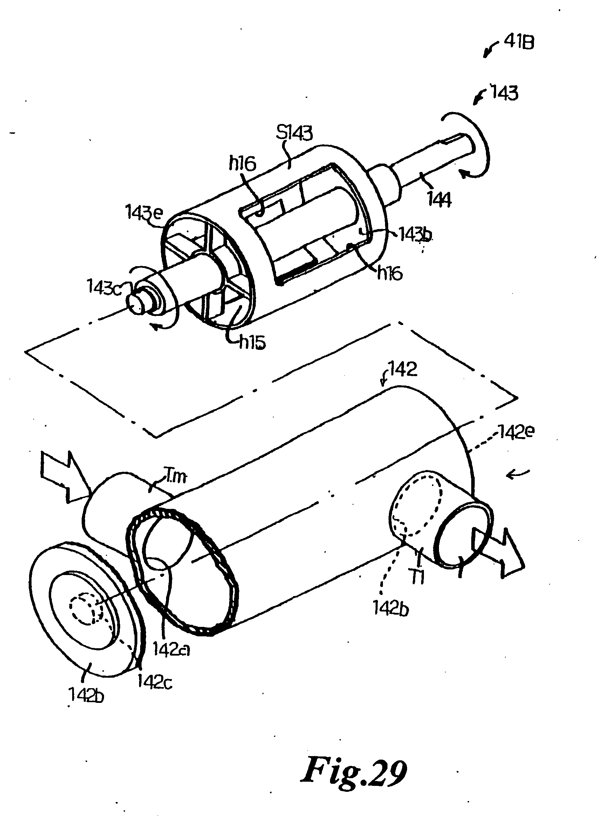

Fig.29 is an exploded perspective view diagrammatically showing other embodiment of a pulsating vibration air generation means.

The pulsating vibration air generation means 41B has a cylindrical body 142 and a rotary valve 143 rotatably provided in the body 142.

The cylindrical body 142 is constructed such that one end 142e is opened and the other end is closed by a cover 142b and a suction port 142a and a transmission port 142b are provided for its circumferential side wall.

A conduit Tm to be connected to the air source 111 is connected to the suction port 142a and a conduit T1 to be connected to the quantitative feeder 51 is connected to the transmission port 142b.

The member shown as 142c in Fig.29 is a bearing hole for pivotally providing the rotary valve 143.

The rotary valve 143 is cylindrical with a hollow part h15 and an opening h16 is provided on its circumferential wall S143. One end 143e of the rotary valve 143 is opened and the other end is closed by the cover 143b. A rotary axis 144 is extended in the rotary center of the rotary valve 143. Rotary drive means such as an electric motor (not shown) is connected to the rotary axis 144 and the rotary valve 143 is rotated around the rotary axis 144 when the rotary drive means (not shown) is driven.

The outer diameter of the circumferential wall S143 of the rotary valve 143 is almost the same as the inner diameter of the cylindrical body 142 in such a manner that the rotary valve 143 is contained in the cylindrical body 142 so that the circumferential wall S143 of the rotary valve 143 rubs against the inner circumference of the body 142 when the rotary valve 143 is rotated.

The member shown as 143c in Fig.29 is a rotary axis rotatably contained in the rotary bearing hole 142c provided for the cover 142b of the cylindrical body 142.

The rotary valve 143 is rotatably provided in the cylindrical body 142 such that the rotary axis 143c is attached to the rotary bearing hole 142c.

When a desired positive pulsating vibration air is supplied to the conduit T1 by means of the pulsating vibration air generation means 41B, a compressed air is supplied to the conduit Tm by driving the air source 111.

The rotary valve 143 is rotated at a fixed rotational speed by rotating the rotary axis 144 at a fixed rotational speed by the rotary drive means such as an electric motor (not shown).

When the opening h16 of the rotary valve 143 is positioned at the transmission port 142b, the conduits Tm and T1 are communicated so that a compressed air is fed to the conduit pipe T1.

When the circumferential wall S143 of the rotary valve 143 is positioned at the transmission port 142b, the conduits Tm and T1 are closed by the wall S143 so that a compressed air isn't fed to the conduit T1.

Repeating such operations by the rotation of the rotary valve 143, a positive pulsating vibration air is fed in the conduit T1.

Any one of the pulsating vibration air generation means 41 shown in Fig.27, the pulsating vibration air generation means 41A shown in Fig.28, and the pulsating vibration air generation means 41B shown in Fig.29 may be used as the pulsating vibration air generation means for the external lubrication type tabletting machine S. However, considering the decrescence property of a positive pulsating vibration air, it is preferable to produce a positive pulsating vibration air with clear on and off conditions from the pulsating vibration air generation means. In order to generate such a clear positive pulsating vibration air, it is preferable to use the rotary cam type pulsating vibration air conversion means 41 in Fig.27 rather than the rotary type pulsating vibration air conversion means 41A and 41B shown in Fig.28 and Fig.29.

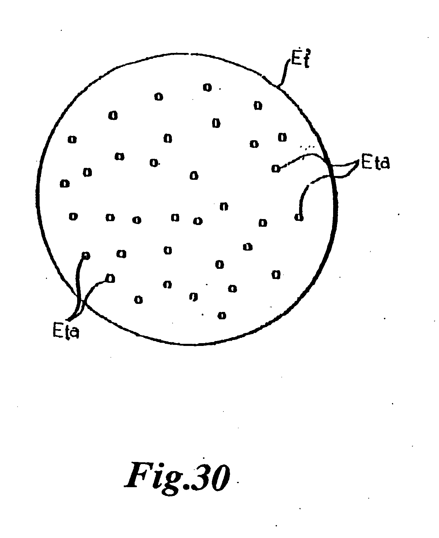

In the above-mentioned embodiment, an elastic membrane Et having one penetrating aperture Eta is explained, however, the elastic membrane isn't limited to the elastic membrane Et having one penetrating aperture Eta. An elastic membrane with plural penetrating apertures Eta··· as shown in Fig.30 may be used.

The above-mentioned external lubrication type tabletting machine and the method for applying lubricant on each surface of the punches 31 and 33 and the die 32 are only preferable embodiments for producing an effevescent tablet of the present invention. Other machines or apply methods may be used as far as a minimum amount of lubricant powder can be applied on each surface of the punches 31 and 33 and the die 32.

Now, the present invention will be explained based on specific experimental data. (Experiment example 1)

Experiment example 1 shows one embodiment when an effevescent tablet for oral administration is produced according to the present invention.

This experiment example 1 corresponds to the effevescent tablet 1 shown in Fig.2 and shows a production example of an effevescent tablet for oral administration wherein granules are produced using a water-soluble high polymer solution as a binder to be compressed.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powders (Japanese Pharmacopoeia) were granulated to produce ascorbic acid granules.

More specifically, ascorbic acid granules were granulated as follows.

A fixed amount of ascorbic acid powders (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution was sprayed from a spray means provided in the granulation tank, thereby producing ascorbic acid granules.

A solution (2w/w% HPC-SL solution) in which a water-soluble high polymers (specifically hydroxypropylcellulose (HPC-SL)) were dissolved in water was used as a binder 5.

Sodium hydrogen carbonate granules were granulated according to the following method.

A fixed amount of sodium hydrogen carbonate powders (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution was sprayed from a spray means provided in the granulation tank, thereby producing sodium hydrogen carbonate granules.

A solution (2w/w% HPC-SL solution) in which a water-soluble high polymers (specifically hydroxypropylcellulose (HPC-SL)) were dissolved in water was used as a binder 5.

For producing sodium hydrogen carbonate granules, the temperature and amount of the heated air supplied in the granulation tank of a fluid bed granulation dryer (Glatt Co., Ltd., WSG-type 5) and the spray amount per hour of the binder solution sprayed from the spray means were controlled in such a manner that the particle size of the sodium hydrogen carbonate granules became the same as that of the ascorbic acid granules.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution was sprayed from a spray means provided in the granulation tank, thereby producing citric acid granules.

A solution (2w/w% HPC-SL solution) in which a water-soluble high polymer (specifically hydroxypropylcellulose (HPC-SL)) were dissolved in water was used as a binder 5.

For producing citric acid granules, the temperature and amount of the heated air supplied in the granulation tank of a fluid bed granulation dryer (Glatt Co., Ltd., WSG-type 5) and the spray amount per hour of the binder solution sprayed from the spray means were controlled in such a manner that the particle size of the citric acid granules becomes the same as that of the ascorbic acid granules.

The particle size distribution of the ascorbic acid granules, the sodium hydrogen carbonate granules and the citric acid granules obtained by the above-mentioned procedure is shown in a table 1.

**Table 1**

| particle size | ascorbic acid granules | sodium hydrogen carbonate granules | citric acid granules |
|---|---|---|---|
| more than 710 µm | 0% | 0% | 0% |
| 500 ∼ 710 µm | 2% | 0% | 1% |
| 350 ∼ 500 µm | 20% | 18% | 22% |
| 250 ∼ 350 µm | 45% | 42% | 47% |
| 105 ∼ 250 µm | 21% | 23% | 23% |
| less than 105 µm | 12% | 17% | 7% |
| total | 100% | 100% | 100% |

The ascorbic acid content of thus obtained ascorbic acid granules was 99.01w/w%, the sodium hydrogen carbonate content of the sodium hydrogen carbonate granules was 99.01w/w% and the citric acid content of the citric acid granules was 99.01w/w%.

Thus prepared ascorbic acid granules, sodium hydrogen carbonate granules and citric acid granules were blended at a fixed rate (in this embodiment, the ascorbic acid, sodium carbonate and citric acid were blended in a weight ratio of 6:1:1).

Then the blended material of the ascorbic acid granules, the sodium carbonate granules and the citric acid granules was mixed with a well-known mixer.

As shown in the table 1, when ascorbic acid granules, sodium hydrogen carbonate granules and citric acid granules having almost the same particle size were used, it was found that each ascorbic acid granule, sodium carbonate granule and citric acid granule was uniformly mixed by themselves spontaneously by the external force given by the mixer.

Thus obtained mixture of the ascorbic acid granules, the sodium hydrogen carbonate granules and the citric acid granules was tabletted by means of the external lubrication type tabletting machine S shown in Fig.10.

An upper punch 31, a lower punch 33 and a die 32 with 11mm diameter were used for a rotary type tabletting machine 81.

An upper punch 31 and a lower punch 33 with flat molding surface were used.

The tabletting pressure was 1500kg/punch and the weight per a tablet was controlled to be 405mg±1mg.

Magnesium stearate (Japanese Pharmacopoeia) was contained in a lubricant storage hopper 52 of a quantitative feeder 51 of the external lubrication type tabletting machine S.

A rotary cam 45 having a concavo-convex pattern suitable for dispersing magnesium stearate (Japanese Pharmacopoeia) in airwasattached for a rotary axis of a rotary drive means (rotary drive means 41M in Fig.10) of a pulsating vibration air generation means 41 of the external lubrication type tabletting machine S.

Then, the mixture of the ascorbic acid granules, the sodium hydrogen carbonate granules and citric acid granules was stored in a molding material storage hopper (not shown) connected to a feed shoe 36.

Next, an air source 111 was driven at a fixed drive amount to rotate a rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range of 10Hz to 40Hz.

The supply amount of magnesium stearate (Japanese Pharmacopoeia) into a lubricant spray chamber (lubricant apply means) 91 was controlled by driving a light permeable type powder concentration measuring means 103.

The supply amount of magnesium stearate (Japanese Pharmacopoeia) into the lubricant spray chamber (lubricant apply means) 91 isn't completely defined. However, the amount ia selected from the range of 200mg/min. to 2000mg/min. Handling of the supply amount of magnesium stearate (Japanese Pharmacopoeia) into the lubricant spray chamber (lubricant apply means) 91 has been considered to be difficult when a steady flow air is used.

Then, the rotary type tabletting machine 81 was driven and at the same time the mixture was supplied from the feed shoe 36. The mixture was compressed to produce a tablet by means of the upper punches 31··· on which a fixed amount of magnesium stearate (Japanese Pharmacopoeia) was applied, the lower punches 33··· on which a fixed amount of magnesium stearate (Japanese Pharmacopoeia) was applied and the dies 32··· on which a fixed amount of magnesium stearate (Japanese Pharmacopoeia) was applied.

Analyzing thus obtained tablet, the drive amount of air source 111, the drive amount of pulsating vibration air generation means 41 were appropriately controlled in such a manner that the applied amount of magnesium stearate per a tablet became 0.4mg±0.1mg. Then the effevescent tablet 1 was produced in earnest.

The preparation of thus produced tablet (effevescent tablet 1) is shown in a table 2.

**Table 2**

| component | content |
|---|---|
| ascorbic acid | 300.0mg |
| sodium hydrogen carbonate | 50.0mg |
| citric acid | 50.0mg |
| hydroxypropylcellulose (HPC-SL) | 4.0mg |
| magnesium stearate | 0.4mg |
| Total | 404mg/tablet |

### (Comparison example 1)

A fixed amount of magnesium stearate (Japanese Pharmacopoeia) was added in the mixture of the ascorbic acid granules, the sodium hydrogen carbonate granules and the citric acid granules and thus blended material of the ascorbic acid granules, the sodium hydrogen carbonate granules, the citric acid granules and the magnesium stearate (Japanese Pharmacopoeia) was mixed with a well-known mixer.

The mixture including magnesium stearate (Japanese Pharmacopoeia) was compressed to produce a tablet without applying magnesium stearate (Japanese Pharmacopoeia) on each surface of the upper punches 31···, the lower punches 33··· and the dies 32··· by means of the rotary type tabletting machine 81.

1.0 weight % of magnesium stearate (Japanese Pharmacopoeia) was required per a tablet in order to prevent the mixture including magnesium stearate (Japanese Pharmacopoeia) from adhering on the upper punches 31···, the lower punches 33··· and the dies 32··· and to avoid tabletting problems such as sticking on the produced tablet.

Then a characteristic test of the tablet (effevescent tablet 1 of the present invention) obtained by the experiment example 1 and the tablet obtained by the comparison example 1 was executed.

### Characteristic Test 1

One of the tablet (effevescent tablet 1 of the present invention) obtained by the experiment example 1 was put in 100ml drinking water, after one minute, the substance floating on the surface of the drinking water and turbidity thereof were observed.

Similarly one of the tablet obtained by the comparison example 1 was put in 100ml drinking water, after one minute, the substance floating on the surface of the drinking water and turbidity thereof were observed.

In the drinking water in which the tablet (effevescent tablet 1 of the present invention) obtained by the experiment example 1 was dissolved, no floating substance was observed on the water surface. However, in the drinking water in which the tablet obtained by the comparison example 1 was dissolved, a floating substance was observed on the water surface.

Further the drinking water in which the tablet (effevescent tablet 1 of the present invention) obtained by the experiment example 1 was dissolved was clear, on the other hand the drinking water in which the tablet obtained by the comparison example 1 was dissolved was cloudy.

### Characteristic Test 2

The disintegration time of each tablet (effevescent tablet 1 of the present invention) obtained by the experiment example 1 and the tablet obtained by the comparison example 1 was observed according to the disintegration test described in the Japanese Pharmacopoeia (13th edition).

Six pieces of sample wherein one tablet (effevescent tablet 1 of the present invention) obtained by the experiment example 1 was put in a glass tube of a tester used for the disintegration test method described in the Japanese Pharmacopoeia (13th edition) were prepared. They were put in a fixed amount of test solution (water of 37±2°C) filled in a beaker, the tester was moved up and down and the time till the tablet in the glass tube was completely dissolved was measured.

Also, six pieces of sample wherein one tablet obtained by the comparison example 1 was put in a glass tube of a tester used for the disintegration test method described in the Japanese Pharmacopoeia (13th edition) were prepared. They were put in a fixed amount of test solution (water of 37±2°C) filled in a beaker, the tester was moved up and down and the time till the tablet in the glass tube was completely dissolved was measured.

The results are shown in a table 3.

**Table 3**

| | sample | disintegration time |
|---|---|---|
| experiment example 1 | sample 1 | 25 sec. |
| | sample 2 | 27 sec. |
| | sample 3 | 27 sec. |
| | sample 4 | 27 sec. |
| | sample 5 | 29 sec. |
| | sample 6 | 31 sec. |
| | average disintegration time | 28 sec. |
| comparison example 1 | sample 1 | 31 sec. |
| | sample 2 | 37 sec. |
| | sample 3 | 48 sec. |
| | sample 4 | 70 sec. |
| | sample 5 | 70 sec. |
| | sample 6 | 91 sec. |
| | average disintegration time | 58 sec. |

As seen from the result of the table 3, the tablet (effevescent tablet 1 of the present invention) obtained by the experiment example 1 has a higher disintegration speed comparing with that obtained by the comparison example 1.

### Characteristic Test 3

The elution test of ascorbic acid for each tablet (effevescent tablet 1 of the present invention) obtained by the experiment example 1 and the tablet obtained by the comparison example 1 was executed according to the elution test method described in the Japanese Pharmacopoeia (13th edition).

Five pieces of sample wherein one tablet (effevescent tablet 1 of the present invention) obtained by the experiment example 1 was put in a basket used for the rotary basket method tester used for the elution test described in the Japanese Pharmacopoeia (13th edition) were prepared. Five pieces of a fixed amount of test solution (water of 37 ± 0.5°C in this embodiment) were prepared in the tester (content:1000ml) of the rotary basket method tester used for the elution test method of the Japanese Pharmacopoeia (13th edition).

Then the basket containing one tablet (effevescent tablet 1 of the present invention) obtained by the experiment example 1 was contained in the tester (content:1000ml) containing a fixed amount of test solution (water of 37 ± 0.5°C in this embodiment). Then the tester was rotated at a predetermined revolution number and after 30 seconds, a fixed amount of elute was sampled from a position 10mm apart from the container wall of the tester and the eluted amount of ascorbic acid contained in the elute ([eluted amount of ascorbic acid contained in the elute / the contained amount of ascorbic acid in a tablet obtained by the experiment example 1 (effevescent tablet 1 of the present invention)] x 100%) was measured. Such test was executed for each one of five samples.

Further five pieces of sample wherein one tablet obtained by the comparison example 1 was put in a basket used for the rotary basket method tester used for the elution test described in the Japanese Pharmacopoeia (13th edition) were prepared. Five pieces of a fixed amount of test solution (water of 37 ± 0.5°C in this embodiment) were prepared in the tester (content:1000ml) of the rotary basket method tester used for the elution test method of the Japanese Pharmacopoeia (13th edition).

Then the basket containing one tablet obtained by the comparison example 1 was contained in the tester (content:1000ml) containing a fixed amount of test solution (water of 37±0.5°C in this embodiment). Then the tester was rotated at a predetermined revolution number and after 30 minutes, a fixed amount of elute was sampled from a position 10mm apart from the container wall of the tester and the eluted amount of ascorbic acid contained in the elute ([eluted amount of ascorbic acid contained in the elute / the contained amount of ascorbic acid in a tablet obtained by the experiment example 1 (effevescent tablet 1 of the present invention)] x 100%) was measured. Such test was executed for each one of five samples.

The result is shown in table 4.

**Table 4**

| | sample | elution rate |
|---|---|---|
| experiment example 1 | sample 1 | 91% |
| | sample 2 | 95% |
| | sample 3 | 95% |
| | sample 4 | 98% |
| | sample 5 | 100% |
| | average elution rate | 96% |
| comparison example 1 | sample 1 | 43% |
| | sample 2 | 58% |
| | sample 3 | 67% |
| | sample 4 | 88% |
| | sample 5 | 97% |
| | average elution rate | 71% |

As seen from the result of the table 4, it was found that the elution characteristic of the tablet (effevescent tablet 1 of the present invention) obtained by the experiment example 1 was superior to the tablet obtained by the comparison example 1.

### (Comparison example 2)

Comparison example 2 shows one embodiment when an effevescent tablet for oral administration is produced according to a conventional method.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powders (Japanese Pharmacopoeia) were granulated to produce ascorbic acid granules.

More specifically, ascorbic acid granules were granulated as follows.

A fixed amount of ascorbic acid powders (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution was sprayed from a spray means provided in the granulation tank, thereby producing ascorbic acid granules.

A solution (2w/w% HPC-SL solution) in which a water-soluble high polymer (specifically hydroxypropylcellulose (HPC-SL)) was dissolved in water was used as a binder 5.

Sodium hydrogen carbonate granules were granulated according to the following method.

A fixed amount of sodium hydrogen carbonate powders (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution was sprayed from a spray means provided in the granulation tank, thereby producing sodium hydrogen carbonate granules.

A solution (2w/w% HPC-SL solution) in which a water-soluble high polymer (specifically hydroxypropylcellulose (HPC-SL)) was dissolved in water was used as a binder 5.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution was sprayed from a spray means provided in the granulation tank, thereby producing citric acid granules.

A solution (2w/w% HPC-SL solution) in which a water-soluble high polymer (specifically hydroxypropylcellulose (HPC-SL)) was dissolved in water was used as a binder 5.

The particle size distribution of the ascorbic acid granules, the sodium hydrogen carbonate granules and the citric acid granules obtained by the above-mentioned procedure is shown in a table 5.

**Table 5**

| particle size | ascorbic acid granules | sodium hydrogen carbonate granules | citric acid granules |
|---|---|---|---|
| more than 710 µm | 2% | 0% | 0% |
| 500 ∼ 110 µm | 24% | 0% | 7% |
| 350 ∼ 500 µm | 37% | 15% | 26% |
| 250 ∼ 350 µm | 28% | 18% | 23% |
| 105 ∼ 250 µm | 7% | 38% | 21% |
| less than 105 µm | 2% | 29% | 23% |
| total | 100% | 100% | 100% |

Thus prepared ascorbic acid granules, sodium hydrogen carbonate granules and citric acid granules were blended at a fixed ratio (in this embodiment, the ascorbic acid, sodium hydrogen carbonate and citric acid were blended in weight ratio of 6:1:1). Then the blended material of the ascorbic acid granules, the sodium hydrogen carbonate granules and the citric acid granules was mixed with a well-known mixer.

As shown in the table 5, when the ascorbic acid granules, the sodium hydrogen carbonate granules and the citric acid granules having different particle sizes were used, it was found that ascorbic acid granules, sodium hydrogen carbonate granules and citric acid granules were not uniformly mixed.

A fixed amount of magnesium stearate (Japanese Pharmacopoeia) was added in the mixture of the ascorbic acid granules, the sodium hydrogen carbonate granules and the citric acid granules and the blended material of the ascorbic acid granules, the sodium hydrogen carbonate granules, the citric acid granules and magnesium stearate (Japanese Pharmacopoeia) was mixed with a well-known mixer.

1.0 weight % of magnesium stearate (Japanese Pharmacopoeia) was required per a tablet in order to prevent the mixture including magnesium stearate (Japanese Pharmacopoeia) from adhering on the upper punches 31···, the lower punches 33··· and the dies 32··· and to avid tabletting problems such as sticking on the produced tablet.

Next the mixture including magnesium stearate (Japanese Pharmacopoeia) was compressed to produce a tablet without applying magnesium stearate (Japanese Pharmacopoeia) on each surface of the upper punches 31···, the lower punches 33··· and the dies 32··· by means of the rotary type tabletting machine 81.

Then a characteristic tests 1, 2 and 3 as mentioned in the experiment example 1 were executed for the tablet obtained by the comparison example 2.

### Characteristic Test 1

One of the tablet (conventional type effevescent tablet) obtained by the comparison example 2 was put in 100ml drinking water, after one minute, the substance floating on the surface of the drinking water and turbidity thereof were observed.

In the drinking water in which the tablet (conventional type effevescent tablet) obtained by the comparison example 2 was dissolved, a floating substance was observed on the water surface.

Further the drinking water in which the tablet (conventional type effevescent tablet) obtained by the comparison example 2 was dissolved was cloudy.

### Characteristic Test 2

The disintegration time of the tablet (conventional type effevescent tablet) obtained by the comparison example 2 was measured according to the disintegration test described in the Japanese Pharmacopoeia (13th edition).

The results are shown in a table 6.

**Table 6**

| | sample | disintegration time |
|---|---|---|
| comparison example 2 | sample 1 | 32 sec. |
| | sample 2 | 37 sec. |
| | sample 3 | 49 sec. |
| | sample 4 | 68 sec. |
| | sample 5 | 75 sec. |
| | sample 6 | 89 sec. |
| | average disintegration time | 70 sec. |

As seen from the result of the tables 3 and 6, the tablet (effevescent tablet 1 of the present invention) obtained by the experiment example 1 has a higher disintegration speed comparing with the tablet (conventional type effevescent tablet) obtained by the comparison example 2.

Further it was found that the disintegration time of the tablet obtained by the comparison example 1 was higher than that obtained by the comparison example 2 (conventional type effevescent tablet).

### Characteristic Test 3

The elution test of the tablet (conventional type effevescent tablet) obtained by the comparison example 2 was executed according to the elution test described in the Japanese Pharmacopoeia (13th edition).

The result is shown in table 7

**Table 7**

| | sample | elution rate |
|---|---|---|
| comparison example 2 | sample 1 | 42% |
| | sample 2 | 50% |
| | sample 3 | 61% |
| | sample 4 | 78% |
| | sample 5 | 88% |
| | average elution rate | 64% |

As seen from the result of the tables 4 and 7, it was found that the elution characteristic of the tablet (effevescent tablet 1 of the present invention) obtained by the experiment example 1 was superior to the tablet (conventional type effevescent tablet) obtained by the comparison example 2.

Further it was also found that the elution characteristic of the tablet obtained by the comparison example 1 was superior to the tablet (conventional type effevescent tablet) obtained by the comparison example 2.

### (Experiment example 2)

Experiment example 2 shows other embodiment when an effevescent tablet for oral administration is produced according to the present invention.

This experiment example 2 corresponds to the effevescent tablet 1A shown in Fig.3 and shows a production example of an effevescent tablet for oral administration wherein granules are produced using a solution including a water-soluble high polymers and surfactant as a binder to be compressed.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powders (Japanese Pharmacopoeia) were granulated to produce ascorbic acid granules.

More specifically, ascorbic acid granules were granulated as follows.

A fixed amount of ascorbic acid powders (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution including a surfactant was sprayed from a spray means provided in the granulation tank, thereby producing ascorbic acid granules.

A solution in which an appropriate amount of surfactant (more specifically polysorbate 80) was dissolved in 2 w/w% solution of a water-soluble high polymer (specifically hydroxypropylcellulose (HPC-SL)) was used as a binder 5.

Sodium hydrogen carbonate granules were granulated according to the following method.

A fixed amount of sodium hydrogen carbonate powders (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution including surfactants was sprayed from a spray means provided in the granulation tank, thereby producing sodium hydrogen carbonate granules.

A solution in which an appropriate amount of surfactants (more specifically polysorbate 80 ) was dissolved in 2 w/w% solution of a water-soluble high polymer (specifically hydroxypropylcellulose (HPC-SL)) was used as a binder 5.

For granulating sodium carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules became the same as that of the ascorbic acid granule.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution including a surfactant was sprayed from a spray means provided in the granulation tank, thereby producing citric acid granules.

A solution in which an appropriate amount of surfactant (more specifically polysorbate 80 ) was dissolved in 2 w/w% solution of a water-soluble high polymer (specifically hydroxypropylcellulose (HPC-SL)) was used as a binder 5.

For granulating citric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the citric acid granules becomes the same as that of the ascorbic acid granules.

The particle size distribution of the ascorbic acid granules, the sodium hydrogen carbonate granules and the citric acid granules obtained by the above-mentioned procedure is shown in a table 8.

**Table 8**

| particle size | ascorbic acid granules | sodium hydrogen carbonate granules | citric acid granules |
|---|---|---|---|
| more than 710 µm | 0% | 0% | 0% |
| 500 ∼710 µm | 1% | 0% | 1% |
| 350 ∼ 500 µm | 20% | 17% | 21% |
| 250 ∼ 350 µm | 46% | 44% | 50% |
| 105 ∼ 250 µm | 22% | 23% | 21% |
| less than 105 µm | 11% | 16% | 7% |
| total | 100% | 100% | 100% |

The ascorbic acid content of thus obtained ascorbic acid granules was 98.91w/w%, the sodium hydrogen carbonate content of the sodium hydrogen carbonate granules was 98.91w/w% and the citric acid content of the citric acid granules was 98.91w/w%.

Thus prepared ascorbic acid granules, sodium hydrogen carbonate granules and citric acid granules were blended at a fixed ratio (in this embodiment, ascorbic acid, sodium hydrogen carbonate and citric acid were blended in weight ratio of 6:1:1).

Then the blended material of the ascorbic acid granules, the sodium hydrogen carbonate granules and the citric acid granules was mixed with a well-known mixer.

As shown in the table 8, when the ascorbic acid granules, the sodium hydrogen carbonate granules and the citric acid granules having almost the same particle size were used, it was found that the ascorbic acid granules, the sodium hydrogen carbonate granules and the citric acid granules were uniformly mixed spontaneously by the external force given by the mixer.

Thus obtained mixture of the ascorbic acid granules, the sodium hydrogen carbonate granules and the citric acid granules was tabletted by means of the external lubrication type tabletting machine S shown in Fig.10.

An upper punch 31, a lower punch 33 and a die 32 with 11mm diameter were used for the rotary type tabletting machine 81.

An upper punch 31 and a lower punch 33 with flat molding surface were used.

The tabletting pressure was 1500kg/punch and the weight per a tablet was controlled to be 405mg±1mg.

Magnesium stearate (Japanese Pharmacopoeia) was contained in the lubricant storage hopper 52 of the quantitative feeder 51 of the external lubrication type tabletting machine S.

A rotary cam 45 having a concavo-convex pattern suitable for dispersing magnesium stearate (Japanese Pharmacopoeia) in air was attached for a rotary axis of the rotary drive means (rotary drive means 41M in Fig.10) of the pulsating vibration air generation means 41 of the external lubrication type tabletting machine S.

Then, the mixture of ascorbic acid granules, sodium hydrogen carbonate granules and citric acid granules was stored in a molding material storage hopper (not shown) connected to the feed shoe 36.

Next, the air source 111 was driven at a fixed drive amount to rotate the rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range of 10Hz to 40Hz.

The supply amount of magnesium stearate (Japanese Pharmacopoeia) into the lubricant spray chamber (lubricant apply means) 91 was controlled by driving the light permeable type powder concentration measuring means 103.

Then, the rotary type tabletting machine 81 was driven and at the same time the mixture was supplied from the feed shoe 36. The mixture was compressed to produce a tablet by means of the upper punches 31··· on which a fixed amount of magnesium stearate (Japanese Pharmacopoeia) was applied, the lower punches 33··· on which a fixed amount of magnesium stearate (Japanese Pharmacopoeia) was applied and the dies 32··· on which a fixed amount of magnesium stearate (Japanese Pharmacopoeia) was applied.

Analyzing thus obtained tablet, the drive amount of air source 111, the drive amount of pulsating vibration air generation means 41 were appropriately controlled in such a manner that the applied amount of magnesium stearate (Japanese Pharmacopoeia) per a tablet became 0.4mg±0.1mg. Then the tablet (effevescent tablet 1A) was produced in earnest.

The preparation of thus produced tablet (effevescent tablet 1A) is shown in a table 9.

**Table 9**

| component | content |
|---|---|
| ascorbic acid | 300.0mg |
| sodium hydrogen carbonate | 50.0mg |
| citric acid | 50.0mg |
| hydroxypropylcellulose (HPC-SL) | 4.0mg |
| polysorbate 80 | 0.4mg |
| magnesium stearate | 0.4mg |
| Total | 405mg/tablet |

### (Experiment example 3)

Experiment example 3 shows other embodiment when an effevescent tablet for oral administration is produced according to the present invention.

This experiment example 1 corresponds to the effevescent tablet 1B shown in Fig.4 and shows a production example of an effevescent tablet for oral administration wherein granules are produced using a solution in which a saccharide with high wettability for water is dissolved as a binder to be compressed.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powders (Japanese Pharmacopoeia) were granulated to produce ascorbic acid granules.

More specifically, ascorbic acid granules were produced as follows.

A fixed amount of ascorbic acid powders (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution containing a surfactant was sprayed from a spray means provided in the granulation tank, thereby producing ascorbic acid granules.

A solution in which a saccharide with high wettability for water was dissolved in water was used instead of a normal binder.

More specifically, mannitol (Japanese Pharmacopoeia) was used as a saccharide with high wettability for water in this embodiment.

Here 20 w/w% solution of mannitol (Japanese Pharmacopoeia) was used as a binder solution.

Sodium hydrogen carbonate granules were granulated according to the following method.

A fixed amount of sodium hydrogen carbonate powder (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution including a surfactant was sprayed from a spray means provided in the granulation tank, thereby producing sodium carbonate granules.

A solution in which a saccharide with high wettability for water was dissolved in water was used instead of a normal binder.

More specifically, mannitol (Japanese Pharmacopoeia) was used as a saccharide with high wettability in this embodiment.

Here 20 w/w% solution of mannitol (Japanese Pharmacopoeia) was used as a binder solution.

For granulating hydrogen sodium carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules to be granulated became the same as that of the ascorbic acid granule.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution including a surfactant was sprayed from a spray means provided in the granulation tank, thereby producing citric acid granules.

A solution in which a saccharide with high wettability for water was dissolved in water was used instead of a normal binder.

More specifically, mannitol (Japanese Pharmacopoeia) was used as a saccharide with high wettability in this embodiment.

Here 20 w/w% solution of mannitol (Japanese Pharmacopoeia) was used as a binder solution.

For granulating citric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the citric acid granules to be granulated becomes the same as that of the ascorbic acid granule.

The particle size distribution of the ascorbic acid granules, the sodium hydrogen carbonate granules and citric acid granules obtained by the above-mentioned procedure is shown in a table 10.

**Table 10**

| particle size | ascorbic acid granules | sodium carbonate granules | citric acid granules |
|---|---|---|---|
| more than 710 µm | 0% | 0% | 0% |
| 500 ∼ 710 µm | 1% | 0% | 1% |
| 350 ∼ 500 µm | 18% | 15% | 20% |
| 250 ∼ 350 µm | 48% | 46% | 51% |
| 105 ∼ 250 µm | 22% | 23% | 21% |
| less than 105 µm | 11% | 16% | 7% |
| total | 100% | 100% | 100% |

From such an experiment, it was found that if a solution in which saccharides were dissolved was used as a binder, the granulated material (granules) with substantially same average particle size and substantially same particle size distribution could be produced even if components were different by controlling granulation conditions such as the temperature and supply amount of the heated air supplied in the granulation tank and spray amount of binder solution per time sprayed from the spray means.

The ascorbic acid content of thus obtained ascorbic acid granules was 90.91w/w%, the sodium hydrogen carbonate content of the sodium hydrogen carbonate granules was 90.91w/w% and the citric acid content of the citric acid granules was 90.91w/w%.

Thus prepared ascorbic acid granules, sodium carbonate granules and citric acid granules were blended at a fixed ratio (in this embodiment, the ascorbic acid, sodium carbonate and citric acid were blended in weight ratio of 6:1:1).

Then the blended material of the ascorbic acid granules, the sodium hydrogen carbonate granules and the citric acid granules was mixed with a well-known mixer.

As shown in the table 10, when the ascorbic acid granules, the sodium hydrogen carbonate granules and the citric acid granules having almost the same particle size were used, it was found that ascorbic acid granules, sodium carbonate granules and citric acid granules were uniformly mixed spontaneously by the external force given by the mixer.

Thus obtained mixture of the ascorbic acid granules, the sodium hydrogen carbonate granules and the citric acid granules was tabletted by means of the external lubrication type tabletting machine S shown in Fig.10.

An upper punch 31, a lower punch 33 and a die 32 with 11mm diameter were used for the rotary type tabletting machine 81.

An upper punch 31 and a lower punch 33 with flat molding surface were used.

The tabletting pressure was 1500kg/punch and the weight per a tablet was controlled to be 440mg±1mg.

Magnesium stearate (Japanese Pharmacopoeia) was contained in the lubricant storage hopper 52 of the quantitative feeder 51 of the external lubrication type tabletting machine S.

A rotary cam 45 having a concavo-convex pattern suitable for dispersing magnesium stearate (Japanese Pharmacopoeia) in air was attached for a rotary axis of the rotary drive means (rotary drive means 41M in Fig.10) of the pulsating vibration air generation means 41 of the external lubrication type tabletting machine S.

Then, the mixture of the ascorbic acid granules, the sodium hydrogen carbonate granules and the citric acid granules was stored in a molding material storage hopper (not shown) connected to the feed shoe 36.

Next, the air source 111 was driven at a fixed drive amount to rotate the rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range pf 10Hz to 40Hz.

The supply amount of the magnesium stearate (Japanese Pharmacopoeia) into the lubricant spray chamber (lubricant apply means) 91 was controlled by driving the light permeable type powder concentration measuring means 103.

Then, the rotary type tabletting machine 81 was driven and at the same time the mixture was supplied from the feed shoe 36. The mixture was compressed to produce a tablet by means of the upper punches 31··· on which a fixed amount of magnesium stearate (Japanese Pharmacopoeia) was applied, the lower punches 33··· on which a fixed amount of magnesium stearate (Japanese Pharmacopoeia) was applied and the dies 32··· on which a fixed amount of magnesium stearate (Japanese Pharmacopoeia) was applied.

Analyzing thus obtained tablet, the drive amount of air source 111, the drive amount of pulsating vibration air generation means 41 were appropriately controlled in such a manner that the applied amount of magnesium stearate per a tablet became 0.4mg±0.1mg. Then the tablet (effevescent tablet 1B) was produced in earnest.

The preparation of thus produced tablet (effevescent tablet 1B) is shown in a table 11.

**Table 11**

| component | content |
|---|---|
| ascorbic acid | 300.0mg |
| sodium hydrogen carbonate | 50.0mg |
| citric acid | 50.0mg |
| mannitol | 41 mg |
| manesium stearate | 0.4mg |
| Total | 441mg/tablet |

### (Experiment example 4)

Experiment example 4 shows other embodiment when an effevescent tablet for oral administration is produced according to the present invention.

This experiment example 4 corresponds to the effevescent tablet 1C shown in Fig.5 and shows a production example of an effevescent tablet for oral administration wherein granules were produced using a solution in which a water-soluble high polymer and a saccharide with high wettability for water were dissolved as a binder to be compressed.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powder (Japanese Pharmacopoeia) were granulated to produce ascorbic acid granules.

More specifically, ascorbic acid granules were granulated as follows.

A fixed amount of ascorbic acid powders (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution was sprayed from a spray means provided in the granulation tank, thereby producing ascorbic acid granules.

A solution in which a water-soluble high polymer and a saccharide with high wettability for water were dissolved in water was used as a binder solution.

More specifically, hydroxypropylcellulose (HPC-SL) was used as a water-soluble high polymer and 2 w/w% solution of hydroxypropylcellulose (HPC-SL) were produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here a binder solution in which mannitol (Japanese Pharmacopoeia) was controlled to be 20 w/w% solution against the entire amount of 2 w/w% solution of hydroxypropylcellulose (HPC-SL) was used.

Sodium hydrogen carbonate granules were granulated according to the following method.

A fixed amount of sodium hydrogen carbonate powders (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution was sprayed from a spray means provided in the granulation tank, thereby producing sodium hydrogen carbonate granules.

A solution in which a water-soluble high polymer and a saccharide with high wettability for water were dissolved in water was used as a binder solution.

More specifically, hydroxypropylcellulose (HPC-SL) was used as a water-soluble high polymer and 2 w/w% solution of hydroxypropylcellulose (HPC-SL) was produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here a binder solution in which mannitol (Japanese Pharmacopoeia) was controlled to be 20 w/w% solution against the entire amount of 2 w/w% solution of hydroxypropylcellulose (HPC-SL) was used.

For granulating sodium hydrogen carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules to be granulated became the same as that of the ascorbic acid granules.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution was sprayed from a spray means provided in the granulation tank, thereby producing citric acid granules.

A solution in which a water-soluble high polymer and a saccharide with high wettability for water were dissolved in water was used as a binder solution.

More specifically, hydroxypropylcellulose (HPC-SL) was used as a water-soluble high polymer and 2 w/w% solution of hydroxypropylcellulose (HPC-SL) were produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here a binder solution in which mannitol (Japanese Pharmacopoeia) was controlled to be 20 w/w% solution against the entire amount of 2 w/w% solution of hydroxypropylcellulose (HPC-SL) was used.

For granulating citric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the citric acid granules to be granulated becomes the same as that of the ascorbic acid granules.

The particle size distribution of the ascorbic acid granules, the sodium hydrogen carbonate granules and the citric acid granules obtained by the above-mentioned procedure is shown in a table 12.

**Table 12**

| particle size | ascorbic acid granules | sodium carbonate granules | citric acid granules |
|---|---|---|---|
| more than 710µm | 0% | 0% | 0% |
| 500 ∼ 710µm | 0% | 0% | 1% |
| 350 ∼ 500µm | 18% | 14% | 20% |
| 250 ∼ 350µm | 49% | 47% | 52% |
| 105 ∼ 250µm | 22% | 23% | 20% |
| less than 105µm | 11% | 16% | 7% |
| total | 100% | 100%. | 100% |

The ascorbic acid content of thus obtained ascorbic acid granules was 90.09w/w%, the sodium hydrogen carbonate content of the sodium hydrogen carbonate granules was 90.09w/w% and the citric acid content of the citric acid granules was 90.09w/w%.

Thus prepared ascorbic acid granules, sodium hydrogen carbonate granules and citric acid granules were blended at a fixed ratio (in this embodiment, ascorbic acid, sodium hydrogen carbonate and citric acid were blended in weight ratio of 6:1:1).

Then the blended material of the ascorbic acid granules, the sodium hydrogen carbonate granules and the citric acid granules was mixed with a well-known mixer.

As shown in the table 12, when the ascorbic acid granules, the sodium hydrogen carbonate granules and citric acid granules having almost the same particle size were used, it was found that ascorbic acid granules, sodium carbonate granules and citric acid granules were uniformly mixed spontaneously by the external force given by the mixer.

Thus obtained mixture of the ascorbic acid granules, the sodium hydrogen carbonate granules and the citric acid granules was tabletted by means of the external lubrication type tabletting machine S shown in Fig.10.

An upper punch 31, a lower punch 33 and a die 32 with 11mm diameter were used for the rotary type tabletting machine 81.

An upper punch 31 and a lower punch 33 with a flat molding surface were used.

The tabletting pressure was 1500kg/punch and the weight per a tablet was controlled to be 445mg±1mg.

Magnesium stearate (Japanese Pharmacopoeia) was contained in the lubricant storage hopper 52 of the quantitative feeder 51 of the external lubrication type tabletting machine S.

A rotary cam 45 having a concavo-convex pattern suitable for dispersing magnesium stearate (Japanese Pharmacopoeia) in air was attached for a rotary axis of the rotary drive means (rotary drive means 41M in Fig.10) of the pulsating vibration air generation means 41 of the external lubrication type tabletting machine S.

Then, the mixture of the ascorbic acid granules, the sodium hydrogen carbonate granules and the citric acid granules was stored in a molding material storage hopper (not shown) connected to the feed shoe 36.

Next, the air source 111 was driven at a fixed drive amount to rotate the rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range of 10Hz to 40Hz.

The supply amount of the magnesium stearate (Japanese Pharmacopoeia) into the lubricant spray chamber (lubricant apply means) 91 was controlled by driving the light permeable type powder concentration measuring means 103.

Then, the rotary type tabletting machine 81 was driven and at the same time the mixture was supplied from the feed shoe 36. The mixture was compressed to produce a tablet by means of the upper punches 31··· on which a fixed amount of magnesium stearate (Japanese Pharmacopoeia) was applied, the lower punches 33··· on which a fixed amount of magnesium stearate (Japanese Pharmacopoeia) was applied and the dies 32··· on which a fixed amount of magnesium stearate (Japanese Pharmacopoeia) was applied.

Analyzing thus obtained tablet, the drive amount of air source 111, the drive amount of pulsating vibration air generation means 41 were appropriately controlled in such a manner that the applied amount of magnesium stearate (Japanese Pharmacopoeia) per a tablet became 0.4mg±0.1mg. Then the tablet (effevescent tablet 1C) was produced in earnest.

The preparation of thus produced tablet (effevescent tablet 1C) is shown in a table 13.

**Table 13**

| component | content |
|---|---|
| ascorbic acid | 300.0mg |
| sodium hydrogen carbonate | 50.0mg |
| citric acid | 50.0mg |
| mannitol | 40.0mg |
| hydroxypropylcellulose (HPC-SL) | 4.0mg |
| manesium stearate | 0.4mg |
| Total | 444mg/tablet |

### (Experiment example 5)

Experiment example 5 shows other embodiment when an effevescent tablet for oral administration is produced according to the present invention.

This experiment example 5 corresponds to the effevescent tablet 1D shown in Fig.6 and shows a production example of an effevescent tablet for oral administration wherein granules were produced using a solution in which a water-soluble high polymer, a saccharide with high wettability for water and surfactants were dissolved as a binder to be compressed.

Ascorbic acid granules were used as granules of a main active ingredient 2···, sodium hydrogen carbonate granules were used as carbonate granules 3··· and citric acid granules were used as organic acid granules 4···.

In this embodiment ascorbic acid powders (Japanese Pharmacopoeia) were granulated to produce ascorbic acid granules.

More specifically, ascorbic acid granules were granulated as follows.

A fixed amount of ascorbic acid powder (Japanese Pharmacopoeia) was contained in a granulation tank of a fluid bed granulation dryer (Glatt Co., Ltd., WSG-type 5). Heated dry air was supplied in the granulation tank so as to fluidize the ascorbic acid powders (Japanese Pharmacopoeia) stored in the granulation tank and a binder solution was sprayed from a spray means provided in the granulation tank, thereby producing ascorbic acid granules.

A solution in which a water-soluble high polymer, a saccharide with high wettability for water and a surfactant were dissolved in water was used as a binder solution.

More specifically, hydroxypropylcellulose (HPC-SL) was used as a water-soluble high polymer and 2 w/w% solution of hydroxypropylcellulose (HPC-SL) was produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here a binder solution in which mannitol (Japanese Pharmacopoeia) was controlled to be 20 w/w% solution against the entire amount of 2 w/w% solution of hydroxypropylcellulose (HPC-SL) was used.

Further a surfactant was added in thus obtained solution containing hydroxypropylcellulose (HPC-SL) and mannitol.

In this embodiment polysorbate 80 was used as a surfactant.

Here a binder solution in which mannitol (Japanese Pharmacopoeia) was controlled to be 0.3 w/w% solution against the entire amount of solution containing hydroxypropylcellulose (HPC-SL) and mannitol was used.

Sodium hydrogen carbonate granules were granulated according to the following method.

A fixed amount of sodium carbonate powder (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution was sprayed from a spray means provided in the granulation tank, thereby producing sodium hydrogen carbonate granules.

A solution in which a water-soluble high polymer and a saccharide with high wettability for water were dissolved in water was used as a binder solution.

A solution in which a water-soluble high polymer, a saccharide with high wettability for water and a surfactant were dissolved in water was used as a binder solution.

More specifically, hydroxypropylcellulose (HPC-SL) was used as a water-soluble high polymer and 2 w/w% solution of hydroxypropylcellulose (HPC-SL) was produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here mannitol (Japanese Pharmacopoeia) was controlled to be 20 w/w% solution against the entire amount of 2 w/w% solution of hydroxypropylcellulose (HPC-SL).

Further surfactant was added in thus obtained solution containing hydroxypropylcellulose (HPC-SL) and mannitol.

In this embodiment polysorbate 80 was used as a surfactant.

Here a binder solution in which mannitol (Japanese Pharmacopoeia) was controlled to be 0.3 w/w% solution against the entire amount of solution containing hydroxypropylcellulose (HPC-SL) and mannitol(Japanese Pharmacopoeia) was used.

For granulating sodium hydrogen carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules to be granulated became the same as that of the ascorbic acid granule.

Citric acid granules were granulated according to the following method.

A fixed amount of citric acid powders (Japanese Pharmacopoeia) was contained in a granulation tank of a fluid bed granulation dryer. Heated dry air was supplied in the granulation tank so as to fluidize the citric acid powders (Japanese Pharmacopoeia) stored in the granulation tank and a binder solution was sprayed from a spray means provided in the granulation tank, thereby producing citric acid granules.

A solution in which a water-soluble high polymer and a saccharide with high wettability for water were dissolved in water was used as a binder solution.

A solution in which a water-soluble high polymer, a saccharide with high wettability for water and a surfactant were dissolved in water was used as a binder solution.

More specifically, hydroxypropylcellulose (HPC-SL) was used as a water-soluble high polymer and 2 w/w% solution of hydroxypropylcellulose (HPC-SL) was produced.

Further, a saccharide with high wettability was added in the 2 w/w% solution of hydroxypropylcellulose (HPC-SL).

In this embodiment, mannitol (Japanese Pharmacopoeia) was used as a saccharide with high wettability for water.

Here a binder solution in which mannitol (Japanese Pharmacopoeia) was controlled to be 20 w/w% solution against the entire amount of 2 w/w% solution of hydroxypropylcellulose (HPC-SL) was used.

Further a surfactant was added in thus obtained solution containing hydroxypropylcellulose (HPC-SL) and mannitol.

In this embodiment polysorbate 80 was used as a surfactant.

Here a binder solution in which mannitol (Japanese Pharmacopoeia) was controlled to be 0.3 w/w% solution against the entire amount of solution containing hydroxypropylcellulose (HPC-SL) and mannitol was used.

For granulating citric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the citric acid granules becomes the same as that of the ascorbic acid granule.

The particle size distribution of the ascorbic acid granules, the sodium hydrogen carbonate granules and the citric acid granules obtained by the above-mentioned procedure is shown in a table 14.

**Table 14**

| particle size | ascorbic acid granules | sodium carbonate granules | citric acid granules |
|---|---|---|---|
| more than 710µm | 0% | 0% | 0% |
| 500 ∼ 710µm | 0% | 0% | 0% |
| 350 ∼ 500µm | 18% | 14% | 20% |
| 250 ∼ 350µm | 50% | 50% | 53% |
| 105 ∼ 250µm | 22% | 23% | 20% |
| less than 105µm | 10% | 13% | 7% |
| total | 100% | 100% | 100% |

The ascorbic acid content of thus obtained ascorbic acid granules was 90.01w/w%, the sodium hydrogen carbonate content of the sodium hydrogen carbonate granules was 90.01w/w% and the citric acid content of the citric acid granules was 90.01w/w%.

Thus prepared ascorbic acid granules, sodium hydrogen carbonate granules and citric acid granules were blended at a fixed ratio (in this embodiment, ascorbic acid, sodium carbonate and citric acid were blended in weight ratio of 6:1:1).

Then the blended material of the ascorbic acid granules, the sodium hydrogen carbonate granules and the citric acid granules was mixed with a well-known mixer.

As shown in the table 14, when the ascorbic acid granules, the sodium hydrogen carbonate granules and the citric acid granules having almost the same particle size were used, it was found that ascorbic acid granules, sodium carbonate granules and citric acid granules were uniformly mixed spontaneously by the external force given by the mixer.

Thus obtained mixture of ascorbic acid granules, the sodium hydrogen carbonate granules and the citric acid granules was tabletted by means of the external lubrication type tabletting machine S shown in Fig.10.

An upper punch 31, a lower punch 33 and a die 32 with 11mm diameter were used for the rotary type tabletting machine 81.

An upper punch 31 and a lower punch 33 with a flat molding surface were used.

The tabletting pressure was 1500kg/punch and the weight per a tablet was controlled to be 445mg±1mg.

Magnesium stearate (Japanese Pharmacopoeia) was contained in the lubricant storage hopper 52 of the quantitative feeder 51 of the external lubrication type tabletting machine S.

A rotary cam 45 having a concavo-convex pattern suitable for dispersing magnesium stearate (Japanese Pharmacopoeia) in air was attached for a rotary axis of the rotary drive means (rotary drive means 41M in Fig.10) of the pulsating vibration air generation means 41 of the external lubrication type tabletting machine S.

Then, the mixture of the ascorbic acid granules, the sodium carbonate granules and the citric acid granules was stored in a molding material storage hopper (not shown) connected to the feed shoe 36.

Next, the air source 111 was driven at a fixed drive amount to rotate the rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range of 10Hz to 40Hz.

The supply amount of magnesium stearate (Japanese Pharmacopoeia) into the lubricant spray chamber (lubricant apply means) 91 was controlled by driving the light permeable type powder concentration measuring means 103.

Then, the rotary type tabletting machine 81 was driven and at the same time the mixture was supplied from the feed shoe 36. The mixture was compressed to produce a tablet by means of the upper punches 31··· on which a fixed amount of magnesium stearate (Japanese Pharmacopoeia) was applied, the lower punches 33··· on which a fixed amount of magnesium stearate (Japanese Pharmacopoeia) was applied and the dies 32··· on which a fixed amount of magnesium stearate (Japanese Pharmacopoeia) was applied.

Analyzing thus obtained tablet, the drive amount of air source 111, the drive amount of pulsating vibration air generation means 41 were appropriately controlled in such a manner that the applied amount of magnesium stearate per a tablet became 0.4mg±0.1mg. Then the tablet (effevescent tablet 1D) was produced in earnest.

The preparation of thus produced tablet (effevescent tablet 1D) is shown in a table 15.

**Table 15**

| component | content |
|---|---|
| ascorbic acid | 300.0mg |
| sodium hydrogen carbonate | 50.0mg |
| citric acid | 50.0mg |
| mannitol | 40.0mg |
| hydroxypropylcellulose (HPC-SL) | 4.0mg |
| polysorbate 80 | 0.4mg |
| manesi um stearate | 0.4mg |
| Total | 444mg/tablet |

Then characteristic tests 1, 2 and 3 as mentioned in the experiment example 1 were executed for the tablet obtained by the experiment examples 2, 3, 4 and 5.

### Characteristic Test 1

In a drinking water in which a tablet (effevescent tablet 1A, 1B, 1C and 1D of the present invention) obtained by each experiment example 2, 3, 4 and 5 was dissolved, the substance floating on the surface of the drinking water wasn't observed.

The drinking water in which a tablet (effevescent tablet 1A, 1B, 1C and 1D of the present invention) obtained by each experiment example 2, 3, 4 and 5 was dissolved was clear.

### Characteristic Test 2

The results of the characteristic test 2 are shown in a table 16.

**Table 16**

| | sample | disintegration time |
|---|---|---|
| experiment example 2 | sample 1 | 15 sec. |
| | sample 2 | 15 sec. |
| | sample 3 | 17 sec. |
| | sample 4 | 19 sec. |
| | sample 5 | 22 sec. |
| | sample 6 | 24 sec. |
| | average disintegration time | 19 sec. |
| experiment example 3 | sample 1 | 19 sec. |
| | sample 2 | 20 sec. |
| | sample 3 | 22 sec. |
| | sample 4 | 25 sec. |
| | sample 5 | 27 sec. |
| | sample 6 | 29 sec. |
| | average disintegration time | 24 sec. |
| experiment example 4 | sample 1 | 17 sec. |
| | sample 2 | 17 sec. |
| | sample 3 | 20 sec. |
| | sample 4 | 23 sec. |
| | sample 5 | 27 sec. |
| | sample 6 | 30 sec. |
| | average disintegration time | 22 sec. |
| experiment example 5 | sample 1 | 16 sec. |
| | sample 2 | 17 sec. |
| | sample 3 | 19 sec. |
| | sample 4 | 22 sec. |
| | sample 5 | 22 sec. |
| | sample 6 | 22 sec. |
| | average disintegration time | 20 sec. |

As seen from the result of the table 16, the tablet (effevescent tablet 1A, 1B, 1C and 1D of the present invention) obtained by each experiment example 2, 3, 4 and 5 had a higher disintegration speed comparing with that obtained by the experiment example 1.

### Characteristic Test 3

The results of the characteristic test 3 are shown in table 17.

**Table 17**

| | sample | elution rate |
|---|---|---|
| experiment example 2 | sample 1 | 93% |
| | sample 2 | 95% |
| | sample 3 | 96% |
| | sample 4 | 96% |
| | sample 5 | 98% |
| | average elution rate | 96% |
| experiment example 3 | sample 1 | 95% |
| | sample 2 | 95% |
| | sample 3 | 96% |
| | sample 4 | 97% |
| | sample 5 | 99% |
| | average elution rate | 96% |
| experiment example 4 | sample 1 | 94% |
| | sample 2 | 95% |
| | sample 3 | 95% |
| | sample 4 | 97% |
| | sample 5 | 99% |
| | average elution rate | 96% |
| experiment example 5 | sample 1 | 94% |
| | sample 2 | 96% |
| | sample 3 | 97% |
| | sample 4 | 97% |
| | sample 5 | 98% |
| | average elution rate | 96% |

As seen from the results of the table 17, it was found that the elution characteristic of the tablet (effevescent tablet 1A, 1B, 1C and 1D of the present invention) obtained by each experiment example 2, 3, 4 and 5 was the same as that of the tablet obtained by the experiment example 1.

### (Experiment example 6)

Experiment example 6 shows one embodiment when an effevescent tablet for a washing detergent is produced according to the present invention.

This experiment example 6 shows an example wherein the effevescent tablet 1D shown in Fig.6 is applied to an effevescent tablet for a washing detergent.

A mixed granules of sodium silicate aluminate and sodium lauryl sulfate (called a mixed granule of sodium silicate aluminate - sodium lauryl sulfate hereinafter) was used as principal (surfactant) agent granules 2A···.

Sodium fatty acid granules (in this embodiment a mixed granules of sodium caprylate, sodium laulrate, sodium myristate, sodium palmitate, sodium stearate, sodium oleate, sodium linoleate) were used for granules of a main active ingredient (fatty acid alkali salt) 2B···.

Sodium hydrogen carbonate granules were used as carbonate granules 3···.

Fumaric acid granules were used as organic acid granules 4···.

Anhydrous sodium sulphate granules were used as anhydrous sodium sulphate.

In this example, a mixed granule of sodium silicate aluminate - sodium lauryl sulfate was obtained by uniformly mixing sodium silicate aluminate powders (Japanese Pharmacopoeia) and sodium lauryl sulfate powders (Japanese Pharmacopoeia) which were blended at a fixed ratio and by granulating the mixed powders of sodium silicate aluminate - sodium lauryl sulfate.

More specifically, the mixed granules of sodium silicate aluminate - sodium lauryl sulfate were granulated as follows.

At first a fixed amount of sodium silicate aluminate powders (Japanese Pharmacopoeia) and a fixed amount of sodium lauryl sulfate powders (Japanese Pharmacopoeia) were blended (in this example sodium silicate aluminate powders (Japanese Pharmacopoeia) and sodium lauryl sulfate powders were blended at a weight ratio of 4:1).

Then thus blended mixture of sodium silicate aluminate powders (Japanese Pharmacopoeia) and sodium lauryl sulfate powders(Japanese Pharmacopoeia) was mixed with a well known mixer, thereby obtaining mixed powders of sodium silicate aluminate powders and sodium lauryl sulfate powders.

A fixed amount of mixed powders of sodium silicate aluminate powders - sodium lauryl sulfate powders was contained in a granulation tank of a fluid bed granulation dryer (Glatt Co., Ltd., WSG-type 5). Heated dry air was supplied in the granulation tank so as to fluidize the mixed powders of sodium silicate aluminate powders - sodium lauryl sulfate powders stored in the granulation tank and a binder solution was sprayed from a spray means provided in the granulation tank, thereby producing a mixed granules of sodium silicate aluminate - sodium lauryl sulfate.

A solution in which a water-soluble high polymer and a surfactant were dissolved in water was used as a binder solution.

More specifically, polyvinyl alcohol (Japanese Pharmacopoeia) was used as water-soluble high polymers and a solution containing 3 w/w% of polyvinyl alcohol was prepared.

Surfactant was added in the solution with 3w/w% polyvinyl alcohol.

Polyethylene glycol (in this example, macrogol 6000 (Japanese Pharmacopoeia)) was used as a surfactant.

A binder solution in which polyethylene glycol (in this example, macrogol 6000 (Japanese Pharmacopoeia)) was controlled to be 0.3 w/w% solution for the entire amount of 3 w/w% polyvinyl alcohol solution was used.

Sodium fatty acid granules were obtained by granulating sodium fatty acid powders (in this example a mixed powder of sodium caprylate, sodium laulrate, sodium myristate, sodium palmitate, sodium stearate, sodium oleate, sodium linoleate) .

More specifically, sodium fatty acid granules were granulated as follows.

At first, a fixed amount of sodium fatty acid powders (in this example a mixed powder of sodium caprylate, sodium laulrate, sodium myristate, sodium palmitate, sodium stearate, sodium oleate, sodium linoleate) (commercial reagent) was contained in a granulation tank of a fluid bed granulation dryer (Glatt Co., Ltd., WSG-type 5). Heated dry air was supplied in the granulation tank so as to fluidize the sodium fatty acid powders stored in the granulation tank and a binder solution was sprayed from a spray means provided in the granulation tank, thereby producing sodium fatty acid granules.

A solution in which water-soluble high polymer and a surfactant were dissolved in water was used as a binder solution.

More specifically, polyvinyl alcohol (Japanese Pharmacopoeia) was use as water-soluble high polymer and a solution containing 3 w/w% of polyvinyl alcohol was prepared.

A surfactant was added in the solution with 3w/w% polyvinyl alcohol.

Polyethylene glycol (in this example, macrogol 6000 (Japanese Pharmacopoeia)) was used as a surfactant.

A binder solution in which polyethylene glycol (in this example, macrogol 6000 (Japanese Pharmacopoeia)) was controlled to be 0.3 w/w% solution for the entire amount of 3 w/w% polyvinyl alcohol solution was used.

For granulating sodium fatty acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium fatty acid granules became the same as that of the mixed granules of sodium silicate aluminate - sodium lauryl sulfate.

Sodium hydrogen carbonate granules were obtained by granulating sodium carbonate powders.

Sodium hydrogen carbonate granules were granulated according to the following method.

A fixed amount of sodium hydrogen carbonate powders (Japanese Pharmacopoeia) was contained in a granulation tank of a fluid bed granulation dryer (Glatt Co., Ltd., WSG-type 5). Heated dry air was supplied in the granulation tank so as to fluidize the sodium hydrogen carbonate powders (Japanese Pharmacopoeia) stored in the granulation tank and a binder solution was sprayed from a spray means provided in the granulation tank, thereby producing sodium carbonate granules.

A solution in which water-soluble high polymer and a surfactant were dissolved in water was used as a binder solution.

More specifically, polyvinyl alcohol (Japanese Pharmacopoeia) was used as water-soluble high polymer and a solution containing 3 w/w% of polyvinyl alcohol was prepared.

A surfactant was added in the solution with 3w/w% polyvinyl alcohol.

Polyethylene glycol (in this example, macrogol 6000 (Japanese Pharmacopoeia)) was used as a surfactant.

A binder solution in which polyethylene glycol (in this example, macrogol 6000 (Japanese Pharmacopoeia)) was controlled to be 0.3 w/w% solution for the entire amount of 3 w/w% polyvinyl alcohol solution was used.

For granulating sodium hydrogen carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules became the same as that of the mixed granule of sodium silicate aluminate - sodium lauryl sulfate.

Fumaric acid granules were obtained by granulating fumaric acid powders.

Fumaric acid granules were produced according to the following method.

A fixed amount of fumaric acid powder (commercial item) was contained in a granulation tank of a fluid bed granulation dryer. Heated dry air was supplied in the granulation tank so as to fluidize the fumaric acid powders stored in the granulation tank and a binder solution was sprayed from a spray means provided in the granulation tank, thereby producing fumaric acid granules.

A solution in which a water-soluble high polymer and a surfactant were dissolved in water was used as a binder solution.

More specifically, polyvinyl alcohol (Japanese Pharmacopoeia) was used as water-soluble high polymer and a solution containing 3 w/w% of polyvinyl alcohol was prepared.

A surfactant was added in the solution with 3w/w% polyvinyl alcohol.

Polyethylene glycol (in this example, macrogol 6000 Japanese Pharmacopoeia)) was used as a surfactant.

A binder solution in which polyethylene glycol (in this example, macrogol 6000 (Japanese Pharmacopoeia)) was controlled to be 0.3 w/w% solution for the entire amount of 3 w/w% polyvinyl alcohol solution was used.

For granulating fumaric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the fumaric acid granules became the same as that of the mixed granule of sodium silicate aluminate - sodium lauryl sulfate.

Anhydrous sodium sulphate granules were obtained by granulating anhydrous sodium sulphate powders.

Anhydrous sodium sulphate granules were produced according to the following method.

A fixed amount of anhydrous sodium sulphate powder (this example used one which is listed on Japanese Standard of Food Additives) was contained in a granulation tank of a fluid bed granulation dryer. Heated dry air was supplied in the granulation tank so as to fluidize the anhydrous sodium sulphate powders stored in the granulation tank and a binder solution was sprayed from a spray means provided in the granulation tank, thereby producing anhydrous sodium sulphate granules.

A solution in which water-soluble high polymer and a surfactant were dissolved in water was used as a binder solution.

More specifically, polyvinyl alcohol (Japanese Pharmacopoeia) was used as water-soluble high polymer and a solution containing 3 w/w% of polyvinyl alcohol was prepared.

A surfactant was added in the solution with 3w/w% polyvinyl alcohol.

Polyethylene glycol (in this example, macrogol 6000 (Japanese Pharmacopoeia)) was used a surfactant.

A binder solution in which polyethylene glycol (in this example, macrogol 6000 (Japanese Pharmacopoeia)) was controlled to be 0.3 w/w% solution for the entire amount of 3 w/w% polyvinyl alcohol solution was used.

For granulating anhydrous sodium sulphate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the anhydrous sodium sulphate granules became the same as that of the mixed granules of sodium silicate aluminate - sodium lauryl sulfate.

The particle size distribution of thus obtained sodium silicate aluminate granules, sodium lauryl sulfate granules, sodium fatty acid granules, (in this example a mixed granule of sodium caprylate, sodium laulrate, sodium myristate, sodium palmitate, sodium stearate, sodium oleate, sodium linoleate), sodium hydrogen carbonate granules, fumaric acid granules and anhydrous sodium sulphate granules is shown in table 18.

**Table 18**

| particle size | sodium silicate aluminate granule | sodium lauryl sulfate granule | sodium fatty acid granule | sodium hydrogen carbonate granule | fumaric acid granule | sodium sulphate anhydrous granule |
|---|---|---|---|---|---|---|
| more than 710µm | 0% | 0% | 0% | 0% | 0% | 0% |
| 500∼710µm | 0% | 0% | 0% | 1% | 0% | 1% |
| 350∼500µm | 16% | 12% | 14% | 19% | 12% | 19% |
| 250∼350µm | 51% | 49% | 48% | 53% | 48% | 53% |
| 105∼250µm | 22% | 23% | 22% | 20% | 22% | 21% |
| less than 105µm | 11% | 16% | 16% | 7% | 17% | 6% |
| total | 100% | 100% | 100% | 100% | 100% | 100% |

In the mixed granules of sodium silicate aluminate granule - sodium lauryl sulfate granule, the content of sodium silicate aluminate was 78.96w/w% and the content of sodium lauryl sulfate was 19.74w/w%.

The content of sodium fatty acid in the obtained sodium fatty acid granules was 98.92w/w%.

The content of sodium hydrogen carbonate in the obtained sodium hydrogen carbonate granules was 98.92w/w%.

The content of fumaric acid in the obtained fumaric acid granules was 98.92w/w%.

The content of anhydrous sodium sulphate in the obtained anhydrous sodium sulphate granules was 98.92w/w%.

Thus prepared mixed granule of sodium silicate aluminate granules - sodium lauryl sulfate granules, sodium fatty acid granules, sodium hydrogen carbonate granules, fumaric acid granules and anhydrous sodium sulphate granules were blended (in this example sodium silicate aluminate granules, sodium lauryl sulfate granules, sodium fatty acid granules, sodium hydrogen carbonate granules, fumaric acid granules and anhydrous sodium sulphate granules were blended so as to be 4:1:2:2:2:2 at weight ratio.)

Then the blended material was mixed with a well-known mixer.

As shown in the table 18, when mixed granules of sodium silicate aluminate granules - sodium lauryl sulfate granules, sodium fatty acid granules, sodium hydrogen carbonate granules, fumaric acid granules and anhydrous sodium sulphate granules having almost the same particle size were used, it was found that mixed granules of sodium silicate aluminate granules - sodium lauryl sulfate granules, sodium fatty acid granules, sodium hydrogen carbonate granules, fumaric acid granules and anhydrous sodium sulphate granules were uniformly mixed spontaneously by the external force given by the mixer.

Thus obtained mixture of mixed granules of sodium silicate aluminate granules - sodium lauryl sulfate granules, sodium fatty acid granules, sodium hydrogen carbonate granules, fumaric acid granules and anhydrous sodium sulphate granules was tabletted by means of the external lubrication type tabletting machine S shown in Fig.10.

An upper punch 31, a lower punch 33 and a die 32 with 14mm diameter were used for the rotary type tabletting machine 81.

An upper punch 31 and a lower punch 33 with a flat molding surface were used.

The tabletting pressure was 2500kg/punch and the weight per a tablet was controlled to be 658mg±1mg.

Sucrose esters of fatty acid (commercial item) was contained in the lubricant storage hopper 52 of the quantitative feeder 51 of the external lubrication type tabletting machine S.

A rotary cam 45 having a concavo-convex pattern suitable for dispersing sucrose esters of fatty acid (commercial item) in air was attached for a rotary axis of the rotary drive means (rotary drive means 41M in Fig.10) of the pulsating vibration air generation means 41 of the external lubrication type tabletting machine S.

Then, the mixture of sodium silicate aluminate granules, sodium lauryl sulfate granules, sodium fatty acid granules, sodium hydrogen carbonate granules, fumaric acid granules and anhydrous sodium sulphate granules was stored in a molding material storage hopper (not shown) connected to the feed shoe 36.

Next, the air source 111 was driven at a fixed drive amount to rotate the rotary drive means (rotary drive means 41M in Fig.10) of the pulsating vibration air generation means 41. The frequency of the positive pulsating vibration air generated from the pulsating vibration air generation means 41 wasn't completely defined, but it was selected from the range of 10Hz to 40Hz.

The supply amount of sucrose esters of fatty acid (commercial item) into the lubricant spray chamber (lubricant apply means) 91 was controlled by driving the light permeable type powder concentration measuring means 103.

The supply amount of sucrose esters of fatty acid (commercial item) into the lubricant spray chamber (lubricant apply means) 91 wasn't completely defined. However, the amount was selected from the range of 200mg/min. to 2000mg/min.

Handling of the supply amount of sucrose esters of fatty acid (commercial item) into the lubricant spray chamber (lubricant apply means) 91 has been considered to be difficult when a steady flow air was used.

Then, the rotary type tabletting machine 81 was driven and at the same time the mixture was supplied from the feed shoe 36. The mixture was compressed to produce a tablet by means of the upper punches 31··· on which a fixed amount of sucrose esters of fatty acid (Japanese Pharmacopoeia) was applied, the lower punches 33··· on which a fixed amount of sucrose esters of fatty acid (commercial item) was applied and the dies 32··· on which a fixed amount of sucrose esters of fatty acid (commercial item) was applied.

Analyzing thus obtained tablet, the drive amount of air source 111, the drive amount of pulsating vibration air generation means 41 were appropriately controlled in such a manner that the applied amount of sucrose esters of fatty acid (commercial item) per a tablet became 0.7mg±0.1mg. Then the tablet (effevescent tablet 1D) was produced in earnest.

The preparation of thus produced tablet (effevescent tablet 1D) is shown in a table 19.

**Table 19**

| component | content |
|---|---|
| sodium silicate aluminate | 200.0mg |
| sodium 1 auryl sulfate | 50.0mg |
| sodium fatty acid | 100.0mg |
| sodium hydrogen carbonate | 100.0mg |
| fumaric acid | 100.0mg |
| anhydrous sodium sulphate | 100.0mg |
| polyvinyl alcohol | 7.0mg |
| macrogol 6000 | 0.7mg |
| sucrose esters of fatty acid | 0.7mg |
| Total | 658mg/tablet |

When water (8 litter) was put in a washing tab of a washing machine for house-use and thus produced tablet (effevescent tablet 1D) was put therein, the tablet was rapidly foamed and dissolved to produce a washing water.

A test wherein the tablet (effevescent tablet 1D) was stored under a room temperature in atmosphere for six months was executed. After six months the tablet wasn't observed to be dissolved by the moisture contained in atmosphere (sample=20 tablets).

From the above-mentioned results, it was found that the tablet (effevescent tablet for a washing detergent) has practicability like powdered or granular washing detergent. Further, because the tablet was a solid agent, a problem such that washing detergents was spilled out of the scoop to be scattered around the washing machine or it gets the user's hands or fingers dirty when it was fed in the tab of the washing machine has been solved.

For producing a practical effevescent tablet for a washing detergent, bleaching agents, protein splitting enzyme, perfumes or PH control agents may be added if necessary.

In this example, the effevescent tablet for a washing detergent including anhydrous sodium sulphate was shown. Anhydrous sodium sulphate was added as drying agents in order to prevent the effevescent tablet for washing detergent from foaming and disintegrating when the tablet sucks moisture in air during storage. It isn't necessarily added in the effevescent tablet for washing detergent.

In this example the mixed granules of sodium silicate aluminate granule and sodium lauryl sulfate granules were used. However, sodium silicate aluminate granules, sodium lauryl sulfate granules, sodium fatty acid granules, sodium hydrogen carbonate granules, fumaric acid granules and anhydrous sodium sulphate granules may be produced respectively and they may be mixed to be compressed with the upper punches 31··· on which a fixed amount of sucrose esters of fatty acid (Japanese Pharmacopoeia) was applied, the lower punches 33··· on which a fixed amount of sucrose esters of fatty acid (commercial item) was applied and the dies 32··· on which a fixed amount of sucrose esters of fatty acid (commercial item) was applied by means of the external lubrication type tabletting machine S.

### (Experiment example 7)

Experiment example 7 shows one embodiment when an effevescent tablet for a bath agent was produced according to the present invention.

Sodium carbonate granules were used as granules of a main active ingredient (a surfactant) 2···.

Sodium hydrogen carbonate granules were used as carbonate granules 3···.

Fumaric acid granules were used as organic acid granules 4.

Sodium carbonate powders (Japanese Pharmacopoeia) were granulated to produce sodium carbonate granules.

More specifically, sodium carbonate granules were produced as follows.

A fixed amount of sodium carbonate powders (commercial item) was contained in a granulation tank of a fluid bed granulation dryer. Heated dry air was supplied in the granulation tank so as to fluidize the sodium carbonate powders stored in the granulation tank and a binder solution was sprayed from a spray means provided in the granulation tank, thereby producing sodium carbonate granules.

Methylcellulose (Japanese Pharmacopoeia) was used as water-soluble high polymer and 3 w/w% methylcellulose solution was prepared and used as a binder solution.

Sodium hydrogen carbonate powders (Japanese Pharmacopoeia) were used to produce sodium hydrogen carbonate granules.

Sodium hydrogen carbonate granules were produced according to the following method.

A fixed amount of sodium hydrogen carbonate powders (commercial item) was contained in a granulation tank of a fluid bed granulation dryer. Heated dry air was supplied in the granulation tank so as to fluidize the sodium hydrogen carbonate powders stored in the granulation tank and a binder solution was sprayed from a spray means provided in the granulation tank, thereby producing sodium hydrogen carbonate granules.

A solution in which water-soluble high polymer was dissolved in water was used as a binder solution.

For granulating sodium hydrogen carbonate granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the sodium hydrogen carbonate granules became the same as that of sodium carbonate granules.

Fumaric acid granules were obtained by granulating fumaric acid powders(Japanese Pharmacopoeia).

Fumaric acid granules were produced according to the following method.

A fixed amount of fumaric acid powder (commercial item) was contained in a granulation tank of a well-known fluid bed granulation dryer. Heated dry air was supplied in the granulation tank so as to fluidize the fumaric acid powders stored in the granulation tank and a binder solution was sprayed from a spray means provided in the granulation tank, thereby producing fumaric acid granules.

A solution in which water-soluble high polymers were dissolved in water was used as a binder solution.

For granulating fumaric acid granules, the temperature and amount of the heated air supplied in the granulation tank of a fluid bed granulation dryer (Glatt Co., Ltd., WSG-type 5) and a spray amount per hour of the binder solution sprayed from the spray means were controlled in such a manner that the particle size of the fumaric acid granules became the same as that of sodium carbonate granule.

The particle size distribution of thus obtained sodium carbonate granules, sodium hydrogen carbonate granules and fumaric acid granules is shown in table 20.

**Table 20**

| particle size | sodium carbonate granules | sodium hydrogen carbonate granules | fumaric acid granules |
|---|---|---|---|
| more than 710µm | 0% | 0% | 0% |
| 500 ∼ 710µm | 0% | 0% | 0% |
| 350 ∼ 500µm | 18% | 14% | 20% |
| 250 ∼ 350µm | 52% | 51% | 55% |
| 105 ∼ 250µm | 20% | 22% | 20% |
| less than 105µm | 10% | 13% | 5% |
| total | 100% | 100% | 100% |

In the mixed granule of sodium carbonate, the content of sodium carbonate was 99.01w/w%. The content of sodium hydrogen carbonate in the obtained sodium hydrogen carbonate granules was 99.01w/w%. The content of fumaric acid in the obtained fumaric acid granules was 99.01w/w%.

Thus prepared sodium carbonate granules, sodium hydrogen carbonate granules and fumaric acid granules were blended (in this example sodium carbonate, sodium hydrogen carbonate and fumaric acid were blended so as to be 3:1:1 at weight ratio.)

Then the blended material was mixed with a well-known mixer.

As shown in the table 20, when sodium carbonate granules, sodium hydrogen carbonate granules and fumaric acid granules having almost the same particle size were used, it was found that those granules were uniformly mixed spontaneously by the external force given by the mixer.

Thus obtained mixture of sodium carbonate granules, sodium hydrogen carbonate granules and fumaric acid granules was tabletted by means of the external lubrication type tabletting machine S shown in Fig.10.

An upper punch 31, a lower punch 33 and a die 32 with 500mm diameter were used for the rotary type tabletting machine 81.

An upper punch 31 and a lower punch 33 with a flat molding surface were used.

The tabletting pressure was 5000kg/punch and the weight per a tablet was controlled to be 51g±0.1g.

Sucrose esters of fatty acid (commercial item) was contained in the lubricant storage hopper 52 of the quantitative feeder 51 of the external lubrication type tabletting machine S.

A rotary cam 45 having a concavo-convex pattern suitable for dispersing sucrose esters of fatty acid in air was attached for a rotary axis of the rotary drive means (rotary drive means 41M in Fig.10) of the pulsating vibration air generation means 41 of the external lubrication type tabletting machine S.

Then, the mixture of the sodium carbonate granule, sodium hydrogen carbonate granules and fumaric acid granules and was stored in a molding material storage hopper (not shown) connected to the feed shoe 36.

Next, the air source 111 was driven at a fixed drive amount to rotate the rotary drive means (rotary drive means 41M) of the pulsating vibration air generation means 41. The frequency of the positive pulsating vibration air generated from the pulsating vibration air generation means 41 wasn't completely defined, but it was selected from the range of 10Hz to 40Hz.

The supply amount of sucrose esters of fatty acid into the lubricant spray chamber (lubricant apply means) 91 was controlled by driving the light permeable type powder concentration measuring means 103.

The supply amount of sucrose esters of fatty acid into the lubricant spray chamber (lubricant apply means) 91 isn't completely defined. However, the amount was selected from the range of 200mg/min. to 2000mg/min.

Then, the rotary type tabletting machine 81 was driven and at the same time the mixture was supplied from the feed shoe 36. The mixture was compressed to produce a tablet by means of the upper punches 31··· on which a fixed amount of sucrose esters of fatty acid was applied, the lower punches 33··· on which a fixed amount of sucrose esters of fatty acid was applied and the dies 32··· on which a fixed amount of sucrose esters of fatty acid was applied.

Analyzing thus obtained tablet, the drive amount of air source 111, the drive amount of pulsating vibration air generation means 41 were appropriately controlled in such a manner that the applied amount of sucrose esters of fatty acid per a tablet became 0.05±0.01g. Then the tablet (effevescent tablet 1D) was produced in earnest.

The preparation of thus produced tablet (effevescent tablet 1D) is shown in a table 21.

**Table 21**

| component | content |
|---|---|
| sodium carbonate | 30.0g |
| sodium hydrogen carbonate | 10.0g |
| fumaric acid | 10.00g |
| methylcellulose | 0.5g |
| sucrose esters of fatty acid | 0.05g |
| Total | 51g/table |

### (Comparison example 3)

A fixed amount of sucrose esters of fatty acid powders (commercial item) was added in the mixture of sodium carbonate granules, sodium hydrogen carbonate granules and fumaric acid granules and the blended material of sodium carbonate granules, sodium hydrogen carbonate granules, fumaric acid granules and sucrose esters of fatty acid powders (commercial item) was mixed with a well-known mixer.

The mixture including sucrose esters of fatty acid powders was compressed to produce a tablet without applying sucrose esters of fatty acid powders on each surface of the upper punches 31···, the lower punches 33··· and the dies 32··· by means of the rotary type tabletting machine 81.

5.0 weight % of sucrose esters of fatty acid powders was required per a tablet in order to prevent the mixture including sucrose esters of fatty acid powders from adhering on the upper punches 31···, the lower punches 33··· and the dies 32··· and to avoid tabletting problems such as sticking on the produced tablet.

Each of the tablet (effevescent tablet for a bath agent) obtained in the experiment example 7 and the tablet (effevescent tablet for a bath agent) obtained in the comparison example 3 was put in a bath tab containing 150 litters of hot water and the conditions of each water were observed visually after 10 minutes.

The hot water in which the tablet (effevescent tablet for a bath agent) obtained in the experiment example 7 was put was clear and didn't show an oil film on the surface. However, the hot water in which the tablet (effevescent tablet for a bath agent) obtained in the comparison example 3 was put was cloudy and showed an oil film on the surface.

From the above-mentioned results, it was found that the tablet (effevescent tablet for a bath agent) of the present invention didn't show an oil film on the surface.

For producing a practical effevescent tablet for a bath agent, disintegrater, disintegration supplements, stabilizers, perfumes or hot spring components may be added in necessary.

### Industrial Applicability

According to the effevescent tablet of the present invention, lubricants aren't contained in the mixture to be compressed with the die and the punch. Only a slight amount of lubricant powders applied on the die and the punch is transferred so as not to include lubricants in a tablet.

Therefore, when the tablet is dissolved in water for use, an oil film is hardly appeared on a solution surface.

On the other hand, according to the effevescent tablet of the present invention, lubricants aren't contained in the mixture to be compressed with the punch and the die, the effevescent tablet is rapidly dissolved while generating carbon dioxide (CO₂) because water is easily permeated in the tablet in water.

According to the effevescent tablet of the present invention, the granules of a main active ingredient, the carbonate granules and the organic acid granules having almost the same diameter are used.

When the composition of the granules of a main active ingredient, the carbonate granules and the organic acid granules at a fixed ratio is mixed with a general mixer, the granules show the same behavior against the external force given by the mixer respectively so that they can be uniformly mixed spontaneously without being distributed unevenly.

When thus obtained mixture is mixed with a general tabletting machine, the granules show the same behavior against the external forces given by the tabletting machine respectively so that they aren't distributed unevenly.

Therefore, the disintegration time, the disintegration pattern and the dissolution time of tablets become uniform when a tablet is put in water for use because the granules of a main active ingredient, the carbonate granules and the organic acid granules are evenly dispersed in the effevescent tablet.

According to the effevescent tablet of the present invention, the granules of a main active ingredient, the composition rate of the granules of a main active ingredient, the carbonate granules and the organic acid granules are arranged in such a manner that the mixture presents a normal particle size distribution having one peak.

As the composition in which the granules of a main active ingredient, the carbonate granules and the organic acid granules are blended in a fixed ratio is mixed with a generally used mixer, it shows the same behavior as the case when one kind of powder material presenting a normal distribution with one peak against the external force given by the mixer. Therefore, the composition is uniformly mixed spontaneously without being distributed unevenly per each granule.

As a result, the granules of a main active ingredient, the carbonate granules and the organic acid granules are uniformly dispersed in the effevescent tablet so that there is no difference between the disintegration time, the disintegration pattern and the dissolution time of the tablets when tablets are put in water.

According to the effevescent tablet of the present invention, carbon dioxide component used in the tablet has been already established as a safe material and generally used. Therefore, such tablet has no problem in view of safety

According to the effevescent tablet of the present invention, the organic acid which has been already established as a safe material, is easily obtainable and can decompose carbonate to generate carbon dioxide (CO₂) getting in touch with water is used, therefore, the resulting effevescent tablet has no problem in view of safety.

According to the effevescent tablet of the present invention, each one of the granules of a main active ingredient, the carbonate granules and the organic acid granules are a granulated material produced with water-soluble polymer as a binder. Therefore, if the effevescent tablet is put in water for use, a binder forming each granule is dissolved in water so that each granule is easily dissolved into a particle level.

As a result, when the effevescent tablet is put in water, the contacting area of the active agent, the carbonate and the organic acid with water becomes large. Then a reaction is caused by the carbonate and the organic acid to be rapidly dissolved in water while generating carbon dioxide(CO₂).

According to the effevescent tablet of the present invention, each one of the granules of a main active ingredient, the carbonate granules and the organic acid granules are granulated using a binder including a surfactant.

As a result, the effevescent tablet is constructed in such a manner that between the particles comprising the granules of a main active ingredient, between the particles comprising the carbonate granules and between the particles comprising the organic acid granules are combined with the binder including a surfactant. Therefore, when the effevescent tablet is put in water for use, the binder bonding the particles comprising each granule easily gets wet because of the surfactant contained therein so that the granules of a main active ingredient, the carbonate granules and the organic acid granules are easily decomposed into a particle unit.

Because the effevescent tablet is constructed in a manner that the granule components are rapidly decomposed into a particle unit when they get in contact with water, the dissolution speed in water is increased comparing with a conventional effevescent tablet.

According to the effevescent tablet for a bath agent of the present invention, sodium carbonate which has been already established as a safe material, are easily obtainable and can decompose carbonate to generate carbon dioxide (CO₂) when they get in touch with water is used for the granules of a main active ingredient, therefore, the resulting effevescent tablet has no problem in view of safety.

According to the effevescent tablet for a washing detergent of the present invention, it is shaped as a tablet, therefore, if one tablet has the amount to be used at one time for putting in the tub of the washing machine, one tablet is merely put in the tub for washing clothes. Unlike conventional powdered or granular washing detergents, it can save the trouble of measuring with a scoop each time of putting the detergent in the washing tub, therefore, such a tabletted detergent is facilitated to be used comparing with the conventional powdered or granular washing detergent.

Further because of the tabletted shape of the effevescent tablet for a washing detergent, there isn't problem such that powdered or granular detergents are scattered around the washing machine or a person's hands or fingers get dirty with the detergent when the detergents are put in the washing tub.

According to the effevescent tablet for a washing detergent of the present invention, because anhydrous sodium sulphate with a hygroscopic property is included in the tablet, it is prevented from naturally foaming by the moisture contained in air while the tablet is stored. Namely, such an effevescent tablet is superior in storage stability.

According to the effevescent tablet for oral administration of the present invention, the granules of a main active ingredient including active agent, the carbonate granules and the organic acid granules are a granulated material produced by means of a binder including a saccharide with high wettability for water.

As a result, the effevescent tablet for oral administration is constructed in such a manner that between the particles comprising the granules of a main active ingredient, between the particles comprising the carbonate granules and between the particles comprising the organic acid granules are combined with the binder including a saccharide with high wettability for water. Therefore, when the effevescent tablet for administration is put in water for dosing, the binder bonding the particles comprising each granule easily gets wet because of the saccharide with high wettability for water contained therein so that the granules of a main active ingredient, the carbonate granules and the organic acid granules are easily decomposed into a particle unit.

Because the effevescent tablet is constructed in a manner that the granule components are rapidly decomposed into a particle unit when they get in contact with water, the dissolution speed in water is increased comparing with a conventional effevescent tablet.

According to the production method of the effevescent tablet of the present invention, lubricant powders are applied on each material contacting surface of a punch and a die which are used for compressing the mixture to produce a tablet and the mixture is compressed with the punch and the die on which material contacting surfaces are lubricated. Therefore no lubricant is required to be contained in the mixture.

The effevescent tablet is produced in such a manner no lubricant powders are included in the mixture or almost no lubricant powders are included therein, an oil film hardly floats on the solution surface if the tablet is dissolved in water for dosing.

If lubricants are contained in the mixture (molding material), water has difficulty to be permeated in the tablet because of the water repellency of the lubricants when the effevescent tablet is put in water so that the dissolution speed of the tablet becomes slow. On the other hand, the effevescent tablet is produced according to the present invention wherein lubricant powders are applied on the material contacting surface of the punch and the die which are used for compressing a mixture to produce a tablet and the mixture is compressed with the lubricated punch and die. Therefore, even if lubricant powders aren't added in the mixture, the effevescent tablet can be produced without causing tabletting problems such as sticking and so on.

Hence, if such a production method of the effevescent tablet is used, the effevescent tablet without including lubricant powders therein or the effevescent tablet scarcely including lubricant powders can be produced. Therefore, according to the resulting effevescent tablet, water is rapidly permeated in the tablet so that the tablet is dissolved in water in a short time while generating carbon dioxide (CO₂).

Comparing with the conventional effevescent tablet, the effevescent tablet obtained by the present production method has higher dissolving speed in water.

According to the production method of an effevescent tablet according to the present invention, the lubricant powders mixed with and dispersed in a positive pulsating vibration air is sprayed on the material contacting surfaces of the punch and the die. Therefore, a minimum amount of lubricant powders can be uniformly applied on the material contacting surfaces of the punch and the die by a function of the positive pulsating vibration air.

As a result of such a production method, an effevescent tablet can be continuously produced without causing tabletting problems such as sticking on the produced tablets and without causing grinding on the punch and the die during tabletting.

In other words, the production method can be preferably applied as a production method of an effevescent tablet which is industrially viable.

According to the production method of an effevescent tablet of the present invention, lubricant powders mixed with and dispersed in a positive pulsating vibration air are sprayed from the lubricant powder spray port for lower punch provided for the lubricant apply means on the material contacting surface (upper face) of the lower punch on which lubricant powders are apt to be easily accumulated by gravity, thereby extra lubricant powders on the material contacting surface (upper face) of the lower punch can be blown off by the positive pulsating vibration air.

Therefore, a minimum amount of lubricant powders can be uniformly applied on the material contacting surface (upper face) of the lower punch on which extra lubricant powders are easily applied by gravity.

The extra lubricant powders blown out of the material contacting surface (upper face) of the lower punch by the positive pulsating vibration air apply on the material contacting surface of the die. Then the extra lubricant powders on the material contacting surface of the die is fed to the slit-like lubricant powder spray port for upper punch provided for the lubricant apply means.

As the result, a minimum amount of lubricant powders can be uniformly applied on the material contacting surface (inner circumference) of the die.

Further according to the production method of an effevescent tablet, lubricant powders can be applied on the material contacting surface (lower face) of the upper punch on which lubricant powders are hardly applied by gravity taking enough time in such a manner that lubricant powders are sprayed from the slit- like lubricant spray port for upper punch into the direction of the material contacting surface (lower face) of the upper punch while the upper punch is moved from the initial end to the terminal end of the slit-like lubricant spray port for upper punch.

Thereby, necessary amount of lubricant powders can be applied on the material contacting surface (lower face) of the upper punch on which lubricant powders are hardly applied by gravity.

In other words, according to this production method of an effevescent tablet, the application method of lubricant powders on the material contacting surface (upper face) of the lower punch and that on the material contacting surface (lower face) of the upper punch are differed considering the gravity, thereby necessary amount of lubricant powders can be uniformly applied on the material contacting surface (upper face) of the lower punch and the material contacting surface (lower face) of the upper punch and necessary amount of lubricant powders can be also uniformly applied on the material contacting surface (inner circumference) of the die.

As a result of such a production method, an effevescent tablet can be continuously produced for a long time without causing tabletting problems such as sticking on the produced tablets and without causing grinding on the punch and the die during tabletting.

In other words, the production method can be preferably applied as an industrially viable production method of an effevescent tablet.

According to the production method of the effevescent tablet of the present invention, the granules of a main active ingredient, the carbonate granules and the organic acid granules which have almost the same diameter are used.

Therefore, when the composition of the granules of a main active ingredient, the carbonate granules and the organic acid granules at a fixed ratio is mixed with a general mixer, each granules show the same behavior against the external force given by the mixer so that each granule can be uniformly mixed spontaneously without being distributed unevenly.

Further, when thus obtained mixture is mixed with a general tabletting machine, each granule shows the same behavior against the external force given by the tabletting machine so that each granule isn't distributed unevenly.

Applying this production method, the effevescent tablet in which the granules of a main active ingredient, the carbonate granules and the organic acid granules are uniformly dispersed can be easily produced. Therefore, the required number of the effevescent tablet which has uniform disintegration time, disintegration pattern and dissolution time when the tablet is put in water for dosing can be easily produced depending on the user's needs.

According to the production method of an effevescent tablet of the present invention, the blended ratio of the granules of a main active ingredient, the carbonate granules and the organic acid granules is designed such that the mixture presents a normal particle size distribution with one peak after they are mixed.

As the composition in which the granules of a main active ingredient, the carbonate granules and the organic acid granules are blended in a fixed ratio is mixed with a general mixer, it shows the same behavior against the external force given by the mixer as the case when one kind of powder material presenting a normal distribution with one peak is mixed. Therefore, the composition is uniformly mixed spontaneously without being distributed unevenly per each granule.

Applying this production method, the effevescent tablet in which the granules of a main active ingredient, the carbonate granules and the organic acid granules are uniformly dispersed can be easily produced. Therefore, the required number of the effevescent tablet which has uniform disintegration time, disintegration pattern and dissolution time when the tablet is put in water for use can be easily produced depending on the user's needs.

According to the production method of an effevescent tablet of the present invention, components of which safety has been already established as carbon dioxide components of an effevescent tablet, which have been generally used and which has no problem in view of safety are used. Therefore, the effevescent tablet produced by this production method also has high reliability.

According to the production method of an effevescent tablet of the present invention, the organic acid granules have safety which has been already established, are easily obtainable and can decompose carbonate to generate carbon dioxide (CO₂) when they get in touch with water, therefore, the resulting effevescent tablets using such components have no problem in view of safety.

According to the production method of an effevescent tablet of the present invention, the granulated material is produced by granulating the granules of a main active ingredient, the carbonate granules and the organic acid granules using water-soluble high polymer as a binder. Therefore, if the effevescent tablet is put in water for use, a binder forming each granule is easily dissolved in water so that each granule is rapidly dissolved into a particle level.

As a result, in the production method, when the effevescent tablet is put in water, the contacting area of the main active agent, the carbonate and the organic acid with water becomes large. Then a reaction is caused by the carbonate and the organic acid to be rapidly dissolved in water while generating carbon dioxide (CO₂).

According to the production method of an effevescent tablet of the present invention, each one of the carbonate granules and the organic acid granules are granulated materials produced with a binder including a surfactant.

As a result, the effevescent tablet is constructed in such a manner that between the particles comprising the granules of a main active ingredient, between the particles comprising the carbonate granules and between the particles comprising the organic acid granules are combined with the binder including surfactant. Therefore, when the effevescent tablet is put in water for use, the binder bonding the particles comprising each granule easily gets wet because of the surfactant contained therein so that the granules of a main active ingredient, the carbonate granules and the organic acid granules are easily decomposed into a particle unit.

Because the effevescent tablet produced by this production method is constructed in a manner that the granule components are rapidly decomposed into a particle unit when they get in contact with water, the dissolution speed in water is increased comparing with a conventional effevescent tablet.

According to the production method of an effevescent tablet for a bath agent of the present invention, sodium carbonate which has been already established as safe component, are easily obtainable and can decompose carbonate to generate carbon dioxide (CO₂) when they get in touch with water is used for the granules of a main active ingredient, therefore, the resulting effevescent tablet has no problem in view of safety.

According to the production method of an effevescent tablet for washing detergent of the present invention, the effevescent tablet for a washing detergent is shaped as a tablet, therefore, if one tablet has the amount to be used at one time for putting in the tub of the washing machine, one tablet is merely put in the tub for washing clothes. Unlike conventional powdered or granular washing detergents, it can save the trouble of measuring with a scoop each time of putting the detergents in the washing tub, therefore, such a tabletted detergent is facilitated to be used comparing with the conventional powdered or granular washing detergent.

Further in the production method, because of the tabletted shape of the effevescent tablet for a washing detergents, there isn't problem such that powdered or granular detergents are scattered around the washing machine or a person's hands or fingers get dirty with the detergents when the detergent are put in the washing tub.

According to the production method of an effevescent tablet for washing detergent of the present invention, because anhydrous sodium sulphate with a hygroscopic property is included in the tablet, it is prevented from naturally foaming by the moisture contained in air while the tablet is stored. Namely, such an effevescent tablet is superior in storage stability.

According to the production method of an effevescent tablet for oral administration of the present invention, the granulated material obtained by granulating granules of a main active ingredient, carbonate granules and organic acid granules using a binder including saccharide with high wettability for water are used.

As a result, the effevescent tablet for oral administration produced by this method is constructed in such a manner that between the particles comprising the granules of a main active ingredient, between the particles comprising the carbonate granules and between the particles comprising the organic acid granules are combined with the binder including a saccharide with high wettability for water. Therefore, when the effevescent tablet for oral administration is put in water for use, the binder bonding the particles comprising each granule easily gets wet because of the saccharide with high wettability for water contained therein so that the granules of a main active ingredient, the carbonate granules and the organic acid granules are easily decomposed into a particle unit.

Because the effevescent tablet for oral administration obtained by this method is constructed in a manner that the granule components are rapidly decomposed into a particle unit when they get in contact with water, the dissolution speed in water is increased comparing with a conventional effevescent tablet.

## Claims

1. An effervescent tablet comprising a tablet body which is produced by compressing a mixture comprising granules of a main active ingredient, carbonate granules, and organic acid granules, wherein said granules of a main active ingredient, said carbonate granules, and said organic acid granules are blended such that the particle size distribution of their granule mixture presents a normal distribution with one peak, said mixture does not comprise lubricants, and wherein lubricant powders are attached on the surface of said tablet body,
said lubricant powders being applied onto a punch and a die and transferred to the surface of said tablet body when a compressing step is executed using said punch and said die.

2. The effervescent tablet as set forth in claim 1,
wherein said granules of a main active ingredient, said carbonate granules, and said organic acid granules are all almost the same in their particle diameters.

3. The effervescent tablet as set forth in any one of claims 1 or 2, wherein said carbonate granules are those comprising at least one kind or a mixture of at least two kinds selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

4. The effervescent tablet as set forth in any one of claims 1 3, wherein said organic acid granules are those comprising at least one kind selected from the group consisting of citric acid, tartaric acid, fumaric acid, succinic acid, adipic acid, malic acid, and maleic acid.

5. The effervescent tablet as set forth in any one of claims 1 - 4, wherein each of said granules of a main active ingredient, said carbonate granules and said organic acid granules are those granulated with a water-soluble polymer as a binder, respectively.

6. The effervescent tablet as set forth in any one of claims 1 - 5, wherein each of said granules of a main active ingredient, said carbonate granules and said organic acid granules are those granulated with a binder including a surfactant.

7. An effervescent tablet for a bath agent,
wherein said granules of a main active ingredient as set forth in any one of claims 1-6 are sodium carbonate granules.

8. An effervescent tablet for a washing detergent,
wherein said granules of a main active ingredient as set forth in any one of claims 1-6 include surfactant granules and fatty acid alkali salt granules.

9. The effervescent tablet for a washing detergent as set forth in claim 8,
wherein anhydrous sodium sulphate is further included.

10. An effervescent tablet for oral administration comprising a tablet body which is produced by compressing a mixture comprising granules of a main active ingredient which are those granulated with a binder including a saccharide with high wettability for water, carbonate granules which are those granulated with a binder including a saccharide with high wettability for water, and organic acid granules which are those granulated with a binder including a saccharide with high wettability for water, wherein said granules of a main active ingredient, said carbonate granules, and said organic acid granules are blended such that the particle size distribution of their granules mixture presents a normal distribution with one peak, said mixture does not comprises lubricants, and wherein lubricant powders are attached on the surface of said tablet body,
said lubricant powders being applied onto a punch and a die and transferred to the surface of said tablet body when a compressing step is executed using said punch and said die.

11. The effervescent tablet for oral administration as set forth in claim 10,
wherein said granules of a main active ingredient, said carbonate granules, and said organic acid granules are all almost the same in their particle diameters.

12. The effervescent tablet for oral administration as set forth in any one of claims 10 or 11, wherein said carbonate granules are those comprising at least one kind or a mixture of at least two kinds selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

13. The effervescent tablet for oral administration as set forth in any of claims 10-12, wherein said organic acid granules are those comprising at least one kind selected from the group consisting of citric acid, tartaric acid, fumaric acid, succinic acid, adipic acid, malic acid, and maleic acid.

14. A production method of an effervescent tablet, comprising the steps of:
preparing a mixture including granules of a main active ingredient, carbonate granules, and organic acid granules; wherein said granules of a main active ingredient, said carbonate granules and said organic acid granules are blended such that the particle size distribution of their granule mixture presents a nornal distribution with one peak and said mixture does not comprise lubricants, and applying lubricant powders onto a material contacting surface of each of a punch and a die, both being used for compressing said mixture to produce said tablet, and
compressing said mixture with said punch and said die, on material contacting surfaces of which said lubricant powders are attached.

15. The production method of an effervescent tablet as set forth in claim 14,
wherein said step of applying lubricant powders onto a material contacting surface of each of a punch and a die, both being used for compressing said mixture to produce said tablet, is executed in a manner that lubricant powders mixed with and dispersed in a positive pulsating vibration air are sprayed onto said material contacting surface of each of said punch and said die, both being used for compressing said mixture to produce said tablet.

16. The production method of an effervescent tablet as set forth in claim 14,
wherein said step of applying lubricant powders onto a material contacting surface of each of a punch and a die, both being used for compressing said mixture to produce said tablet, comprises the steps of:
a first lubricant applying step in which lubricant powders are applied onto said material contacting surfaces of a lower punch and a die, and
a second lubricant applying step in which lubricant powders are applied onto said material contacting surface of an upper punch,
said lower punch, said upper punch and said die being used for compressing said mixture to produce said tablet;
wherein said first lubricant applying step comprises:
- spraying lubricant powders mixed with and dispersed in a positive pulsating vibration air from a lubricant spray port for lower punch, which is provided in a lubricant application means, onto the material contacting surface of said lower punch, which is inserted in a predetermined position in said die; and
- further applying lubricant powders onto said material contacting surface of said die, in which said lubricant powders are those blown off from said material contacting surface of said lower punch by said positive pulsating vibration air among lubricant powders sprayed onto said material contacting surface of said lower punch; and
wherein said second lubricant applying step comprises:
- blowing lubricant powders into a direction of the material contacting surface of said upper punch from a slit-like lubricant spray port for upper punch provided in said lubricant application means, said lubricant powders being such lubricant powders as to be left as residual lubricant powders which have not been attached onto each of said material contacting surfaces of said lower punch and said die among those sprayed, while being mixed with and dispersed in said positive pulsating vibration air onto each of said material contacting surface of said lower punch and that of said die from a lubricant spray port for lower punch, which is provided in a lubricant application means; and
- moving said upper punch from the initial end of said slit-like lubricant spray port for upper punch to the terminal end thereof, thereby taking enough time and applying lubricant powders to said material contacting surface of said upper punch.

17. The production method of an effervescent tablet as set forth in any one of claims 14-16, wherein said granules of a main active ingredient, said carbonate granules and said organic acid granules are almost the same in their particle diameters.

18. The production method of an effervescent tablet as set forth in any one of claims 14-17, wherein said carbonate granules are those comprising at least one kind or a mixture of at least two kinds selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

19. The production method of an effervescent tablet as set forth in any one of claims 14-18, wherein said organic acid granules are those comprising at least one kind selected from the group consisting of citric acid, tartaric acid, fumaric acid, succinic acid, adipic acid, malic acid, and maleic acid.

20. The production method of an effervescent tablet as set forth in any one of claims 14-19, wherein each one of said granules of a main active ingredient, said carbonate granules and said organic acid granules are those granulated with a water-soiuble polymer as a binder.

21. The production method of an effervescent tablet as set forth in any one of claims 14-20, wherein each one of said granules of a main active ingredient, said carbonate granules and said organic acid granules are granulated with a binder including a surfactant.

22. A production method of an effervescent tablet for a bath agent,
wherein said granules of a main active ingredient used in the production method of an effervescent tablet as set forth in any one of claims 14-21 are sodium carbonate granules.

23. A production method of an effervescent tablet for a washing detergent, wherein said granules of a main active ingredient used in the production method of an effevescent tablet as set forth in any one of claims 14-21 include surfactant granules and fatty acid alkali salt granules.

24. The production method of an effervescent tablet for a washing detergent as set forth in claim 23, wherein anhydrous sodium sulphate is further added in said mixture.

25. A production method of an effervescent tablet for oral administration comprising a tablet body which is produced by compressing a mixture comprising granules of a main ingredient which are those granulated with a binder including a saccharides with high wettability for water, carbonate granules which are those granulated with a binder including a saccharide with high wettability for water, and organic acid granules which are those granulated with a binder including a saccharide with high wettability for water, wherein said granules of a main active ingredient, said carbonate granules, and said organic acid granules are blended such that the particle size distribution of their granule mixture presents a normal dístrihution with one peak and said mixture does not comprise lubricants and wherein lubricant powders are attached on the surface of said tablet body,
said lubricant powders being applied onto a punch and a die and transferred to the surface of said tabler body when a compressing step is executed using said punch and said die.

26. The production method of effervescent tablet for oral administration as set forth in claim 25,
wherein said granules of a main active ingredient, said carbonate granules, and said organic acid granules are all almost the same in their particle diameters.

27. The production method of effervescent tablet for oral administration as set forth in any one of claims 25 or 26; wherein said carbonate granules are those comprising at least one kind or a mixture of at least two kinds selected from the group consisting of sodium hydrogen carbonate, sodium carbonate, sodium sesquicarbonate, calcium carbonate, potassium carbonate, magnesium carbonate, calcium hydrogen carbonate, and potassium hydrogen carbonate.

28. The production method of effervescent tablet for oral administration as set forth in any of claims 25-27, wherein said organic acid granules are those comprising at least one hind selected from the group consisting of citric acid, tartaric acid, fumaric acid, succinic acid, adipic acid, malic acid, and maleic acid.

## Patentansprüche

1. Brausetablette, umfassend einen Tablettenkörper, der durch Komprimieren eines Gemisches hergestellt ist, das Körnchen eines Hauptwirkstoffes, Carbonatkörnchen und Körnchen einer organischen Säure umfasst, wobei die Körnchen eines Hauptwirkstoffes, die Carbonatkörnchen und die Körnchen der organischen Säure derart gemischt sind, dass die Teilchengrößenverteilung ihres Körnchengemisches eine normale Verteilung mit einem Peak aufweist, das Gemisch keine Schmiermittel umfasst und wobei Schmiermittelpulver an die Oberfläche des Tablettenkörpers gebunden ist, wobei das Schmiermittelpulver auf einen Stempel und ein Formwerkzeug aufgetragen und auf die Oberfläche des Tablettenkörpers übertragen wird, wenn ein Kompressionsschritt unter Verwendung des Stempels und des Formwerkzeuges ausgeführt wird.

2. Brausetablette gemäß Anspruch 1, wobei die Körnchen eines Hauptwirkstoffes, die Carbonatkörnchen und die Körnchen einer organischen Säure alle in ihren Teilchendurchmessern nahezu gleich sind.

3. Brausetablette gemäß einem der Ansprüche 1 oder 2, wobei die Carbonatkörnchen solche sind, die mindestens eine Art oder ein Gemisch von mindestens zwei Arten, ausgewählt aus der Gruppe Natriumhydrogencarbonat, Natriumcarbonat, Natriumsesquicarbonat, Calciumcarbonat, Kaliumcarbonat, Magnesiumcarbonat, Calciumhydrogencarbonat und Kaliumhydrogencarbonat, umfassen.

4. Brausetablette gemäß einem der Ansprüche 1 bis 3, wobei die Körnchen der organischen Säure solche sind, die mindestens eine Art, ausgewählt aus der Gruppe Zitronensäure, Weinsäure, Fumarsäure, Bernsteinsäure, Adipinsäure, Äpfelsäure und Maleinsäure, umfassen.

5. Brausetablette gemäß einem der Ansprüche 1 bis 4, wobei die Körnchen eines Hauptwirkstoffes, die Carbonatkörnchen und die Körnchen einer organischen Säure alle solche sind, die jeweils mit einem wasserlöslichen Polymer als Bindemittel granuliert sind.

6. Brausetablette gemäß einem der Ansprüche 1 bis 5, wobei die Körnchen eines Hauptwirkstoffes, die Carbonatkörnchen und die Körnchen einer organischen Säure alle solche sind, die mit einem Bindemittel, das ein grenzflächenaktives Mittel einschließt, granuliert sind.

7. Brausetablette für einen Badezusatz, wobei die Körnchen eines Hauptwirkstoffes gemäß einem der Ansprüche 1 bis 6 Natriumcarbonatkörnchen sind.

8. Brausetablette für ein Waschmittel, wobei die Körnchen eines Hauptwirkstoffes gemäß einem der Ansprüche 1 bis 6 Körnchen eines grenzflächenaktiven Mittels und Körnchen von Fettsäurealkalisalzen einschließen.

9. Brausetablette für ein Waschmittel gemäß Anspruch 8, wobei ferner wasserfreies Natriumsulfat enthalten ist.

10. Brausetablette zur oralen Verabreichung, umfassend einen Tablettenkörper, der durch Komprimieren eines Gemisches hergestellt ist, das Körnchen eines Hauptwirkstoffes, die solche sind, die mit einem ein Saccharid mit hoher Benetzbarkeit für Wasser einschließenden Bindemittel granuliert sind, Carbonatkörnchen, die solche sind, die mit einem ein Saccharid mit hoher Benetzbarkeit für Wasser einschließenden Bindemittel granuliert sind, und Körnchen einer organischen Säure, die solche sind, die mit einem ein Saccharid mit hoher Benetzbarkeit für Wasser einschließenden Bindemittel granuliert sind, umfasst, wobei die Körnchen eines Hauptwirkstoffes, die Carbonatkörnchen und die Körnchen der organischen Säure derart gemischt sind, dass die Teilchengrößenverteilung ihres Körnchengemisches eine normale Verteilung mit einem Peak aufweist, das Gemisch keine Schmiermittel umfasst und wobei Schmiermittelpulver auf die Oberfläche des Tablettenkörpers aufgebracht ist, wobei das Schmiermittelpulver auf einen Stempel und ein Formwerkzeug aufgetragen und auf die Oberfläche des Tablettenkörpers übertragen wird, wenn ein Kompressionsschritt unter Verwendung des Stempels und des Formwerkzeuges ausgeführt wird.

11. Brausetablette zur oralen Verabreichung gemäß Anspruch 10, wobei die Körnchen eines Hauptwirkstoffes, die Carbonatkörnchen und die Körnchen einer organischen Säure alle in ihren Teilchendurchmessern nahezu gleich sind.

12. Brausetablette zur oralen Verabreichung gemäß einem der Ansprüche 10 oder 11, wobei die Carbonatkörnchen solche sind, die mindestens eine Art oder ein Gemisch von mindestens zwei Arten, ausgewählt aus der Gruppe Natriumhydrogencarbonat, Natriumcarbonat, Natriumsesquicarbonat, Calciumcarbonat, Kaliumcarbonat, Magnesiumcarbonat, Calciumhydrogencarbonat und Kaliumhydrogencarbonat, umfassen.

13. Brausetablette zur oralen Verabreichung gemäß einem der Ansprüche 10 bis 12, wobei die Körnchen einer organischen Säure solche sind, die mindestens eine Art, ausgewählt aus der Gruppe Zitronensäure, Weinsäure, Fumarsäure, Bernsteinsäure, Adipinsäure, Äpfelsäure und Maleinsäure, umfassen.

14. Verfahren zur Herstellung einer Brausetablette, umfassend die Schritte:
Herstellung eines Gemisches, das Körnchen eines Hauptwirkstoffes, Carbonatkörncnen und Körnchen einer organischen Säure einschließt; wobei die Körnchen eines Hauptwirkstoffes, Carbonatkörnchen und Körnchen einer organischen Säure derart gemischt werden, dass die Teilchengrößenverteilung ihres Körnchengemisches eine normale Verteilung mit einem Peak aufweist, und das Gemisch keine Schmiermittel umfasst, und
Auftragen von Schmiermittelpulver auf ein Material, das jeweils mit der Oberfläche eines Stempels und eines Formwerkzeuges in Kontakt ist, wobei beide zur Komprimierung des Gemisches verwendet werden, um die Tablette herzustellen, und
Komprimierung des Gemisches mit dem Stempel und dem Formwerkzeug auf Material, das mit den Oberflächen, auf denen Schmiermittelpulver aufgetragen ist, in Kontakt ist.

15. Verfahren zur Herstellung einer Brausetablette gemäß Anspruch 14, wobei der Schritt des Auftragens von Schmiermittelpulver auf ein Material, das mit der Oberfläche sowohl eines Stempels als auch eines Formwerkzeuges in Kontakt ist, wobei beide zur Komprimierung des Gemisches verwendet werden, um die Tablette herzustellen, in einer Weise ausgeführt wird, dass die Schmiermittelpulver, gemischt mit und dispergiert in einer positiv pulsierenden Vibrationsluft, auf das Material gesprüht werden, das mit der Oberfläche sowohl des Stempels und des Formwerkzeuges in Kontakt ist, wobei beide zur Komprimierung des Gemisches verwendet werden, um die Tablette herzustellen.

16. Verfahren zur Herstellung einer Brausetablette gemäß Anspruch 14, wobei der Schritt des Auftragens von Schmiermittelpulver auf ein Material, das mit der Oberfläche sowohl eines Stempels als auch eines Formwerkzeuges in Kontakt ist, wobei beide zur Komprimierung des Gemisches verwendet werden, um die Tablette herzustellen, die Schritte umfasst:
einen ersten Schritt zum Auftragen eines Schmiermittels, in dem Schmiermittelpulver auf das Material aufgebracht wird, das mit den Oberflächen eines unteren Stempels und eines Formwerkzeuges in Kontakt ist und
einen zweiten Schritt zum Auftragen eines Schmiermittels, in dem Schmiermittelpulver auf das Material aufgebracht wird, das mit der Oberfläche eines oberen Stempels in Kontakt ist,
wobei der untere Stempel, der obere Stempel und das Formwerkzeug zur Komprimierung des Gemisches verwendet werden, um die Tablette herzustellen;
wobei der erste Schritt zum Auftragen eines Schmiermittels umfasst:
- Aufsprühen von Schmiermittelpulver, gemischt mit und dispergiert in einer positiv pulsierenden Vibrationsluft, von einem Schmiermittelsprühauslass für den unteren Stempel, welcher in einer Schmiermittelaufbringvorrichtung vorgesehen ist, auf das Material, das mit der Oberfläche des unteren Stempels in Kontakt ist, der in einer vorher festgelegten Position in das Formwerkzeug eingeführt wird; und
- weiteres Aufbringen von Schmiermittelpulver auf das Material, das mit der Oberfläche des Formwerkzeuges in Kontakt ist, wobei das Schmiermittelpulver von dem auf das Material, das mit der Oberfläche des unteren Stempels in Kontakt ist aufgesprühten Schmiermittelpulver jenes ist, das durch die positiv pulsierende Vibrationsluft von dem Material weggeblasen wird, das mit der Oberfläche des unteren Stempels in Kontakt ist; und
wobei der zweite Schritt zum Aufbringen eines Schmiermittels umfasst:
- Blasen des Schmiermittelpulvers in Richtung des Materials, das mit der Oberfläche des oberen Stempels in Kontakt ist, von einem schlitzartigen Schmiermittelsprühauslass für den oberen Stempel, der in der Schmiermittelaufbringvorrichtung vorgesehen ist, wobei das Schmiermittelpulver von dem aufgesprühten dasjenige Schmiermittelpulver ist, das als restliches Schmiermittelpulver übrig geblieben ist, welches nicht auf dem mit den Oberflächen des unteren Stempels und des Formwerkzeuges in Kontakt stehenden Material haften geblieben ist, während es aus einem Schmiermittelsprühauslass für den unteren Stempel, welcher in einer Sprühmittelaufbringvorrichtung vorgesehen ist mit der positiv pulsierenden Vibrationsluft gemischt und in ihr auf das Material dispergiert wird, das mit der Oberfläche des unteren Stempels und der des Formwerkzeuges in Kontakt ist; und
- Bewegen des oberen Stempels von dem einen Ende des schlitzähnlichen Schmiermittelsprühauslasses für den oberen Stempel zum anderen Ende davon,
wobei genügend Zeit in Anspruch genommen wird und Schmiermittelpulver auf das Material aufgebracht wird, das mit der Oberfläche des oberen Stempels in Kontakt ist.

17. Verfahren zur Herstellung einer Brausetablette gemäß einem der Ansprüche 14 bis 16, wobei die Körnchen eines Hauptwirkstoffes, die Carbonatkörnchen und die Körnchen einer organischen Säure in ihren Teilchendurchmessern nahezu gleich sind.

18. Verfahren zur Herstellung einer Brausetablette gemäß einem der Ansprüche 14 bis 17, wobei die Carbonatkörnchen solche sind, die mindestens eine Art oder ein Gemisch von mindestens zwei Arten, ausgewählt aus der Gruppe Natriumhydrogencarbonat, Natriumcarbonat, Natriumsesquicarbonat, Calciumcarbonat, Kaliumcarbonat, Magnesiumcarbonat, Calciumhydrogencarbonat und Kaliumhydrogencarbonat, umfassen.

19. Verfahren zur Herstellung einer Brausetablette gemäß einem der Ansprüche 14 bis 18, wobei die Körnchen einer organischen Säure solche sind, die mindestens eine Art, ausgewählt aus der Gruppe Zitronensäure, Weinsäure, Fumarsäure, Bernsteinsäure, Adipinsäure, Äpfelsäure und Maleinsäure, umfassen.

20. Verfahren zur Herstellung einer Brausetablette gemäß einem der Ansprüche 14 bis 19, wobei die Körnchen eines Hauptwirkstoffes, die Carbonatkörnchen und die Körnchen einer organischen Säure alle solche sind, die mit einem wasserlöslichen Polymer als Bindemittel granuliert sind.

21. Verfahren zur Herstellung einer Brausetablette gemäß einem der Ansprüche 14 bis 20, wobei die Körnchen eines Hauptwirkstoffes, die Carbonatkörnchen und die Körnchen einer organischen Säure mit einem Bindemittel, das ein grenzflächenaktives Mittel einschließt, granuliert sind.

22. Verfahren zur Herstellung einer Brausetablette für einen Badezusatz, wobei die Körnchen eines Hauptwirkstoffes, die in dem Verfahren zur Herstellung einer Brausetablette gemäß einem der Ansprüche 14 bis 21 verwendet werden, Natriumcarbonatkörnchen sind.

23. Verfahren zur Herstellung einer Brausetablette für ein Waschmittel, wobei die Körnchen eines Hauptwirkstoffes, die im Verfahren zur Herstellung einer Brausetablette gemäß einem der Ansprüche 14 bis 21 verwendet werden, Körnchen eines grenzflächenaktiven Mittels und Körnchen von Fettsäurealkalisalzen einschließen.

24. Verfahren zur Herstellung einer Brausetablette für ein Waschmittel gemäß Anspruch 23,
wobei ferner wasserfreies Natriumsulfat in das Gemisch hinzugefügt wird.

25. Verfahren zur Herstellung einer Brausetablette zur oralen Verabreichung, umfassend einen Tablettenkörper, der durch Komprimierung eines Gemisches hergestellt wird, das Körnchen eines Hauptwirkstoffes, die solche sind, die mit einem ein Saccharid mit hoher Benetzbarkeit für Wasser einschließenden Bindemittel granuliert sind, Carbonatkörnchen, die solche sind, die mit einem ein Saccharid mit hoher Benetzbarkeit für Wasser einschließenden Bindemittel granuliert sind, und Körnchen einer organischen Säure, die solche sind, die mit einem ein Saccharid mit hoher Benetzbarkeit für Wasser einschließenden Bindemittel granuliert sind, umfasst, wobei die Körnchen eines Hauptwirkstoffes, die Carbonatkörnchen und die Körnchen der organischen Säure derart gemischt werden, dass die Teilchengrößenverteilung ihres Körnchengemisches eine normale Verteilung mit einem Peak aufweist, und das Gemisch keine Schmiermittel umfasst und wobei Schmiermittelpulver auf die Oberfläche des Tablettenkörpers aufgebracht wird, wobei das Schmiermittelpulver auf einen Stempel und ein Formwerkzeug aufgetragen und auf die Oberfläche des Tablettenkörpers übertragen werden, wenn ein Kompressionsschritt unter Verwendung des Stempels und des Formwerkzeuges ausgeführt wird.

26. Verfahren zur Herstellung einer Brausetablette zur oralen Verabreichung gemäß Anspruch 25, wobei die Körnchen eines Hauptwirkstoffes, die Carbonatkörnchen und die Körnchen einer organischen Säure alle in ihren Teilchendurchmessern nahezu gleich sind.

27. Verfahren zur Herstellung einer Brausetablette zur oralen Verabreichung gemäß einem der Ansprüche 25 oder 26, wobei die Carbonatkörnchen solche sind, die mindestens eine Art oder ein Gemisch von mindestens zwei Arten, ausgewählt aus der Gruppe Natriumhydrogencarbonat, Natriumcarbonat, Natriumsesquicarbonat, Calciumcarbonat, Kaliumcarbonat, Magnesiumcarbonat, Calciumhydrogencarbonat und Kaliumhydrogencarbonat, umfassen.

28. Verfahren zur Herstellung einer Brausetablette zur oralen Verabreichung gemäß einem der Ansprüche 25 bis 27, wobei die Körnchen einer organischen Säure solche sind, die mindestens eine Art, ausgewählt aus der Gruppe Zitronensäure, Weinsäure, Fumarsäure, Bernsteinsäure, Adipinsäure, Äpfelsäure und Maleinsäure, umfassen.

## Revendications

1. Comprimé effervescent comprenant un corps de comprimé qui est produit par compression d'un mélange comprenant des granules d'un ingrédient actif principal, des granules de carbonate et des granules d'acide organique, où lesdits granules d'un ingrédient actif principal, lesdits granules de carbonate et lesdits granules d'acide organique sont mélangés de telle sorte que la distribution de taille de particule de leur mélange de granules présente une distribution normale avec un pic, ledit mélange ne comprend pas de lubrifiants, et où des poudres de lubrifiant sont fixées sur la surface dudit corps de comprimé,
lesdites poudres de lubrifiant étant appliquées sur un poinçon et une matrice et transférées à la surface dudit corps de comprimé quand une étape de compression est exécutée au moyen dudit poinçon et de ladite matrice.

2. Comprimé effervescent selon la revendication 1, où lesdits granules d'un ingrédient actif principal, lesdits granules de carbonate et lesdits granules d'acide organique sont tous sensiblement les mêmes concernant leurs diamètres de particules.

3. Comprimé effervescent selon l'une quelconque des revendications 1 ou 2, où lesdits granules de carbonate sont ceux comprenant au moins un type ou un mélange d'au moins deux types choisis dans le groupe consistant en l'hydrogénocarbonate de sodium, le carbonate de sodium, le sesquicarbonate de sodium, le carbonate de calcium, le carbonate de potassium, le carbonate de magnésium, l'hydrogénocarbonate de calcium et l'hydrogénocarbonate de potassium.

4. Comprimé effervescent selon l'une quelconque des revendications 1-3, où lesdits granules d'acide organique sont ceux comprenant au moins un type choisi dans le groupe consistant en l'acide citrique, l'acide tartrique, l'acide fumarique, l'acide succinique, l'acide adipique, l'acide malique et l'acide maléique.

5. Comprimé effervescent selon l'une quelconque des revendications 1-4, où chacun desdits granules d'un ingrédient actif principal, desdits granules de carbonate et desdits granules d'acide organique sont ceux granulés avec un polymère hydrosoluble comme liant, respectivement.

6. Comprimé effervescent selon l'une quelconque des revendications 1-5, où chacun desdits granules d'un ingrédient actif principal, desdits granules de carbonate et desdits granules d'acide organique sont ceux granulés avec un liant incluant un tensioactif.

7. Comprimé effervescent pour un agent pour le bain, où lesdits granules d'un ingrédient actif principal selon l'une quelconque des revendications 1-6 sont des granules de carbonate de sodium.

8. Comprimé effervescent pour un détergent de lavage, où lesdits granules d'un ingrédient actif principal selon l'une quelconque des revendications 1-6 incluent des granules de tensioactif et des granules de sel alcalin d'acide gras.

9. Comprimé effervescent pour un détergent de lavage selon la revendication 8, où du sulfate de sodium anhydre est inclus également.

10. Comprimé effervescent pour l'administration orale comprenant un corps de comprimé qui est produit par compression d'un mélange comprenant des granules d'un ingrédient actif principal qui sont ceux granulés avec un liant incluant un saccharide à haute mouillabilité pour l'eau, des granules de carbonate qui sont ceux granulés avec un liant incluant un saccharide à haute mouillabilité pour l'eau, et des granules d'acide organique qui sont ceux granulés avec un liant incluant un saccharide à haute mouillabilité pour l'eau, où lesdits granules d'un ingrédient actif principal, lesdits granules de carbonate et lesdits granules d'acide organique sont mélangés de telle sorte que la distribution de taille de particules de leur mélange de granules présente une distribution normale avec un pic, ledit mélange ne comprend pas de lubrifiants, et où des poudres de lubrifiant sont fixées sur la surface dudit corps de comprimé,
lesdites poudres de lubrifiant étant appliquées sur un poinçon et une matrice et transférées à la surface dudit corps de comprimé quand une étape de compression est exécutée au moyen dudit poinçon et de ladite matrice.

11. Comprimé effervescent pour l'administration orale selon la revendication 10, où lesdits granules d'un ingrédient actif principal, lesdits granules de carbonate et lesdits granules d'acide organique sont tous sensiblement les mêmes concernant leurs diamètres de particules.

12. Comprimé effervescent pour l'administration orale selon l'une quelconque des revendications 10 ou 11, où lesdits granules de carbonate sont ceux comprenant au moins un type ou un mélange d'au moins deux types choisis dans le groupe consistant en l'hydrogénocarbonate de sodium, le carbonate de sodium, le sesquicarbonate de sodium, le carbonate de calcium, le carbonate de potassium, le carbonate de magnésium, l'hydrogénocarbonate de calcium et l'hydrogénocarbonate de potassium.

13. Comprimé effervescent pour l'administration orale selon l'une quelconque des revendications 10-12, où lesdits granules d'acide organique sont ceux comprenant au moins un type choisi dans le groupe consistant en l'acide citrique, l'acide tartrique, l'acide fumarique, l'acide succinique, l'acide adipique, l'acide malique et l'acide maléique.

14. Procédé de production d'un comprimé effervescent comprenant les étapes de :
préparation d'un mélange incluant des granules d'un ingrédient actif principal, des granules de carbonate et des granules d'acide inorganique ; où lesdits granules d'un ingrédient actif principal, lesdits granules de carbonate et lesdits granules d'acide organique sont mélangés de telle sorte que la distribution de taille de particule de leur mélange de granules présente une distribution normale avec un pic, et ledit mélange ne comprend pas de lubrifiants, et
application de poudres de lubrifiant sur une surface de contact de produit d'un poinçon et une surface de contact de produit d'une matrice, l'un et l'autre étant utilisés pour compresser ledit mélange pour produire ledit comprimé, et
compression dudit mélange avec ledit poinçon et ladite matrice, sur les surfaces de contact de produit desquels lesdites poudres de lubrifiant sont fixées.

15. Procédé de production d'un comprimé effervescent selon la revendication 14, où ladite étape d'application de poudres de lubrifiant sur une surface de contact de produit d'un poinçon et une surface de contact de produit d'une matrice, l'un et l'autre étant utilisés pour compresser ledit mélange pour produire ledit comprimé, est exécutée d'une manière telle que des poudres de lubrifiant mélangées avec et dispersées dans un air à vibrations à pulsations positives sont pulvérisées sur ladite surface de contact de produit dudit poinçon et ladite surface de contact de produit de ladite matrice, l'un et l'autre étant utilisés pour compresser ledit mélange pour produire ledit comprimé.

16. Procédé de production d'un comprimé effervescent selon la revendication 14, où ladite étape d'application de poudres de lubrifiant sur une surface de contact de produit d'un poinçon et une surface de contact de produit d'une matrice, l'un et l'autre étant utilisés pour compresser ledit mélange pour produire ledit comprimé, comprend les étapes de :
une première d'application de lubrifiant où des poudres de lubrifiant sont appliquées sur lesdites surface de contact de produit d'un poinçon inférieur et d'une matrice, et
une seconde étape d'application de lubrifiant où des poudres de lubrifiant sont appliquées sur ladite surface de contact de produit d'un poinçon supérieur,
ledit poinçon inférieur, ledit poinçon supérieur et ladite matrice étant utilisés pour compresser ledit mélange pour produire ledit comprimé,
où ladite première étape d'application de lubrifiant comprend :
- la pulvérisation de poudres de lubrifiant mélangées avec et dispersées dans un air à vibrations à pulsations positives depuis un orifice de pulvérisation de lubrifiant pour un poinçon inférieur, qui est prévu dans un moyen d'application de lubrifiant, sur la surface de contact de produit dudit poinçon inférieur, qui est inséré dans une position prédéterminée dans ladite matrice ; et
- l'application supplémentaire de poudres de lubrifiant sur ladite surface de contact de produit de ladite matrice, où lesdites poudres de lubrifiant sont celles qui sont retirées par soufflage de ladite surface de contact de produit dudit poinçon inférieur par ledit air à vibrations à pulsations positives parmi les poudres de lubrifiant pulvérisées sur ladite surface de contact de produit dudit poinçon inférieur ; et
où ladite seconde étape d'application de lubrifiant comprend :
- le soufflage de poudres de lubrifiant dans une direction de la surface de contact de produit dudit poinçon supérieur depuis un orifice de pulvérisation de lubrifiant analogue à une fente pour un poinçon supérieur prévu dans ledit moyen d'application de lubrifiant, lesdites poudres de lubrifiant étant des poudres de lubrifiant destinées à être laissées comme poudres de lubrifiant résiduelles qui n'ont pas été fixées sur chacune desdites surfaces de contact de produit dudit poinçon inférieur et de ladite matrice parmi celles pulvérisées, tout en étant mélangées avec et dispersées dans ledit air à vibrations à pulsations positives sur chacune desdites surfaces de contact de produit dudit poinçon inférieur et de ladite matrice depuis un orifice de pulvérisation de lubrifiant pour un poinçon inférieur, qui est prévu dans un moyen d'application de lubrifiant ; et
- le déplacement dudit poinçon supérieur depuis l'extrémité initiale dudit orifice de pulvérisation de lubrifiant analogue à une fente pour un poinçon supérieur jusqu'à son extrémité terminale, en prenant suffisamment de temps et en appliquant des poudres de lubrifiant sur ladite surface de contact de produit dudit poinçon supérieur.

17. Procédé de production d'un comprimé effervescent selon l'une quelconque des revendications 14-16, où lesdits granules d'un ingrédient actif principal, lesdits granules de carbonate et lesdits granules d'acide organique sont sensiblement les mêmes en ce qui concerne leurs diamètres de particules.

18. Procédé de production d'un comprimé effervescent selon l'une quelconque des revendications 14-17, où lesdits granules de carbonate sont ceux comprenant au moins un type ou un mélange d'au moins deux types choisis dans le groupe consistant en l'hydrogénocarbonate de sodium, le carbonate de sodium, le sesquicarbonate de sodium, le carbonate de calcium, le carbonate de potassium, le carbonate de magnésium, l'hydrogénocarbonate de calcium et l'hydrogénocarbonate de potassium.

19. Procédé de production d'un comprimé effervescent selon l'une quelconque des revendications 14-18, où lesdits granules d'acide organique sont ceux comprenant au moins un type choisi dans le groupe consistant en l'acide citrique, l'acide tartrique, l'acide fumarique, l'acide succinique, l'acide adipique, l'acide malique et l'acide maléique.

20. Procédé de production d'un comprimé effervescent selon l'une quelconque des revendications 14-19, où chacun desdits granules d'un ingrédient actif principal, desdits granules de carbonate et desdits granules d'acide organique sont ceux granulés avec un polymère hydrosoluble comme liant.

21. Procédé de production d'un comprimé effervescent selon l'une quelconque des revendications 14-20, où chacun desdits granules d'un ingrédient actif principal, desdits granules de carbonate et desdits granules d'acide organique sont granulés avec un liant incluant un tensioactif.

22. Procédé de production d'un comprimé effervescent pour un agent pour le bain, où lesdits granules d'un ingrédient actif principal utilisés dans le procédé de production d'un comprimé effervescent selon l'une quelconque des revendications 14-21 sont des granules de carbonate de sodium.

23. Procédé de production d'un comprimé effervescent pour un détergent de lavage, où lesdits granules d'un ingrédient actif principal utilisés dans le procédé de production d'un comprimé effervescent selon l'une quelconque des revendications 14-21 incluent des granules de tensioactif et des granules de sel alcalin d'acide gras.

24. Procédé de production d'un comprimé effervescent pour un détergent de lavage selon la revendication 23, où du sulfate de sodium anhydre est ajouté aussi audit mélange.

25. Procédé de production d'un comprimé effervescent pour l'administration orale comprenant un corps de comprimé qui est produit par compression d'un mélange comprenant des granules d'un ingrédient actif principal qui sont ceux granulés avec un liant incluant un saccharide à haute mouillabilité pour l'eau, des granules de carbonate qui sont ceux granulés avec un liant incluant un saccharide à haute mouillabilité pour l'eau, et des granules d'acide organique qui sont ceux granulés avec un liant incluant un saccharide à haute mouillabilité pour l'eau, où lesdits granules d'un ingrédient actif principal, lesdits granules de carbonate et lesdits granules d'acide organique sont mélangés de telle sorte que la distribution de taille de particule de leur mélange de granules présente une distribution normale avec un pic, et ledit mélange ne comprend pas de lubrifiants, et où des poudres de lubrifiant sont fixées sur la surface dudit corps de comprimé,
lesdites poudres de lubrifiant étant appliquées sur un poinçon et une matrice et transférées à la surface dudit corps de comprimé quand une étape de compression est exécutée au moyen dudit poinçon et de ladite matrice.

26. Procédé de production d'un comprimé effervescent pour l'administration orale selon la revendication 25, où lesdits granules d'un ingrédient actif principal, lesdits granules de carbonate et lesdits granules d'acide organique sont tous sensiblement les mêmes en ce qui concerne leurs diamètres de particules.

27. Procédé de production d'un comprimé effervescent pour l'administration orale selon l'une quelconque des revendications 25 ou 26, où lesdits granules de carbonate sont ceux comprenant au moins un type ou un mélange d'au moins deux types choisis dans le groupe consistant en l'hydrogénocarbonate de sodium, le carbonate de sodium, le sesquicarbonate de sodium, le carbonate de calcium, le carbonate de potassium, le carbonate de magnésium, l'hydrogénocarbonate de calcium et l'hydrogénocarbonate de potassium.

28. Procédé de production d'un comprimé effervescent pour l'administration orale selon l'une quelconque des revendications 25-27,
où lesdits granules d'acide organique sont ceux comprenant au moins un type choisi dans le groupe consistant en l'acide citrique, l'acide tartrique, l'acide fumarique, l'acide succinique, l'acide adipique, l'acide malique et l'acide maléique.
